(19) Europäisches Patentamt
European Patent Office
Office européen des brevets

(11) EP 1 346 998 B1

(12) EUROPEAN PATENT SPECIFICATION

(45) Date of publication and mention of the grant of the patent:
**19.09.2007 Bulletin 2007/38**

(51) Int Cl.:
*C07K 14/08* (2006.01) *A61K 39/12* (2006.01)
*C12N 15/40* (2006.01)

(21) Application number: **02002250.5**

(22) Date of filing: **30.01.2002**

(54) **Equine arteritis virus vaccine**

Impfstoff gegen Pferdearteritisvirus

Vaccin contre le virus de l'artérite équine

(84) Designated Contracting States:
**AT BE CH CY DE DK ES FI FR GB GR IE IT LI LU MC NL PT SE TR**

(43) Date of publication of application:
**24.09.2003 Bulletin 2003/39**

(73) Proprietor: **Giese, Matthias Dr.**
**69123 Heidelberg (DE)**

(72) Inventors:
- **Giese, Matthias**
  **69123 Heidelberg (DE)**
- **Darai, Gholamreza**
  **69120 Heidelberg (DE)**

(74) Representative: **Grund, Martin**
**GRUND**
**Intellectual Property Group**
**Postfach 44 05 16**
**80754 München (DE)**

(56) References cited:
**WO-A-95/19438** **WO-A-98/02549**

- **TOBIASCH E ET AL: "Large envelope glycoprotein and nucleocapsid protein of equine arteritis virus (EAV) induce an immune response in Balb/c mice by DNA vaccination;strategy for developing a DNA-vaccine against EAV-infection." VIRUS GENES, vol. 22, no. 2, March 2001 (2001-03), pages 187-199, XP008006216 ISSN: 0920-8569**
- **CHOW Y-H ET AL: "IMPROVEMENT OF HEPATITIS B VIRUS DNA VACCINES BY PLASMIDS COEXPRESSING HEPATITIS B SURFACE ANTIGEN AND INTERLEUKIN-2" JOURNAL OF VIROLOGY, NEW YORK, US, US, vol. 71, no. 1, January 1997 (1997-01), pages 169-178, XP001007203 ISSN: 0022-538X**
- **KRIEG ARTHUR M ET AL: "The role of CpG dinucleotides in DNA vaccines." TRENDS IN MICROBIOLOGY, vol. 6, no. 1, January 1998 (1998-01), pages 23-27, XP000857633 ISSN: 0966-842X**
- **O'HAGAN D T ET AL: "Recent developments in adjuvants for vaccines against infectious diseases" BIOMOLECULAR ENGINEERING, ELSEVIER, NEW YORK, NY, US, vol. 18, no. 3, 15 October 2001 (2001-10-15), pages 69-85, XP004305905 ISSN: 1389-0344**
- **PIRZADEH B ET AL: "IMMUNE RESPONSE IN PIGS VACCINATED WITH PLASMID DNA ENCODING ORF5 OF PORCINE REPRODUCTIVE AND RESPIRATORY SYNDROME VIRUS" JOURNAL OF GENERAL VIROLOGY, SOCIETY FOR GENERAL MICROBIOLOGY, READING, GB, vol. 79, 1998, pages 989-999, XP000827985 ISSN: 0022-1317**
- **BARRY ET AL: "Biological features of genetic immunization" VACCINE, BUTTERWORTH SCIENTIFIC. GUILDFORD, GB, vol. 15, no. 8, 1997, pages 788-791, XP002099107 ISSN: 0264-410X**
- **SNIJDER E.J. ET AL: 'Identification of a novel structural protein of arteriviruses' JOURNAL OF VIROLOGY vol. 73, no. 8, September 1999, pages 6335 - 6345**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- BAASURIYA U.B.R. ET AL: 'Equine Arteritis virus derived from an infectious cDNA clone is attenuated and genetically stable in infected stallions' VIROLOGY vol. 260, 1999, pages 201 - 208

SEQUENCE LISTING

<110> Boehringer Ingelheim Vetmedica GmbH

<120> Equine Arterivirus Vaccine

<130> 1-1281-1

<140> not yet assigned
<141> 2002-01-19

<160> 61

<170> Patent In Ver. 2.1

<210> 1
<211> 9031
<212> DNA
<213> Artificial Sequence

<220>
<223> Description of Artificial Sequence:ORF 1a and b of EAV

<400> 1

```
taaaactttt gccacctgac cgccgtgagg ctggtttgcg gttgtattac aaccattacc 60
gcgaacaaag gaccgggtgg ctgtctaaaa caggacttcg cttatggctt ggagacctgg 120
gtttgggcat caatgcgagc tctggagggc tgaaattcca cattatgagg ggttcgcctc 180
agcgagcttg gcatatcaca acacgcagct gcaagctgaa gagctactac gtttgtgaca 240
tctctgaagc agactggtcc tgtttgcctg ctggcaacta cggcggctac aatccaccag 300
gggacggagc ttgcggttac aggtgcttgg ccttcatgaa tggcgccact gttgtgtcgg 360
ctggttgcag ttctgacttg tggtgtgatg atgagttggc ttatcgagtc tttcaattgt 420
cacccacgtt cacggttacc atcccaggtg ggcgagtttg tccgaatgcc aagtacgcaa 480
tgatttgtga caagcagcac tggcgcgtca aacgtgcaaa gggcgtcggc ctgtgtctcg 540
atgaaagctg tttcaggggc atctgcaatt gccaacgcat gagtggacca ccacctgcac 600
ccgtgtcagc cgccgtgtta gatcacatac tggaggcggc gacgtttggc aacgttcgcg 660
tggttacacc tgaagggcag ccacgccccg taccagcgcc gcgagttcgt cccagcgcca 720
actcttctgg agatgtcaaa gatccggcgc ccgttccgcc agtaccaaaa ccaaggacca 780
agcttgccac accgaaccca actcaggcgc ccatcccagc accgcgcacg cgacttcaag 840
gggcctcaac acaggagcca ctggcgagtg caggagttgc ttctgactcg gcacctaaat 900
ggcgtgtggc caaaactgtg tacagctccg cggagcgctt tcggaccgaa ctggtacaac 960
gtgctcggtc cgttggggac gttcttgttc aagcgctacc gctcaaaacc ccagcagtgc 1020
agcggtatac catgactctg aagatgatgc gttcacgctt cagttggcac tgcgacgtgt 1080
ggtacccttt ggctgtaatc gcttgtttgc tccctatatg gccatctctt gctttgctcc 1140
ttagctttgc cattgggttg atacccagtg tgggcaataa tgttgttctg acagcgcttc 1200
tggtttcatc agctaattat gttgcgtcaa tggaccatca atgtgaaggt gcggcttgct 1260
tagccttgct ggaagaagaa cactattata gagcggtccg ttggcgcccg attacaggcg 1320
cgctgtcgct tgtgctcaat ttactggggc aggtaggcta tgtagctcgt tccacctttg 1380
atgcagctta tgttccttgc actgtgttcg atctttgcag ctttgctatt ctgtacctct 1440
gccgcaatcg ttgctggaga tgcttcggac gctgtgtgcg agttgggcct gccacgcatg 1500
ttttgggctc caccgggcaa cgagtttcca aactggcgct cattgatttg tgtgaccact 1560
tttcaaagcc caccatcgat gttgtgggca tggcaactgg ttggagcgga tgttacacag 1620
gaaccgccgc aatggagcgt cagtgtgcct ctacggtgga ccctcactcg ttcgaccaga 1680
agaaggcagg agcgactgtt tacctcaccc cccctgtcaa cagcgggtca gcgctgcagt 1740
gcctcaatgt catgtggaag cgaccaattg ggtccactgt ccttggggaa caaacaggag 1800
ctgttgtgac ggcggtcaag agtatctctt tctcacctcc ctgctgcgtc tctaccactt 1860
tgcccacccg acccggtgtg accgttgtcg accatgctct ttacaaccgg ttgactgctt 1920
caggggtcga tcccgcttta ttgcgtgttg ggcaaggtga ttttctaaaa cttaatccgg 1980
ggttccggct gataggtgga tggatttatg ggatatgcta ttttgtgttg gtggttgtgt 2040
caacttttac ctgcttacct atcaaatgtg gcattggcac ccgcgaccct ttctgccgca 2100
gagtgttttc tgtacccgtc accaagaccc aagagcactg ccatgctgga atgtgtgcta 2160
gcgctgaagg catctctctg gactctctgg ggttaactca gttacaaagt tactggatcg 2220
cagccgtcac tagcggatta gtgatcttgt tggtctgcca ccgcctggcc atcagcgcct 2280
```

tggacttgtt gactctagct tcccctttag tgttgcttgt gttcccttgg gcatctgtgg 2340
ggcttttact tgcttgcagt ctcgctggtg ctgctgtgaa aatacagttg ttggcgacgc 2400
tttttgtgaa tctgttcttt ccccaagcta cccttgtcac tatgggatac tgggcgtgcg 2460
tggcggcttt ggccgtttac agtttgatgg gcttgcgagt gaaagtgaat gtgcccatgt 2520
gtgtgacacc tgcccatttt ctgctgctgg cgaggtcagc tggacagtca agagagcaga 2580
tgctccgggt cagcgctgct gcccccacca attcactgct tggagtggct cgtgattgtt 2640
atgtcacagg cacaactcgg ctgtacatac ccaaggaagg cgggatggtg tttgaagggc 2700
tattcaggtc accgaaggcg cgcggcaacg tcggcttcgt ggctggtagc agctacggca 2760
cagggtcagt gtggaccagg aacaacgagg tcgtcgtact gacagcgtca cacgtggttg 2820
gccgcgctaa catggccact ctgaagatcg gtgacgcaat gctgactctg actttcaaaa 2880
agaatggcga cttcgccgag gcagtgacga cacagtccga gctcccaggc aattggccac 2940
agttgcattt cgcccaacca acaaccgggc ccgcttcatg gtgcactgcc acaggagatg 3000
aagaaggctt gctcagtggc gaggtttgtc tggcgtggac tactagtggc gactctggat 3060
ctgcagtggt tcagggtgac gctgtggtag ggtccacac cggttcgaac acaagtggtg 3120
ttgcctacgt gaccacccca agcggaaaac tccttggcgc cgacaccgtg actttgtcat 3180
cactgtcaaa gcatttcaca ggccctttga catcaatccc gaaggacatc cctgacaaca 3240
ttattgccga tgttgatgct gttcctcgtt ctctggccat gctgattgat ggcttatcca 3300
atagagagag cagcctttct ggacctcagt tgttgttaat tgcttgtttt atgtggtctt 3360
atcttaacca acctgcttac ttgccttatg tgctgggctt ctttgccgct aacttcttcc 3420
tgccaaaaag tgttggccgc cctgtggtca ctgggcttct atggttgtgc tgcctcttca 3480
caccgctttc catgcgcttg tgcttgttcc atctggtctg tgctaccgtc acgggaaacg 3540
tgatatcttt gtggttctac atcactgccg ctggcacgtc ttacctttct gagatgtggt 3600
tcggaggcta tcccaccatg ttgtttgtgc cacggttcct agtgtaccag ttccccggct 3660
gggctattgg cacagtacta gcggtatgca gcatcaccat gctggctgct gccctcggtc 3720
acaccctgtt actggatgtg ttctccgcct caggtcgctt tgacaggact ttcatgatga 3780
aatacttcct ggagggagga gtgaaagaga gtgtcaccgc ctcagtcacc cgcgcttatg 3840
gcaaaccaat tacccaggag agtctcactg caacattagc tgccctcact gatgatgact 3900
tccaattcct ctctgatgtg cttgactgtc gggccgtccg atcggcaatg aatctgcgtg 3960
ccgctctcac aagttttcaa gtggcgcagt atcgtaacat ccttaatgca tccttgcaag 4020
tcgatcgtga cgctgctcgt agtcgcagac taatggcaaa actggctgat tttgcggttg 4080
aacaagaagt aacagctgga gaccgtgttg tggttatcga cggtctggac cgcatggctc 4140
acttcaaaga cgatttggtg ctggttcctt tgaccaccaa agtagtaggc ggttctaggt 4200
gcaccatttg tgacgtcgtt aaggaagaag ccaatgacac cccagttaag ccaatgccca 4260
gcaggagacg ccgcaagggc ctgcctaaag gtgctcagtt ggagtgggac cgtcaccagg 4320
aagagaagag gaacgccggt gatgatgatt ttgcggtctc gaatgattat gtcaagagag 4380
tgccaaagta ctgggatccc agcgacaccc gaggcacgac agtgaaaatc gccggcacta 4440
cctatcagaa agtggttgac tattcaggca atgtgcatta cgtggagcat caggaagatc 4500
tgctagacta cgtgctgggc aaggggagct atgaaggcct agatcaggac aaagtgttgg 4560
acctcacaaa catgcttaaa gtggacccca cggagctctc ctccaaagac aaagccaagg 4620
cgcgtcagct tgctcatctg ctgttggatc tggctaaccc agttgaggca gtgaatcagt 4680
taaactgaga gcgccccaca tctttcccgg cgatgtgggg cgtcggacct ttgctgactc 4740
taaagacaag ggtttcgtgg ctctacacag tcgcacaatg tttttagctg cccgggactt 4800
tttatttaac atcaaatttg tgtgcgacga agagttcaca aagaccccaa aagacacact 4860
gcttgggtac gtacgcgcct gccctggtta ctggtttatt ttccgtcgta cgcaccggtc 4920
gctgattgat gcatactggg acagtatgga gtgcgtttac gcgcttccca ccatatctga 4980
ttttgatgtg agcccaggtg acgtcgcagt gacgggcgag cgatgggatt ttgaatctcc 5040
cggaggaggc cgtgcaaaac gtctcacagc tgatctggtg cacgcttttc aagggttcca 5100
cggagcctct tattcctatg atgacaaggt ggcagctgct gtcagtggtg acccgtatcg 5160
gtcggacggc gtcttgtata acacccgttg gggcaacatt ccatattctg tcccaaccaa 5220
tgctttggaa gccacagctt gctaccgtgc tggatgtgag gccgttaccg acgggaccaa 5280
cgtcatcgca acaattgggc ccttcccgga gcaacaaccc ataccggaca tcccaaagag 5340
cgtgcttgac aactgcgctg acatcagctg tgacgctttc atagcgcccg ctgcagagac 5400
agccctgtgt ggagatttag agaaatacaa cctatccacg caggtgtttg tgttgcctag 5460
tgttttctcc atggtgcggg cgtacttaaa agaggagatt ggagacgctc caccactcta 5520
cttgccatct actgtaccat ctaaaaattc acaagccgga attaacggcg ctgagtttcc 5580
tacaaagtct ttacagagct actgtttgat tgatgacatg gtgtcacagt ccatgaaaag 5640
caatctacaa accgccacca tggcgacttg taaacggcaa tactgttcca aatacaagat 5700
taggagcatt ctgggcacca acaattacat tggcctaggt ttgcgtgcct gcctttcggg 5760
ggttacggcc gcattccaaa aagctggaaa ggatgggtca ccgatttatt tgggcaagtc 5820
aaaattcgac ccgataccag ctcctgacaa gtactgcctt gaaacagacc tggagagttg 5880
tgatcgctcc accccggctt tggtgcgttg gttcgctact aatcttattt ttgagctagc 5940
tggccagccc gagttggtgc acagctacgt gttgaattgc tgtcacgatc tagttgtggc 6000
gggtagtgta gcattcacca aacgcggggg tttgtcatct ggagacccta tcacttccat 6060

ttccaatacc atctattcat tggtgctgta cacccagcac atgttgctat gtggacttga 6120
aggctatttc ccagagattg cagaaaaata tcttgatggc agcctggagc tgcgggacat 6180
gttcaagtac gttcgagtgt acatctactc ggacgatgtg gttctaacca cacccaacca 6240
gcattacgcg gccagctttg accgctgggt cccccacctg caggcgctgc taggtttcaa 6300
ggttgaccca aagaaaactg tgaacaccag ctccccttcc tttttgggct gccggttcaa 6360
gcaagtggac ggcaagtgtt atctagccag tcttcaggac cgcgttacac gctctctgtt 6420
ataccacatt ggtgcaaaga atccctcaga gtactatgaa gctgctgttt ccatctttaa 6480
ggactccatt atctgctgtg atgaagactg gtggacggac ctccatcgac gtatcagtgg 6540
cgctgcgcgt accgacggag ttgagttccc caccattgaa atgttaacat ccttccgcac 6600
caagcagtat gagagtgccg tgtgcacagt ttgtggggcc gcccccgtgg ccaagtctgc 6660
ttgtggaggg tggttctgtg gcaattgtgt cccgtaccac gcgggtcatt gtcacacaac 6720
ctcgctcttc gccaactgcg ggcacgacat catgtaccgc tccacttact gcacaatgtg 6780
tgagggttcc ccaaaacaga tggtaccaaa agtgcctcac ccgatcctgg atcatttgct 6840
gtgccacatt gattacggca gtaaagagga actaactctg gtagtggcgg atggtcgaac 6900
aacatcaccg cccgggcgct acaaagtggg tcacaaggta gtcgccgtgg ttgcagatgt 6960
gggaggcaac attgtgtttg ggtgcggtcc tggatcacac atcgcagtac cacttcagga 7020
tacgctcaag ggcgtggtgg tgaataaagc tctgaagaac gccgccgcct ctgagtacgt 7080
ggaaggaccc cctgggagtg ggaagacttt tcacctggtc aaagatgtgc tagccgtggt 7140
cggtagcgcg accttggttg tgcccaccca cgcgtccatg ctggactgca tcaacaagct 7200
caaacaagcg ggcgccgatc catactttgt ggtgcccaag tatacagttc ttgactttcc 7260
ccggcctggc agtggaaaca tcacagtgcg actgccacag gtcggaacca gtgagggaga 7320
aacctttgtg gatgaggtgg cctacttctc accagtggat ctggcgcgca ttttaaccca 7380
gggtcgagtc aagggttacg gtgatttaaa tcagctcggg tgcgtcggac ccgcgagcgt 7440
gccacgtaac ctttggctcc gacattttgt cagcctggag cccttgcgag tgtgccatcg 7500
attcggcgct gctgtgtgtg atttgatcaa gggcatttat ccttattatg agccagctcc 7560
acataccact aaagtggtgt ttgtgccaaa tccagacttt gagaaaggtg tagtcatcac 7620
cgcctaccac aaagatcgcg gtcttggtca ccgcacaatt gattcaattc aaggctgtac 7680
attccctgtt gtgactcttc gactgcccac accccaatca ctgacgcgcc cgcgcgcagt 7740
tgtggcggtt actagggcgt ctcaggaatt atacatctac gacccctttg atcagcttag 7800
cgggttgttg aagttcacca aggaagcaga ggcgcaggac ttgatccatg gccccaccac 7860
agcatgccac ctgggccaag aaattgacct ttggtccaat gagggcctcg aatattacaa 7920
ggaagtcaac ctgctgtaca cacacgtccc catcaaggat ggtgtaatac acagttaccc 7980
taattgtggc cctgcctgtg gctgggaaaa gcaatccaac aaaatttcgt gcctccccgag 8040
agtggcacaa aatttgggct accactattc cccagactta ccaggatttt gccccatacc 8100
aaaagaactc gctgagcatt ggcccgtagt gtccaatgat agatacccga attgcttgca 8160
aattacctta cagcaagtat gtgaactcag taaaccgtgc tcagcgggct atatggttgg 8220
acaatctgtt ttcgtgcaga cgcctggtgt gacatcttac tggcttactg aatgggtcga 8280
cggcaaagcg cgtgctctac cagattcctt attctcgtcc ggtaggttcg agactaacag 8340
ccgcgctttc ctcgatgaag ccgaggaaaa gtttgccgcc gctcaccctc atgcctgttt 8400
gggagaaatt aataagtcca ccgtgggagg atcccacttc atcttttccc aatatttacc 8460
accattgcta cccgcagacg ctgttgccct ggtaggtgct tcattggctg ggaaagctgc 8520
taaagctgct tgcagcgttg ttgatgtcta tgctccatca tttgaacctt atctacaccc 8580
tgagacactg agtcgcgtgt acaagattat gatcgatttc aagccgtgta ggcttatggt 8640
gtggagaaac gcgacctttt atgtccaaga gggtgttgat gcagttacat cagcactagc 8700
agctgtgtcc aaactcatca aagtgccggc caatgagcct gtttcattcc atgtggcatc 8760
agggtacaga accaacgcgc tggtagcgcc ccaggctaaa atttcaattg gagcctacgc 8820
cgccgagtgg gcactgtcaa ctgaaccgcc acctgctggt tatgcgatcg tgcggcgata 8880
tattgtaaag aggctcctca gctcaacaga agtgttcttg tgccgcaggg gtgttgtgtc 8940
ttccacctca gtgcagacca tttgtgcact agagggatgt aaacctctgt tcaacttctt 9000
acaaattggt tcagtcattg ggcccgtgtg a 9031

<210> 2
<211> 684
<212> DNA
<213> Artificial Sequence

<220>
<223> Description of Artificial Sequence:ORF2 of EAV

<400> 2

```
tgctatttct gtgattgatg cagcgctttt ctttctcatg ctacttgcat tggctgttgt 60
tactgtgttt cttttctggc tcattgttgc catcggccgc agcttggtgg cgcggtgttc 120
acgaggtgcg cgttacagac ctgtttaagg atttgcagtg cgacaacctg cgcgcgaaag 180
atgccttccc gagtctggga tatgctctgt cgattggcca gtcgaggcta tcgtatatgc 240
tgcaggattg gttgcttgct gcgcaccgca aggaagttat gccttccaat atcatgccta 300
tgcccggtct tactcctgat tgctttgacc atctggagtc ttctagctat gctccattta 360
tcaatgccta tcggcaggca attttgagtc aatacccaca agagctccag ctcgaagcca 420
tcaactgtaa attgcttgct gtggttgcac cggcattgta tcataattac catctagcca 480
atttgaccgg accggccaca tgggtcgtgc ctacagtggg ccagttgcac tattatgctt 540
cttcctctat ttttgcttca tctgtggaag tgttggcagc aataatacta ctatttgcat 600
gcataccact agtgacacga gtgtacatct cttttacgcg gctaatgtca ccttcccgtc 660
gcacttccag cggcactttg ccgc                                        684
```

<210> 3
<211> 501
<212> DNA
<213> Artificial Sequence

<220>
<223> Description of Artificial Sequence : ORF 3 of EAV

<400> 3

```
accggccaca tgggtcgtgc ctacagtggg ccagttgcac tattatgctt cttcctctat 60
ttttgcttca tctgtggaag tgttggcagc aataatacta ctatttgcat gcataccact 120
agtgacacga gtgtacatct cttttacgcg gctaatgtca ccttcccgtc gcacttccag 180
cggcactttg ccgcggcgca agattttgta gtgcacacgg gttatgaata tgccggggtc 240
actatgttag tgcacttgtt tgccaacttg gttctgacat ttccgagctt agttaattgt 300
tcccgccctg tgaatgtctt tgctaatgct tcttgcgtgc aagtggtttg tagtcatacc 360
aactcaacta ctggcttggg tcaactttct ttttcctttg tagatgaaga tctacggctg 420
catatcaggc ctactcttat ttgttggttt gccttgttgt tggtgcactt tctacccatg 480
ccacgctgca gaggctcgta a                                           501
```

<210> 4
<211> 458
<212> DNA
<213> Artificial Sequence

<220>
<223> Description of Artificial Sequence:ORF4 of EAV

<400> 4

```
tgaagatcta cggctgcata tcaggcctac tcttatttgt tggtttgcct tgttgttggt 60
gcactttcta cccatgccac gctgcagagg ctcgtaattt tacttacatt agtcatggat 120
tgggccacgt gcacggtcat gaggggtgta ggaattttat taatgtcact cattctgcat 180
ttctttatct taatcccacc actcccactg cgccggctat aactcattgt ttacttctgg 240
ttctggcagc caaaatggaa cacccaaacg ctactatctg gctgcagctg cagccgtttg 300
ggtatcatgt ggctggcgat gtcattgtca acttggaaga ggacaagagg catccttact 360
ttaaactttt gagagcgccg gctttaccgc ttggttttgt ggctatagtt tatgttcttt 420
tacgactggt acgttgggct caacgatgtt atctatga                         458
```

<210> 5
<211> 767
<212> DNA
<213> Artificial Sequence

<220>
<223> Description of Artificial Sequence:Description of Artificial SequenceORF 5 of EAV

<400> 5

```
tacgttgggc tcaacgatgt tatctatgat tgtattgcta ttcttgcttt ggggtgcgcc 60
atcacatgct tacttctcat actacaccgc tcagcgcttc acagacttca ccttgtgtat 120
```

```
gctgacggat cgcggcgtta ttgccaattt gctgcgatat gatgagcaca ctgctttgta 180
caattgttcc gccagtaaaa cctgttggta ttgcacattc ctggacgaac agattatcac 240
gtttggaacc gattgtgatg acacctacgc ggtcccagtt gctgaggtcc tggaacaggc 300
gcatggaccg tacagtgcgc tgtttgatga catgcccct tttatttact atggccgtga 360
attcggcata gttgtgttgg atgtgtttat gttctatccc gttttagttc tgtttttctt 420
atcagtacta ccctatgcta cgcttattct tgaaatgtgt gtatctattc tgtttataat 480
ctatggcatt tacagcgggg cctacttggc catgggcata tttgcggcca cgcttgctat 540
acattcaatt gtggtcctcc gccaattact gtggttatgc ctggcttggc gataccgctg 600
tacgcttcac gcgtccttta tatcagctga ggggaaagtg taccccgtag accccggact 660
cccggttgcc gccgtgggca atcggttgtt agtcccaggt aggcccacta tcgattatgc 720
agtggcctac ggcagcaaag tcaaccttgt gaggttgggg gcagctg                767
```

<210> 6
<211> 489
<212> DNA
<213> Artificial Sequence

<220>
<223> Description of Artificial Sequence:ORF 6 of EAV

<400> 6

atgggagcca tagattcatt ttgtggtgac gggattttag gtgagtatct agattacttt 60
attctgtccg tcccactctt gctgttgctt actaggtatg tagcatctgg gttagtgtat 120
gttttgactg ccttgttcta ttcctttgta ttagcagctt atatttggtt tgttatagtt 180
ggaagagcct tttctactgc ttatgctttt gtgcttttgg ctgcttttct gttattagta 240
atgaggatga ttgtgggtat gatgcctcgt cttcggtcca ttttcaacca tcgccaactg 300
gtggtagctg attttgtgga cacacctagt ggacctgttc ccatcccccg ctcaactact 360
caggtagtgg ttcgcggcaa cgggtacacc gcagttggta acaagcttgt cgatggcgtc 420
aagacgatca cgtccgcagg ccgcctcttt tcgaaacgga cggcggcgac agcctacaag 480
ctacaatga 489

<210> 7
<211> 333
<212> DNA
<213> Artificial Sequence

<220>
<223> Description of Artificial Sequence:ORF7 of EAV

<400> 7

tggtaacaag cttgtcgatg gcgtcaagac gatcacgtcc gcaggccgcc tcttttcgaa 60
acggacggcg gcgacagcct acaagctaca atgacctact gcgcatgttt ggtcagatgc 120
gggtccgcaa accgcccgcg caacccactc aggctattat tgcagagcct ggagacctta 180
ggcatgattt aaatcaacag gagcgcgcca ccctttcgtc gaacgtacaa cggttcttca 240
tgattgggca tggttcactc actgcagatg ccggaggact cacgtacacc gtcagttggg 300
ttcctaccaa acaaatccag cgcaaagttg cgc 333

<210> 8
<211> 495
<212> DNA
<213> Artificial Sequence

<220>
<223> Description of Artificial Sequence:Partial ORF1 sequence

<400> 8

tggcaacctt ctccgctact ggatttggag ggagttttgt tagggactgg tccctggact 60
tacccgacgc ttgtgagcat ggcgcgggat tgtgctgcga agtggacggc tccaccttat 120
gcgccgagtg ttttcgcggt tgcgaaggaa tggagcaatg tcctggcttg ttcatgggac 180

tgttaaaact ggcttcgcca gttccagtgg gacataagtt cctgattggt tggtatcgag 240
ctgccaaagt caccgggcgt tacaatttcc ttgagctgtt gcaacaccct gctttcgccc 300
agctgcgtgt ggttgatgct aggttagcca ttgaagaggc aagtgtgttt atttccactg 360
accacgcgtc tgctaagcgt ttccctggcg ctagatttgc gctgacaccg gtgtatgcta 420
acgcttgggt tgtgagcccg gctgctaaca gtttgatagt gaccactgac caggaacaag 480
atgggttctg ctggt 495

<210> 9
<211> 363
<212> DNA
<213> Artificial Sequence

<220>
<223> Description of Artificial Sequence:Truncated ORF5 of EAV

<400> 9

```
tacgttgggc tcaacgatgt tatctatgat tgtattgcta ttcttgcttt ggggtgcgcc 60
atcacatgct tacttctcat actacaccgc tcagcgcttc acagacttca ccttgtgtat 120
gctgacggat cgcggcgtta ttgccaattt gctgcgatat gatgagcaca ctgctttgta 180
caattgttcc gccagtaaaa cctgttggta ttgcacattc ctggacgaac agattatcac 240
gtttggaacc gattgtgatg acacctacgc ggtcccagtt gctgaggtcc tggaacaggc 300
gcatggaccg tacagtgcgc tgtttgatga catgccccct tttatttact atggccgtga 360
att                                                               363
```

<210> 10
<2.11> 12704
<212> DNA
<213> Equine arteritis virus

<400> 10

```
gctcgaagtg tgtatggtgc catatacggc tcaccaccat atacactgca agaattacta 60
ttcttgtggg cccctctcgg taaatcctag agggctttcc tctcgttatt gcgagattcg 120
tcgttagata acggcaagtt cccttttctta ctatcctatt ttcatcttgt ggcttgacgg 180
gtcactgcca tcgtcgtcga tctctatcaa ctacccttgc gactatggca accttctccg 240
ctactggatt tggagggagt tttgttaggg actggtccct ggacttaccc gacgcttgtg 300
agcatggcgc gggattgtgc tgcgaagtgg acggctccac cttatgcgcc gagtgttttc 360
gcggttgcga aggaatggag caatgtcctg gcttgttcat gggactgtta aaactggctt 420
cgccagttcc agtgggacat aagttcctga ttggttggta tcgagctgcc aaagtcaccg 480
ggcgttacaa tttccttgag ctgttgcaac accctgcttt cgcccagctg cgtgtggttg 540
atgctaggtt agccattgaa gaggcaagtg tgtttatttc cactgaccac gcgtctgcta 600
agcgtttccc tggcgctaga tttgcgctga caccggtgta tgctaacgct tgggttgtga 660
gcccggctgc taacagtttg atagtgacca ctgaccagga acaagatggg ttctgctggt 720
taaaactttt gccacctgac cgccgtgagg ctggtttgcg gttgtattac aaccattacc 780
gcgaacaaag gaccgggtgg ctgtctaaaa caggacttcg cttatggctt ggagacctgg 840
gtttgggcat caatgcgagc tctggagggc tgaaattcca cattatgagg ggttcgcctc 900
agcgagcttg gcatatcaca acacgcagct gcaagctgaa gagctactac gtttgtgaca 960
tctctgaagc agactggtcc tgtttgcctg ctggcaacta cggcggctac aatccaccag 1020
gggacggagc ttgcggttac aggtgcttgg ccttcatgaa tggcgccact gttgtgtcgg 1080
ctggttgcag ttctgacttg tggtgtgatg atgagttggc ttatcgagtc tttcaattgt 1140
cacccacgtt cacggttacc atcccaggtg ggcgagtttg tccgaatgcc aagtacgcaa 1200
tgatttgtga caagcagcac tggcgcgtca aacgtgcaaa gggcgtcggc ctgtgtctcg 1260
atgaaagctg tttcaggggc atctgcaatt gccaacgcat gagtggacca ccacctgcac 1320
ccgtgtcagc cgccgtgtta gatcacatac tggaggcggc gacgtttggc aacgttcgcg 1380
tggttacacc tgaagggcag ccacgccccg taccagcgcc gcgagttcgt cccagcgcca 1440
actcttctgg agatgtcaaa gatccggcgc ccgttccgcc agtaccaaaa ccaaggacca 1500
agcttgccac accgaaccca actcaggcgc ccatcccagc accgcgcacg cgacttcaag 1560
gggcctcaac acaggagcca ctggcgagtg caggagttgc ttctgactcg gcacctaaat 1620
ggcgtgtggc caaaactgtg tacagctccg cggagcgctt tcggaccgaa ctggtacaac 1680
gtgctcggtc cgttggggac gttcttgttc aagcgctacc gctcaaaacc ccagcagtgc 1740
agcggtatac catgactctg aagatgatgc gttcacgctt cagttggcac tgcgacgtgt 1800
```

```
ggtacccttt ggctgtaatc gcttgtttgc tccctatatg gccatctctt gctttgctcc 1860
ttagctttgc cattgggttg atacccagtg tgggcaataa tgttgttctg acagcgcttc 1920
tggtttcatc agctaattat gttgcgtcaa tggaccatca atgtgaaggt gcggcttgct 1980
tagccttgct ggaagaagaa cactattata gagcggtccg ttggcgcccg attacaggcg 2040
cgctgtcgct tgtgctcaat ttactggggc aggtaggcta tgtagctcgt tccacctttg 2100
atgcagctta tgttccttgc actgtgttcg atctttgcag ctttgctatt ctgtacctct 2160
gccgcaatcg ttgctggaga tgcttcggac gctgtgtgcg agttgggcct gccacgcatg 2220
ttttgggctc caccgggcaa cgagtttcca aactggcgct cattgatttg tgtgaccact 2280
tttcaaagcc caccatcgat gttgtgggca tggcaactgg ttggagcgga tgttacacag 2340
gaaccgccgc aatggagcgt cagtgtgcct ctacggtgga ccctcactcg ttcgaccaga 2400
agaaggcagg agcgactgtt tacctcaccc cccctgtcaa cagcgggtca gcgctgcagt 2460
gcctcaatgt catgtggaag cgaccaattg ggtccactgt ccttggggaa caaacaggag 2520
ctgttgtgac ggcggtcaag agtatctctt tctcacctcc ctgctgcgtc tctaccactt 2580
tgcccacccg acccggtgtg accgttgtcg accatgctct ttacaaccgg ttgactgctt 2640
caggggtcga tcccgcttta ttgcgtgttg ggcaaggtga ttttctaaaa cttaatccgg 2700
ggttccggct gataggtgga tggatttatg ggatatgcta ttttgtgttg gtggttgtgt 2760
caacttttac ctgcttacct atcaaatgtg gcattggcac ccgcgaccct ttctgccgca 2820
gagtgttttc tgtacccgtc accaagaccc aagagcactg ccatgctgga atgtgtgcta 2880
gcgctgaagg catctctctg gactctctgg ggttaactca gttacaaagt tactggatcg 2940
cagccgtcac tagcggatta gtgatcttgt tggtctgcca ccgcctggcc atcagcgcct 3000
tggacttgtt gactctagct tccccttag tgttgcttgt gttcccttgg gcatctgtgg 3060
ggcttttact tgcttgcagt ctcgctggtg ctgctgtgaa aatacagttg ttggcgacgc 3120
ttttgtgaa tctgttcttt ccccaagcta cccttgtcac tatgggatac tggcgtgcg 3180
tggcggcttt ggccgtttac agtttgatgg gcttgcgagt gaaagtgaat gtgcccatgt 3240
gtgtgacacc tgcccatttt ctgctgctgg cgaggtcagc tggacagtca agagagcaga 3300
tgctccgggt cagcgctgct gcccccacca attcactgct tggagtggct cgtgattgtt 3360
atgtcacagg cacaactcgg ctgtacatac ccaaggaagg cgggatggtg tttgaagggc 3420
tattcaggtc accgaaggcg cgcggcaacg tcggcttcgt ggctggtagc agctacggca 3480
cagggtcagt gtggaccagg aacaacgagg tcgtcgtact gacagcgtca cacgtggttg 3540
gccgcgctaa catggccact ctgaagatcg gtgacgcaat gctgactctg actttcaaaa 3600
agaatggcga cttcgccgag gcagtgacga cacagtccga gctcccaggc aattggccac 3660
agttgcattt cgcccaacca acaaccgggc ccgcttcatg gtgcactgcc acaggagatg 3720
aagaaggctt gctcagtggc gaggtttgtc tggcgtggac tactagtggc gactctggat 3780
ctgcagtggt tcagggtgac gctgtggtag ggtccacac cggttcgaac acaagtggtg 3840
ttgcctacgt gaccacccca agcggaaaac tccttggcgc cgacaccgtg actttgtcat 3900
cactgtcaaa gcatttcaca ggccctttga catcaatccc gaaggacatc cctgacaaca 3960
ttattgccga tgttgatgct gttcctcgtt ctctggccat gctgattgat ggcttatcca 4020
atagagagag cagcctttct ggacctcagt tgttgttaat tgcttgtttt atgtggtctt 4080
atcttaacca acctgcttac ttgccttatg tgctgggctt ctttgccgct aacttcttcc 4140
tgccaaaaag tgttggccgc cctgtggtca ctgggcttct atggttgtgc tgcctcttca 4200
caccgctttc catgcgcttg tgcttgttcc atctggtctg tgctaccgtc acgggaaacg 4260
tgatatcttt gtggttctac atcactgccg ctggcacgtc ttacctttct gagatgtggt 4320
tcggaggcta tcccaccatg ttgtttgtgc cacggttcct agtgtaccag ttccccggct 4380
gggctattgg cacagtacta gcggtatgca gcatcaccat gctggctgct gccctcggtc 4440
acaccctgtt actggatgtg ttctccgcct caggtcgctt tgacaggact ttcatgatga 4500
aatacttcct ggagggagga gtgaaagaga gtgtcaccgc ctcagtcacc cgcgcttatg 4560
gcaaaccaat tacccaggag agtctcactg caacattagc tgccctcact gatgatgact 4620
tccaattcct ctctgatgtg cttgactgtc gggccgtccg atcggcaatg aatctgcgtg 4680
ccgctctcac aagttttcaa gtggcgcagt atcgtaacat ccttaatgca tccttgcaag 4740
tcgatcgtga cgctgctcgt agtcgcagac taatggcaaa actggctgat tttgcggttg 4800
aacaagaagt aacagctgga gaccgtgttg tggttatcga cggtctggac cgcatggctc 4860
acttcaaaga cgatttggtg ctggttcctt tgaccaccaa agtagtaggc ggttctaggt 4920
gcaccatttg tgacgtcgtt aaggaagaag ccaatgacac cccagttaag ccaatgccca 4980
gcaggagacg ccgcaagggc ctgcctaaag gtgctcagtt ggagtgggac cgtcaccagg 5040
aagagaagag gaacgccggt gatgatgatt ttgcggtctc gaatgattat gtcaagagag 5100
tgccaaagta ctgggatccc agcgacaccc gaggcacgac agtgaaaatc gccggcacta 5160
cctatcagaa agtggttgac tattcaggca atgtgcatta cgtggagcat caggaagatc 5220
tgctagacta cgtgctgggc aaggggagct atgaaggcct agatcaggac aaagtgttgg 5280
acctcacaaa catgcttaaa gtggacccca cggagctctc ctccaaagac aaagccaagg 5340
cgcgtcagct tgctcatctg ctgttggatc tggctaaccc agttgaggca gtgaatcagt 5400
taaactgaga gcgccccaca tctttcccgg cgatgtgggg cgtcggacct ttgctgactc 5460
taaagacaag ggtttcgtgg ctctacacag tcgcacaatg tttttagctg cccgggactt 5520
tttatttaac atcaaatttg tgtgcgacga agagttcaca aagaccccaa aagacacact 5580
```

gcttgggtac gtacgcgcct gccctggtta ctggtttatt ttccgtcgta cgcaccggtc 5640
gctgattgat gcatactggg acagtatgga gtgcgtttac gcgcttccca ccatatctga 5700
ttttgatgtg agcccaggtg acgtcgcagt gacgggcgag cgatgggatt ttgaatctcc 5760
cggaggaggc cgtgcaaaac gtctcacagc tgatctggtg cacgcttttc aagggttcca 5820
cggagcctct tattcctatg atgacaaggt ggcagctgct gtcagtggtg acccgtatcg 5880
gtcggacggc gtcttgtata acacccgttg gggcaacatt ccatattctg tcccaaccaa 5940
tgctttggaa gccacagctt gctaccgtgc tggatgtgag gccgttaccg acgggaccaa 6000
cgtcatcgca acaattgggc ccttccccgga gcaacaaccc ataccggaca tcccaaagag 6060
cgtgcttgac aactgcgctg acatcagctg tgacgctttc atagcgcccg ctgcagagac 6120
agccctgtgt ggagatttag agaaatacaa cctatccacg cagggttttg tgttgcctag 6180
tgttttctcc atggtgcggg cgtacttaaa agaggagatt ggagacgctc caccactcta 6240
cttgccatct actgtaccat ctaaaaattc acaagccgga attaacggcg ctgagtttcc 6300
tacaaagtct ttacagagct actgtttgat tgatgacatg gtgtcacagt ccatgaaaag 6360
caatctacaa accgccacca tggcgacttg taaacggcaa tactgttcca aatacaagat 6420
taggagcatt ctgggcacca acaattacat tggcctaggt ttgcgtgcct gcctttcggg 6480
ggttacggcc gcattccaaa aagctggaaa ggatgggtca ccgatttatt tgggcaagtc 6540
aaaattcgac ccgataccag ctcctgacaa gtactgcctt gaaacagacc tggagagttg 6600
tgatcgctcc accccggctt tggtgcgttg gttcgctact aatcttattt ttgagctagc 6660
tggccagccc gagttggtgc acagctacgt gttgaattgc tgtcacgatc tagttgtggc 6720
gggtagtgta gcattcacca aacgcggggg tttgtcatct ggagacccta tcacttccat 6780
ttccaatacc atctattcat tggtgctgta cacccagcac atgttgctat gtggacttga 6840
aggctatttc ccagagattg cagaaaaata tcttgatggc agcctggagc tgcgggacat 6900
gttcaagtac gttcgagtgt acatctactc ggacgatgtg gttctaacca cacccaacca 6960
gcattacgcg gccagctttg accgctgggt cccccacctg caggcgctgc taggtttcaa 7020
ggttgaccca aagaaaactg tgaacaccag ctccccttcc tttttgggct gccggttcaa 7080
gcaagtggac ggcaagtgtt atctagccag tcttcaggac cgcgttacac gctctctgtt 7140
ataccacatt ggtgcaaaga atccctcaga gtactatgaa gctgctgttt ccatctttaa 7200
ggactccatt atctgctgtg atgaagactg gtggacggac ctccatcgac gtatcagtgg 7260
cgctgcgcgt accgacggag ttgagttccc caccattgaa atgttaacat ccttccgcac 7320
caagcagtat gagagtgccg tgtgcacagt ttgtggggcc gcccccgtgg ccaagtctgc 7380
ttgtggaggg tggttctgtg gcaattgtgt cccgtaccac gcgggtcatt gtcacacaac 7440
ctcgctcttc gccaactgcg ggcacgacat catgtaccgc tccacttact gcacaatgtg 7500
tgagggttcc ccaaaacaga tggtaccaaa agtgcctcac ccgatcctgg atcatttgct 7560
gtgccacatt gattacggca gtaaagagga actaactctg gtagtggcgg atggtcgaac 7620
aacatcaccg cccgggcgct acaaagtggg tcacaaggta gtcgccgtgg ttgcagatgt 7680
gggaggcaac attgtgtttg ggtgcggtcc tggatcacac atcgcagtac cacttcagga 7740
tacgctcaag ggcgtggtgg tgaataaagc tctgaagaac gccgccgcct ctgagtacgt 7800
ggaaggaccc cctgggagtg ggaagacttt tcacctggtc aaagatgtgc tagccgtggt 7860
cggtagcgcg accttggttg tgcccaccca cgcgtccatg ctggactgca tcaacaagct 7920
caaacaagcg ggcgccgatc catactttgt ggtgcccaag tatacagttc ttgactttcc 7980
ccggcctggc agtggaaaca tcacagtgcg actgccacag gtcggaacca gtgagggaga 8040
aacctttgtg gatgaggtgg cctacttctc accagtggat ctggcgcgca ttttaaccca 8100
gggtcgagtc aagggttacg gtgatttaaa tcagctcggg tgcgtcggac ccgcgagcgt 8160
gccacgtaac ctttggctcc gacattttgt cagcctggag cccttgcgag tgtgccatcg 8220
attcggcgct gctgtgtgtg atttgatcaa gggcatttat ccttattatg agccagctcc 8280
acataccact aaagtggtgt ttgtgccaaa tccagacttt gagaaaggtg tagtcatcac 8340
cgcctaccac aaagatcgcg gtcttggtca ccgcacaatt gattcaattc aaggctgtac 8400
attccctgtt gtgactcttc gactgcccac accccaatca ctgacgcgcc cgcgcgcagt 8460
tgtggcggtt actagggcgt ctcaggaatt atacatctac gacccctttg atcagcttag 8520
cgggttgttg aagttcacca aggaagcaga ggcgcaggac ttgatccatg gcccacctac 8580
agcatgccac ctgggccaag aaattgacct ttggtccaat gagggcctcg aatattacaa 8640
ggaagtcaac ctgctgtaca cacacgtccc catcaaggat ggtgtaatac acagttaccc 8700
taattgtggc cctgcctgtg gctgggaaaa gcaatccaac aaaatttcgt gcctcccgag 8760
agtggcacaa aatttgggct accactattc cccagactta ccaggatttt gccccatacc 8820
aaaagaactc gctgagcatt ggcccgtagt gtccaatgat agatacccga attgcttgca 8880
aattacctta cagcaagtat gtgaactcag taaaccgtgc tcagcgggct atatggttgg 8940
acaatctgtt ttcgtgcaga cgcctggtgt gacatcttac tggcttactg aatgggtcga 9000
cggcaaagcg cgtgctctac cagattcctt attctcgtcc ggtaggttcg agactaacag 9060
ccgcgctttc ctcgatgaag ccgaggaaaa gtttgccgcc gctcaccctc atgcctgttt 9120
gggagaaatt aataagtcca ccgtgggagg atcccacttc atcttttccc aatatttacc 9180
accattgcta cccgcagacg ctgttgccct ggtaggtgct tcattggctg ggaaagctgc 9240
taaagctgct tgcagcgttg ttgatgtcta tgctccatca tttgaacctt atctacaccc 9300
tgagacactg agtcgcgtgt acaagattat gatcgatttc aagccgtgta ggcttatggt 9360

gtggagaaac gcgacctttt atgtccaaga gggtgttgat gcagttacat cagcactagc 9420
agctgtgtcc aaactcatca aagtgccggc caatgagcct gtttcattcc atgtggcatc 9480
agggtacaga accaacgcgc tggtagcgcc ccaggctaaa atttcaattg gagcctacgc 9540
cgccgagtgg gcactgtcaa ctgaaccgcc acctgctggt tatgcgatcg tgcggcgata 9600
tattgtaaag aggctcctca gctcaacaga agtgttcttg tgccgcaggg gtgttgtgtc 9660
ttccacctca gtgcagacca tttgtgcact agagggatgt aaacctctgt tcaacttctt 9720
acaaattggt tcagtcattg ggcccgtgtg atgggcttag tgtggtcact gatttcaaat 9780
tctattcaga ctattattgc tgattttgct atttctgtga ttgatgcagc gcttttcttt 9840
ctcatgctac ttgcattggc tgttgttact gtgtttcttt tctggctcat tgttgccatc 9900
ggccgcagct tggtggcgcg gtgttcacga ggtgcgcgtt acagacctgt ttaaggattt 9960
gcagtgcgac aacctgcgcg cgaaagatgc cttcccgagt ctgggatatg ctctgtcgat 10020
tggccagtcg aggctatcgt atatgctgca ggattggttg cttgctgcgc accgcaagga 10080
agttatgcct tccaatatca tgcctatgcc cggtcttact cctgattgct ttgaccatct 10140
ggagtcttct agctatgctc catttatcaa tgcctatcgg caggcaattt tgagtcaata 10200
cccacaagag ctccagctcg aagccatcaa ctgtaaattg cttgctgtgg ttgcaccggc 10260
attgtatcat aattaccatc tagccaattt gaccggaccg gccacatggg tcgtgcctac 10320
agtgggccag ttgcactatt atgcttcttc ctctattttt gcttcatctg tggaagtgtt 10380
ggcagcaata atactactat ttgcatgcat accactagtg acacgagtgt acatctcttt 10440
tacgcggcta atgtcacctt cccgtcgcac ttccagcggc actttgccgc ggcgcaagat 10500
tttgtagtgc acacgggtta tgaatatgcc ggggtcacta tgttagtgca cttgtttgcc 10560
aacttggttc tgacatttcc gagcttagtt aattgttccc gccctgtgaa tgtctttgct 10620
aatgcttctt gcgtgcaagt ggtttgtagt cataccaact caactactgg cttgggtcaa 10680
ctttcttttt cctttgtaga tgaagatcta cggctgcata tcaggcctac tcttatttgt 10740
tggtttgcct tgttgttggt gcactttcta cccatgccac gctgcagagg ctcgtaattt 10800
tacttacatt agtcatggat tgggccacgt gcacggtcat gaggggtgta ggaattttat 10860
taatgtcact cattctgcat ttctttatct taatcccacc actcccactg cgccggctat 10920
aactcattgt ttacttctgg ttctggcagc caaaatggaa cacccaaacg ctactatctg 10980
gctgcagctg cagccgtttg ggtatcatgt ggctggcgat gtcattgtca acttggaaga 11040
ggacaagagg catccttact ttaaactttt gagagcgccg gctttaccgc ttggttttgt 11100
ggctatagtt tatgttcttt tacgactggt acgttgggct caacgatgtt atctatgatt 11160
gtattgctat tcttgctttg ggtgcgccca tcacatgctt acttctcata ctacaccgct 11220
cagcgcttca cagacttcac cttgtgtatg ctgacggatc gcggcgttat tgccaatttg 11280
ctgcgatatg atgagcacac tgctttgtac aattgttccg ccagtaaaac ctgttggtat 11340
tgcacattcc tggacgaaca gattatcacg tttggaaccg attgtgatga cacctacgcg 11400
gtcccagttg ctgaggtcct ggaacaggcg catggaccgt acagtgcgct gtttgatgac 11460
atgccccctt ttatttacta tggccgtgaa ttcggcatag ttgtgttgga tgtgtttatg 11520
ttctatcccg ttttagttct gtttttctta tcagtactac cctatgctac gcttattctt 11580
gaaatgtgtg tatctattct gtttataatc tatggcattt acagcggggc ctacttggcc 11640
atgggcatat ttgcggccac gcttgctata cattcaattg tggtcctccg ccaattactg 11700
tggttatgcc tggcttggcg ataccgctgt acgcttcacg cgtcctttat atcagctgag 11760
gggaaagtgt accccgtaga ccccggactc ccggttgccg ccgtgggcaa tcggttgtta 11820
gtcccaggta ggcccactat cgattatgca gtggcctacg gcagcaaagt caaccttgtg 11880
aggttggggg cagctgaggt atgggagcca tagattcatt ttgtggtgac gggattttag 11940
gtgagtatct agattacttt attctgtccg tcccactctt gctgttgctt actaggtatg 12000
tagcatctgg gttagtgtat gttttgactg ccttgttcta ttcctttgta ttagcagctt 12060
atatttggtt tgttatagtt ggaagagcct tttctactgc ttatgctttt gtgcttttgg 12120
ctgcttttct gttattagta atgaggatga ttgtgggtat gatgcctcgt cttcggtcca 12180
ttttcaacca tcgccaactg gtggtagctg attttgtgga cacacctagt ggacctgttc 12240
ccatccccccg ctcaactact caggtagtgg ttcgcggcaa cgggtacacc gcagttggta 12300
acaagcttgt cgatggcgtc aagacgatca cgtccgcagg ccgcctcttt tcgaaacgga 12360
cggcggcgac agcctacaag ctacaatgac ctactgcgca tgtttggtca gatgcgggtc 12420
cgcaaaccgc ccgcgcaacc cactcaggct attattgcag agcctggaga ccttaggcat 12480
gatttaaatc aacaggagcg cgccacccctt tcgtcgaacg tacaacggtt cttcatgatt 12540
gggcatggtt cactcactgc agatgccgga ggactcacgt acaccgtcag ttgggttcct 12600
accaaacaaa tccagcgcaa agttgcgcct ccagcagggc cgtaagacgt ggatattctc 12660
ctgtgtggcg tcatgttgaa gtagttatta gccacccagg aacc 12704

<210> 11
<211> 33

<212> DNA
<213> Artificial Sequence

<220>
<223> Description of Artificial Sequence:oligonucleotide primer

<400> 11
gggtctagag tcaccacaaa atgaatctat ggc 33

<210> 12
<211> 34
<212> DNA
<213> Artificial Sequence

<220>
<223> Description of Artificial Sequence:oligonucleotide primer

<400> 12
ggggggatcc cctttgtaga tgaagatcta cggc 34

<210> 13
<211> 31
<212> DNA
<213> Artificial Sequence

<220>
<223> Description of Artificial Sequence:oligonucleotide primer

<400> 13
gggggatcct ggtacgttgg gctcaacgat g 31

<210> 14
<211> 43
<212> DNA
<213> Artificial Sequence

<220>
<223> Description of Artificial Sequence:oligonucleotide primer

<400> 14
gggggtctag agcaatacaa tcatagataa catcgttgag ccc 43

<210> 15
<211> 32
<212> DNA
<213> Artificial Sequence

<220>
<223> Description of Artificial Sequence:oligonucleotide primer

<400> 15
gggtctagac cacacaggag aatatccacg tc 32

<210> 16
<211> 33
<212> DNA
<213> Artificial Sequence

<220>
<223> Description of Artificial Sequence:oligonucleotide primer

<400> 16
ggggggatcc cagctgaggt atgggagcca tag 33

<210> 17
<211> 32
<212> DNA
<213> Artificial Sequence

<220>
<223> Description of Artificial Sequence:oligonucleotide primer

<400> 17
gggggatccc gcagttggta acaagcttgt cg 32

<210> 18
<211> 34
<212> DNA
<213> Artificial Sequence

<220>
<223> Description of Artificial Sequence:oligonucleotide primer

<400> 18
ggggggaatt ccatgcgcag taggtcattg tagc 34

<210> 19
<211> 33
<212> DNA
<213> Artificial Sequence

<220>
<223> Description of Artificial Sequence:oligonucleotide primer

<400> 19
gggtctagag tcaccacaaa atgaatctat ggc 33

<210> 20
<211> 33
<212> DNA
<213> Artificial Sequence

<220>
<223> Description of Artificial Sequence:oligonucleotide primer

<400> 20
gggggggtcg actggctccc atacctcagc tgc 33

<210> 21
<211> 31
<212> DNA
<213> Artificial Sequence

<220>
<223> Description of Artificial Sequence:oligonucleotide primer

<400> 21
gggggatcct ggtacgttgg gctcaacgat g 31

<210> 22
<211> 36
<212> DNA
<213> Artificial Sequence

<220>
<223> Description of Artificial Sequence:oligonucleotide primer

<400> 22
gggggggagat ctatggcgtc aagacgatca cgtccg 36

<210> 23
<211> 31
<212> DNA
<213> Artificial Sequence

<220>
<223> Description of Artificial Sequence : oligonucleotide primer

<400> 23
gggggatcca tttctgtgat tgatgcagcg c 31

<210> 24
<211> 33
<212> DNA
<213> Artificial Sequence

<220>
<223> Description of Artificial Sequence:oligonucleotide primer

<400> 24
gggggggtcg accggccctg ctggaggcgc aac 33

<210> 25
<211> 36
<212> DNA
<213> Artificial Sequence

<220>
<223> Description of Artificial Sequence:oligonucleotide primer

<400> 25
gggtctagag gcatattcat aacccgtgtg cactac 36

<210> 26
<211> 36
<212> DNA
<213> Artificial Sequence

<220>
<223> Description of Artificial Sequence:oligonucleotide primer

<400> 26
gggggggagat ctatgcagcg cttttctttc tcatgc 36

<210> 27
<211> 34
<212> DNA
<213> Artificial Sequence

<220>
<223> Description of Artificial Sequence:oligonucleotide primer

<400> 27
gggggatcct ttgaccggac cggccacatg ggtc 34

<210> 28
<211> 35
<212> DNA
<213> Artificial Sequence

<220>
<223> Description of Artificial Sequence:oligonucleotide primer

<400> 28
gggggggtcg accaaaatct tgcgccgcgg caaag 35

<210> 29
<211> 36
<212> DNA
<213> Artificial Sequence

<220>
<223> Description of Artificial Sequence:oligonucleotide primer

<400> 29
gggtctagag taagtaaaat tacgagcctc tgcagc 36

<210> 30
<211> 38
<212> DNA
<213> Artificial Sequence

<220>
<223> Description of Artificial Sequence:oligonucleotide primer

<400> 30
ggggggagat ctatgggagc catagattca ttttgtgg 38

<210> 31
<211> 33
<212> DNA
<213> Artificial Sequence

<220>
<223> Description of Artificial Sequence:oligonucleotide primer

<400> 31
gggggatccc ctttgtagat gaagatctac ggc 33

<210> 32
<211> 35
<212> DNA

<213> Artificial Sequence

<220>
<223> Description of Artificial Sequence:oligonucleotide primer

<400> 32
gggggggtcg acttgtagct tgtaggctgt cgccg 35

<210> 33
<211> 41
<212> DNA
<213> Artificial Sequence

<220>
<223> Description of Artificial Sequence:oligonucleotide primer

<400> 33
gggtctagag caatacaatc atagataaca tcgttgagcc c 41

<210> 34
<211> 35
<212> DNA
<213> Artificial Sequence

<220>
<223> Description of Artificial Sequence:oligonucleotide primer

<400> 34
gggggggaat tcatggcaac cttctccgct actgg 35

<210> 35
<211> 32
<212> DNA
<213> Artificial Sequence

<220>
<223> Description of Artificial Sequence:oligonucleotide primer

<400> 35
gggggatccc agctgaggta tgggagccat ag 32

<210> 36
<211> 36
<212> DNA
<213> Artificial Sequence

<220>
<223> Description of Artificial Sequence:oligonucleotide primer

<400> 36
ggggggtcta gatcacacgg gcccaatgac tgaacc 36

<210> 37
<211> 32
<212> DNA
<213> Artificial Sequence

<220>

<223> Description of Artificial Sequence:oligonucleotide primer

<400> 37
ggggaattcc atgcgcagta ggtcattgta gc 32

<210> 38
<211> 38
<212> DNA
<213> Artificial Sequence

<220>
<223> Description of Artificial Sequence:oligonucleotide primer

<400> 38
ccccttaagt gccatatacg gctcaccacc atatacac 38

<210> 39
<211> 32
<212> DNA
<213> Artificial Sequence

<220>
<223> Description of Artificial Sequence:oligonucleotide primer

<400> 39
ggggggatcc tggtacgttg ggctcaacga tg 32

<210> 40
<211> 37
<212> DNA
<213> Artificial Sequence

<220>
<223> Description of Artificial Sequence:oligonucleotide primer

<400> 40
cccttccgga ggttcctggg tggctaataa ctacttc 37

<210> 41
<211> 35
<212> DNA
<213> Artificial Sequence

<220>
<223> Description of Artificial Sequence:oligonucleotide primer

<400> 41
gggggtctag agtcaccaca aaatgaatct atggc 35

<210> 42
<211> 22
<212> DNA
<213> Artificial Sequence

<220>
<223> Description of Artificial Sequence:oligonucleotide primer

<400> 42

gggggatcc cagtactttg gc 22

<210> 43
<211> 33
<212> DNA
<213> Artificial Sequence

<220>
<223> Description of Artificial Sequence:oligonucleotide primer

<400> 43
ggggggatcc cgcagttggt aacaagcttg tcg 33

<210> 44
<211> 29
<212> DNA
<213> Artificial Sequence

<220>
<223> Description of Artificial Sequence:oligonucleotide primer

<400> 44
ggggggatcc cagcgacacc cgaggcacg 29

<210> 45
<211> 34
<212> DNA
<213> Artificial Sequence

<220>
<223> Description of Artificial Sequence:oligonucleotide primer

<400> 45
gggggtctag accacacagg agaatatcca cgtc 34

<210> 46
<211> 36
<212> DNA
<213> Artificial Sequence

<220>
<223> Description of Artificial Sequence : oligonucleotide primer

<400> 46
ggggggcatg ctgtaggtgg gccatggatc aagtcc 36

<210> 47
<211> 33
<212> DNA
<213> Artificial Sequence

<220>
<223> Description of Artificial Sequence: oligonucleotide primer

<400> 47
ggggtcgacg tcaccacaaa atgaatctat ggc 33

<210> 48

<211> 33
<212> DNA
<213> Artificial Sequence

<220>
<223> Description of Artificial Sequence:oligonucleotide primer

<400> 48
ggggggcatg ccacctgggc caagaaattg acc 33

<210> 49
<211> 31
<212> DNA
<213> Artificial Sequence

<220>
<223> Description of Artificial Sequence:oligonucleotide primer

<400> 49
ggggaattcc acacaggaga atatccacgt c 31

<210> 50
<211> 24
<212> DNA
<213> Artificial Sequence

<220>
<223> Description of Artificial Sequence:oligonucleotide primer

<400> 50
gctggccgcg taatgctggt tggg 24

<210> 51
<211> 22
<212> DNA
<213> Artificial Sequence

<220>
<223> Description of Artificial Sequence:oligonucleotide primer

<400> 51
caactatgcc gaattcacgg cc 22

<210> 52
<211> 26
<212> DNA
<213> Artificial Sequence

<220>
<223> Description of Artificial Sequence:oligonucleotide primer

<400> 52
ccgtgctgga tgtgaggccg ttaccg 26

<210> 53
<211> 22
<212> DNA
<213> Artificial Sequence

<220>
<223> Description of Artificial Sequence:oligonucleotide primer

<400> 53
ggccgtgaat tcggcatagt tg 22

<210> 54
<211> 37
<212> DNA
<213> Artificial Sequence

<220>
<223> Description of Artificial Sequence:oligonucleotide primer

<400> 54
ggggggatcc atgttatcta tgattgtatt gctattc 37

<210> 55
<211> 32
<212> DNA
<213> Artificial Sequence

<220>
<223> Description of Artificial Sequence:oligonucleotide primer

<400> 55
ggggggatcc atttctgtga ttgatgcagc gc 32

<210> 56
<211> 35
<212> DNA
<213> Artificial Sequence

<220>
<223> Description of Artificial Sequence:oligonucleotide primer

<400> 56
ggggggtcta gacaacacaa ctatgccgaa ttcac 35

<210> 57
<211> 38
<212> DNA
<213> Artificial Sequence

<220>
<223> Description of Artificial Sequence:oligonucleotide primer

<400> 57
gggggtctag aggcatattc ataacccgtg tgcactac 38

<210> 58
<211> 23
<212> DNA
<213> Artificial Sequence

<220>
<223> Description of Artificial Sequence:oligonucleotide primer

<400> 58
atggtatggc tagcatgact ggt 23

<210> 59
<211> 35
<212> DNA
<213> Artificial Sequence

<220>
<223> Description of Artificial Sequence:oligonucleotide primer

<400> 59
ggggggatcc tttgaccgga ccggccacat gggtc 35

<210> 60
<211> 23
<212> DNA
<213> Artificial Sequence

<220>
<223> Description of Artificial Sequence:oligonucleotide primer

<400> 60
caaacaacag atggctggca act 23

<210> 61
<211> 38
<212> DNA
<213> Artificial Sequence

<220>
<223> Description of Artificial Sequence:oligonucleotide primer

<400> 61
gggggtctag agtaagtaaa attacgagcc tctgcagc 36

## Description

### Field of the invention

The invention belongs to the field of animal health and in equine arteritis virus (EAV). The invention provides vaccine compositions consisting of open reading frame (ORF) 2b,ORF 5 and ORF 7 nucleic acid of EAV, nucleic acid said ORF2b,ORF 5 and ORF 7 and vectors comprising said ORFs said nucleic acids being contained in a single vector backbone or in a plurality of vector backbones, each of which comprises regulatory sequences. The invention further relates to the use of said ORFs and vectors in the manufacture of a medicament for the prevention and treatment of EAV infections.

### Background of the invention

Equine arteritis is a contagious disease of horses and is spread via respiratory or reproductive tract and caused by equine arteritis virus (EAV) which is a member of the *Arteriviridae* family, that includes lactate dehydrogenase-elevating virus (LDV), porcine reproductive and respiratory syndrome virus (PRRSV), and simian haemorrhagic fever virus (SHFV).

EAV is well investigated and its biological and biophysical properties together with the data on viral pathogenesis and cell virus interactions had been documented in over 200 scientific reports (enclosure 1). The genomic organization and transcriptional strategy of arteriviruses are shown in Fig. 1. The EAV virions are 60-65 nm in diameter and possess a 49S RNA genome that is a single-stranded, nonsegmented, capped and polyadenylated message-sense RNA (12687 nucleotides; den *Boon, et al.* 1991; GenBank accession number: X53459). The EAV genome is infectious and contains at least eight open reading frames (ORF) 1a, 1b, 2, 3, 4, 5, 6, and 7 (SEQ ID No. 10). The two largest viral ORFs (ORF 1a and ORF 1b) encode the viral replicase (den *Boon, et al.* 1991) and are located at the 5'-end of the viral genome between the nucleotide positions 1 and 9807. The ORFs 2 to 7 are overlapping and located at the 3'-end of EAV genome. These ORFs encode four known structural viral proteins and two proteins of unknown function (gene products of ORFs 3 and 4). The EAV virion consists of a phosphorylated nucleocapsid protein (N, 14 kDa, gene product of ORF 7), a N-glycosylated major membrane protein ($G_L$, 30-44 kDa, gene product of ORF 5), a N-glycosylated minor membrane protein ($G_S$, 25 kDa, gene product of (ORF 2b), and an unglycosylated membrane protein (M, 17 kDa, gene product of ORF 6). Tobiash et al. describes that the large envelope glycoprotein and the nucleocapsid protein of EAV induce a immune response in Balb/c mice by DNA vaccination (Virus Genes, vol. 22, no. 2, March 2001, pp. 1787-199). Chow Y.H. et al. discusses the improvement of Hepatitis B Virus DNA vaccines by plasmids coexpressing Hepatitis B surface antigen and interleukin-2 (Journal of Virology, vol. 71, no. 1, January 1997, pp. 169-178) and Krieg A.M. et al. discusses the role of CpG dinucleotides in DNA vaccines (Trends in Microbiology, vol. 6, no. 1, January 1998, pp. 23-27). A novel structural protein of arteriviruses is identified by Snijder E. et al. (Journal of Virology, August 1999, vol. 73, no. 8, pp. 6335-6345).

The EAV infection is maintained in horse population, because chronic carrier animals shed EAV in their semen and transmit it venereally to mares during the mating season. PRRSV is also characterized by reproductive failure e.g. late-term abortions in sows and causing respiratory illness and mortality in young pigs.

The analysis of the genetic stability of EAV during horizontal and vertical transmission in an outbreak of equine viral arteritis revealed that the carrier stallion is the source of genetic diversity of EAV (Balasuriya *et a1.,1999).* It is known that the infected carrier stallion is the critical natural reservoir of EAV. The outbreak of an EAV infection can be initiated by the horizontal aerosol transmission of specific viral variants that occur in the semen of carrier stallions. However, Patton and co-workers show that not only the carrier stallion is the critical natural reservoir of EAV, but also genetic diversity of the virus is generated in the course of persistent infection of carrier stallions (Patton *et al.,* 1999).

Consequently, PRRSV and EAV cause economically important infectious diseases in swine and horse farms worldwide. The development of an efficient vaccine is of particular importance, since it focuses attention on the prevention of the diseases.

In the art, there was a long lasting need for an effective EAV vaccine capable of preventing or curing an EAV-associated disease. Therefore, the technical problem underlying this invention was to provide such a vaccine capable of preventing or curing an EAV-associated disease.

### Figures

Fig. 1
Schematic diagram of the genomic organisation and transcriptional strategy of the family *Arteriviridae.*

Fig. 2:
Schematic diagram of the strategy used for molecular cloning of neutralizing domain of equine arteritis virus (EAV).

Fig. 3:
The results of neutralization tests obtained by the analysis of the sera of the individual Balb/c mice that were inoculated in two independent experiments (A and B) with the DNA of recombinant plasmid pCR3.1-EAV-O5-BX-C14 harboring and expressing ORF 5 of equine arteritis virus (EAV).

Fig. 4:
The results of neutralization test obtained by the analysis of the sera of the individual Balb/c mice that were inoculated with the DNA of recombinant plasmids pCR3.1-EAV-O5-BX-C14 and pCR3.1-EAV-O7-BX-C3 harboring and expressing ORFs 5 and 7 of equine arteritis virus (EAV).

Fig. 5:
The results of neutralization test obtained by the analysis of the sera of the individual Balb/c mice that were inoculated with the DNA of recombinant plasmids pDP-EAV-O5-BsS-C2 and pDP-EAV-O7-BsS-C1 harboring and expressing ORFs 5 and 7 of equine arteritis virus (EAV).

Fig. 6:
The results of neutralization test obtained by the analysis of the sera of the individual Balb/c mice that were inoculated with the DNA of recombinant plasmids pCR3.1-EAV-O5-BX-C14 and pCR3.1-EAV-06-BE-C4 harboring and expressing ORFs 5 and 6 of equine arteritis virus (EAV).

Fig. 7:
The results of neutralization test obtained by the analysis of the sera of the individual Balb/c mice that were inoculated with the DNA of recombinant plasmid pCR3.1-EAV-O4-BX-C3 harboring and expressing ORF 4 of equine arteritis virus (EAV). The individual animals are indicated with number 1 to 10.

Fig. 8:
The results of neutralization test obtained by the analysis of the sera of the individual Balb/c mice that were inoculated with the DNA of recombinant plasmid pCR3.1-EAV-O4-BE-C3 harboring and expressing ORF 4 of equine arteritis virus (EAV). The individual animals are indicated with number 1 to 10.

Fig. 9:
The results of neutralization tests obtained by the analysis of the sera of the individual Balb/c mice that were inoculated with the DNA of recombinant plasmid pCR31-EAV-O5-del-121 harboring and expressing the N-terminal hydrophilic ectodomain of $G_L$ envelope glycoprotein (amino acid residue 1-121 of ORF 5) of equine arteritis virus (EAV).

Fig. 10:
The results of neutralization test obtained by the analysis of the sera of the individual Balb/c mice that were inoculated with the DNA of recombinant plasmids pCR3.1-EAV-O2-BX-C5, pCR3.1-EAV-O5-BX-C14, and pCR3.1-EAV-O6-BE-C4 harboring and expressing ORFs 2b (small glycoprotein), 5 (large envelope glycoprotein), and 6 (membrane protein), of equine arteritis virus (EAV).

Fig. 11:
The results of neutralization test obtained by the analysis of the sera of the individual Balb/c mice that were inoculated with the DNA of recombinant plasmids pCR3.1-EAV-O2-BX-C5 and pCR3.1-EAV-O4-BX-C3 harboring and expressing ORFs 2b and 4 of equine arteritis virus (EAV).

Fig. 12:
An example of the results obtained by enzyme linked immunosorbent assay (ELISA) for the detection of EAV specific antibodies.

Fig. 13: Establishing a lymphoproliferation assay for detection of cellular immune response

Fig. 14 (Fig 14-14b) Determination of the maximum cellular $^{51}$Cr-uptake

Fig. 15 (Fig. 15a-15e) Calculated specific lysis after the 2nd and 3rd booster immunization

**Disclosure of the invention**

Definitions of terms used in the description:

Before the embodiments of the present invention it must be noted that as used herein and in the appended claims, the singular forms "a", "an", and "the" include plural reference unless the context clearly dictates otherwise. Thus, for example, reference to "a nucleic acid molecule" includes a plurality of such nucleic acid molecules, reference to the "vector" is a reference to one or more vectors and equivalents thereof known to those skilled in the art, and so forth. Unless defined otherwise, all technical and scientific terms used herein have the same meanings as commonly understood by one of ordinary skill in the art to which this invention belongs.

Although any methods and materials similar or equivalent to those described herein can be used in the practice or testing of the present invention, the preferred methods, devices, and materials are now described. All publications mentioned herein are incorporated herein by reference for the purpose of describing and disclosing the cell lines, vectors, and methodologies as reported in the publications which might be used in connection with the invention. Nothing herein is to be construed as an admission that the invention is not entitled to antedate such disclosure by virtue of prior invention.

The term "EAV" as used herein refers to all viruses belonging to species equine arteritis virus within the family Arteriviridae.

A "fragment" according to the invention is any subunit of a DNA molecule (e.g. part of an open reading frame (ORF)) of a longer DNA molecule (e.g. an entire ORF) EAV according to the invention, i.e. any subset, characterized in that it is encoded by a shorter nucleic acid molecule than disclosed which can still be transcribed into RNA. "Fragment" also relates to subsets of proteins, i.e. smaller proteins encoded by said DNA fragments. The expression is to be understood depending upon the context in which it is used.

A "functional variant" of the DNA molecule according to the invention or protein encoded thereby is a DNA molecule or protein which possesses a biological activity (either functional or structural) that is substantially similar to the DNA molecule or protein according to the invention. The term "functional variant" also includes "a fragment", "a functional variant", "variant based on the degenerative nucleic acid code" or "chemical derivative". Such a "functional variant" e.g. may carry one or several nucleic acid exchanges, deletions or insertions. Said exchanges, deletions or insertions may account for 10% of the entire sequence. Said functional variant at least partially retains its biological activity, e.g. function as an infectious clone or a vaccine strain, or even exhibits improved biological activity.

A "variant based on the degenerative nature of the genetic code" is a variant resulting from the fact that a certain amino acid may be encoded by several different nucleotide tripletts. Said variant at least partially retains its biological activity, or even exhibits improved biological activity.

According to the invention, "mutation" means the replacement of a nucleotide by another (e.g. C for a T) a so-called "substitution" or any other mutation such as "deletion" or "insertion". "Deletion" means the removal of one or several nucleotides or amino acids.

A "fusion molecule" may be the DNA molecule or protein according to the invention fused to e.g. a reporter such as a radiolabel, a chemical molecule such as a fluorescent label or any other molecule known in the art.

As used herein, a "chemical derivative" according to the invention is a DNA molecule or protein according to the invention chemically modified or containing additional chemical moieties not normally being part of the molecule. Such moieties may improve the molecule's solubility, absorption, biological half life etc.

A molecule is "substantially similar" to another molecule if both molecules have substantially similar nucleotide sequences or biological activity. Thus, provided that two molecules possess a similar activity, they are considered variants as that term is used herein if the nucleotide sequence is not identical, and two molecules which have a similar nucleotide sequence are considered variants as that term is used herein even if their biological activity is not identical.

The terms "vaccine" and "vaccine composition" are used interchangeably.

The term "vaccine" as used herein refers to a pharmaceutical composition comprising at least one immunologically active component that induces an immunological response in an animal and possibly but not necessarily one or more additional components that enhance the immunological activity of said active component. A vaccine may additionally comprise further components typical to pharmaceutical compostions. The immunologically active component of a vaccine may comprise complete virus particles in either their original form or as attenuated particles in a so called modified live vaccine (MLV) or particles inactivated by appropriate methods in a so called killed vaccine (KV). In another form the immunologically active component of a vaccine may comprise appropriate elements of said organisms (subunit vaccines) whereby these elements are generated either by destroying the whole particle or the growth cultures containing such particles and optionally subsequent purification steps yielding the desired structure(s), or by synthetic processes including an appropriate manipulation by use of a suitable system based on, for example, bacteria, insects, mammalian or other species plus optionally subsequent isolation and purification procedures, or by induction of said synthetic processes in the animal needing a vaccine by direct incorporation of genetic material using suitable pharmaceutical compositions (polynucleotide vaccination). A vaccine may comprise one or simultaneously more than one of the elements described above.

The term "vaccine" as understood herein is a vaccine for veterinary use comprising antigenic substances and is administered for the purpose of inducing a specific and active immunity against a disease provoked by EAV. The EAV vaccine according to the invention confers active immunity that may be transferred passively via maternal antibodies against the immunogens it contains and sometimes also against antigenically related organisms.

Additional components to enhance the immune response are constituents commonly referred to as adjuvants, like e.g. aluminiumhydroxide, mineral or other oils or ancillary molecules added to the vaccine or generated by the body after the respective induction by such additional components, like but not restricted to interferons, interleukins or growth factors.

A "vaccine composition" essentially consists of one or more ingredients capable of modifying physiological e.g. immunological functions of the organism it is administered to, or of organisms living in or on the organism. The term includes, but is not restricted to antibiotics or antiparasitics, as well as other constituents commonly used to achieve certain other objectives like, but not limited to, processing traits, sterility, stability, feasibility to administer the composition via enteral or parenteral routes such as oral, intranasal, intravenous, intramuscular, subcutaneous, intradermal or other suitable route, tolerance after administration, controlled release properties.

Disclosure of the invention

The solution to the above technical problem is achieved by the description and the embodiments characterized in the claims.

The long lasting need in the art for a vaccine capable of preventing or curing an EAV-associated disease has been overcome by the present inventors who provided a such a nucleic acid-based prophylactic or therapeutic vaccine for EAV-associated diseases. Said vaccine is described in more details *infra.*

Surprisingly, the nucleic acid-based vaccine according to the invention for the first time in the art is capable of not only generating a humoral (antibody-based) response (demonstrated in an exemplary manner in e.g. example 1), but also a cellular immune response in horses. Only this cellular immune response, as exemplified in examples 2 to 5, is protective against horizontal and vertical EAV transmission in horses. More surprisingly, quite contrary to what was expected by the artisan (Barry and Johnston, 1997) the vaccine according to the invention is protective against infection not only in young horses, but also in horses of all ages (as exemplified by data, see table 8 and 10). Thus, the vaccines according to the invention as described *infra* are capable of inducing a cellular immune response in horses and are protective against horizontal and vertical EAV transmission in horses of different ages.

In a first important embodiment, the invention relates to a vaccine composition which is protective against equine arterivirus (EAV) infections in horses and induces a cellular immune response, consisting of open reading frame nucleic acid (ORF) 2b, ORF 5 and ORF7 of EAV, said nucleic acids being contained in a single vector backbone or a plurality of vector backbones, each of which comprises regulatory sequences. The invention also relates to vaccine compositions wherein said ORF 2b, ORF5, and ORF7 are fragments, functional variants or carry mutations as defined *supra.*

To prepare such nucleic acids, the artisan may follow the examples of the present invention and apply also standard molecular biology methods which can be found e.g. in Sambrook et al.(1989) Molecular Cloning. A Laboratory Manual, 2nd ed., Cold Spring Harbor Laboratory Press, Cold Spring Harbor, New York and Bertram, S. and Gassen, H.G. Gentechnische Methoden, G. Fischer Verlag, Stuttgart, New York, 1991).

Surprisingly, contrary to the opinion in the art, a vaccine comprising nucleic acid said three ORFs is particularly effective and much better than a vaccine wherein the entire cDNA for EAV is used. ORF 2b encodes a small glycoprotein. ORF 5 encodes a large envelope glycoprotein. ORF 7 encodes the nucleocapsid protein. The invention also relates to vaccine compositions comprising mutated or truncated ORFs, such as ORF 5 in a deleted form (SEQ ID No. 9).

The invention further relates to a vaccine composition according to the invention as disclosed *supra,* wherein said vaccine composition further comprises one ORF selected from the group of ORF 1a, ORF 1b, ORF 3, ORF 4, ORF 6. ORF 1a and ORF 1b encode the viral replicase. ORF 3 and ORF 4 encode proteins of yet unknown function. ORF 6 encodes an unglycosylated membrane protein. Preferably, said ORF or ORFs are as disclosed in SEQ ID No. 1 or SEQ ID No. 8, SEQ ID No. 3, SEQ ID No. 4, SEQ ID No. 6.

The invention also relates to vaccine compositions comprising mutated or truncated ORFs, such as the partial ORF1 sequence in SEQ ID No. 8.

The invention further relates to a vaccine composition according to the invention as disclosed *supra,* wherein said nucleic acid is cDNA.

The invention further relates to a vaccine composition according to the invention as disclosed *supra,* wherein said vaccine composition consists of one or several nucleic acid vectors each comprising said ORFs. One embodiment of the invention relates to such a vector comprising more than one ORF.

The invention further relates to a vaccine composition according to the invention as disclosed *supra*, wherein said vector(s) is/are expression vector(s).

The invention further relates to a vaccine composition according to the invention as disclosed *supra,* wherein said expression vector(s) further comprises a eukaryotic cis-acting transcription/translation sequence functionally linked to said ORF(s).

The invention further relates to a vaccine composition according to the invention as disclosed *supra,* wherein said expression vector is selected from the group of pCR3.1, pcDNA3.1/His A, pcDNA3.1/His B, pcDNA3.1/His C, and pDisplay (pD) Such vectors are commercially available (Invitrogen).

The invention further relates to a vaccine composition according to the invention as disclosed *supra,* further comprising the nucleic acid encoding interleukin 2 (IL-2) or a vector or expression vector comprising said nucleic acid encoding IL-2.

The invention further relates to a vaccine composition according to the invention as disclosed *supra,* further comprising pharmaceutically acceptable carrier or excipient.

The invention further relates to a vaccine composition according to the invention as disclosed *supra,* further comprising one or several adjuvants selected from the group of Muramyl Dipeptide (MDP), Montanide 720, Poly Inosine: Cytosine (Poly I:C) or plasmid DNA comprising unmethylated cytosine, guanine dinucleotide sequence motifs (CpG).

The invention relates to a vaccine according to the invention as described *supra* wherein the adjuvants is any one of the compounds described in Chapter 7 (pp 141-227) of "Vaccine Design, The Subunit and Adjuvant Approach" (eds. Powell, M. F. and Newman, M. J.) Pharmaceutical Biotechnology, Volume 6, Plenum Press (New York). Examples from this compendium include Muramyl Dipeptide (MDP) and Montanide 720 as disclosed *supra.* Molecules such as Poly Inosine:Cytosine (Poly I:C) or "immunostimulatory nucleic acid molecules" such as plasmid DNA containing CpG motifs can also be administered as adjuvants in combination with antigens encapsulated in microparticles. An "immunostimulatory nucleic acid molecule" refers to a nucleic acid molecule, which contains an unmethylated cytosine, guanine dinucleotide sequence (i.e. "CpG DNA" or DNA containing a cytosine followed by guanosine and linked by a phosphate bond) and stimulates (e.g. has a mitogenic effect on, or induces or increases cytokine expression by) a vertebrate lymphocyte. An immunostimulatory nucleic acid molecule can be double-stranded or single-stranded. Generally, double-stranded molecules are more stable *in vivo,* while single-stranded molecules have increased immune activity. The instant invention is based on the finding that certain "immunostimulatory nucleic acid molecules" containing unmethylated cytosine-guanine (CpG) dinucleotides activate lymphocytes in a subject and redirect a subject's immune response from a Th2 to a Th1 (e.g. by inducing monocytic cells and other cells to produce Th1 cytokines, including IL-12, IFN-gamma. and GM-CSF).

The invention further relates to a vaccine composition according to the invention as disclosed *supra,* consisting of expression vectors comprising ORF2b, ORF5 and ORF7 of EAV, respectively, and optionally carrier, excipients or adjuvants and an expression vector comprising the nucleic acid encoding IL-2. Said nucleic acid is preferably equine IL-2. Preferably also, said the coding nucleic acid equine IL-2 is co-expressed on a vector as disclosed supra encoding one or several EAV ORFs.

The invention further relates to a vaccine composition according to the invention as disclosed *supra,* wherein ORF 2b is SEQ ID No. 2, ORF 5 is SEQ ID No. 5 or SEQ ID No. 9 and ORF 7 is SEQ ID No. 7.

Suitable for targeted delivery of the vaccine composition according to the invention are colloidal dispersion systems or liposomes. One example of a targeted delivery system for the EAV ORF nucleic acid molecules according to the invention is said colloidal dispersion system. Colloidal dispersion systems include macromolecule complexes, nanocapsules, microspheres, beads, and lipid-based systems including oil-in-water emulsions, micelles, mixed micelles, and liposomes or liposome formulations. The preferred colloidal system of this invention is a liposome. Liposomes are artificial membrane vesicles which are useful as delivery verhicles in vitro and in vivo. These formulations may have net cationic, anionic or neutral charge characteristics are useful characteristics with in vitro, in vivo and ex vivo delivery methods. It has been shown that large unilamellar vesicles (LUV), which range in size from 0.2-4.0 μm can encapsulate a substantial percentage of an aqueous buffer containing large macromolecules. RNA, DNA and intact virions can be encapsulated within the aqueous interior and be delivered to cells in a biologically active form (Fraley R and Papahadjopoulos D (1981). New generation liposomes - The engineering of an efficient vehicle for intracellular delivery of nucleic acids. Trends Biochem Sci 6, 77-80). In addition to mammalian cells, liposomes have been used for delivery of polynucleotides in plant, yeast and bacterial cells. In order for a liposome to be an efficient gene transfer vehicle of the EAV ORFs according to the invention, the following characteristics should be present: (1) encapsulation of the genes of interest at high efficiency while not compromising their biological activity; (2) preferential and substantial binding to a target cell in comparison to non-target cells; (3) delivery of the aqueous contents of the vesicle to the target cell cytoplasm at high efficiency; and (4) accurate and effective expression of genetic information (Mannino RJ, and Gould-Fogerite S (1988). Liposome mediated gene transfer. BioTechniques 6, 682-690.).

The composition of the liposome is usually a combination of phospholipids, particularly high-phase-transition-temperature phospholipids, usually in combination with steroids, especially cholesterol. Other phospholipids or other lipids may also be used. The physical characteristics of liposomes depend on pH, ionic strength, and the presence of divalent cations.

The vaccine composition of the present invention may contain said recombinant vector as a naked "gene expression vector". This means that the construct is not associated with a delivery vehicle (e.g. liposomes, colloidal particles and the like). One of the principal advantages of naked DNA vectors is the lack of a immune response stimulated by the vector itself.

The invention further relates to a vaccine composition according to the invention as disclosed *supra,* wherein the nucleic acid or nucleic acid vector or expression vector is encapsulated into liposomes.

Several types of liposomal preparations may be used for encapsulation, including large multilamellar vesicles, small unilamellar vesicles, neutral, anionic liposomes or simple cationic amphiphiles. Most preferred are cationic liposomes.

These synthetic gene delivery systems are described by many terms:

The cationic lipid-mediated transfection has been also called liposome-mediated-transfection, cationic liposome-mediated transfection, lipofection, cytofection, amphifection, and lipid-mediated transfection. Similarly, the complexes that are produced when cationic lipids are mixed with DNA have been referred to as cytosomes, amphisomes, liposomes, nucleolipidic particles, cationic lipid-DNA complexes, lipid-DNA complexes, DNA-lipid complexes etc. Recently, a common nomenclature was proposed: *Lipoplex* - replaces all of the terms for cationic lipid-nucleic acid complexes (including DNA, RNA, or synthetic oligonucleotides) and *lipofection* means the nucleic acid delivery mediated by lipoplexes. Any of said gene delivery system may be used according to the invention.

The positive charge-on cationic lipid molecules facilitates their association with negatively charged nucleic acid as well as with membrane phospholipids (negatively charged) what is the basis for the non-specific interaction of the complex.

The specific binding to the cell is mediated by use of specific ligands for cellular receptors. Cationic Liposomes may deliver DNA either directly across the plasma membrane or via endosome compartment. Regardless of its exact entry point, much of the DNA does accumulate in the endosomes and is lost by the internal hydrolytic digestion within the endosomes. To protect the plasmid DNA several strategies may be used according to the invention. This includes the use of acidotropic, weak amines such as chloroquine, which presumably prevent DNA degradation by inhibiting endosomol acidification. But also viral fusion peptides or whole viruses may be included to disrupt endosomes or promote fusion of liposomes with endosomes and facilitate release of DNA into the cytoplasm. Such protection of the plasmid DNA is also a preferred embodiment of the invention.

The DNA concentration, the ratio of lipid reagent to DNA, the transfection time and the effect of serum are the most critical factors in each transfection.

Liposomes must be stable. In case of leakage they would lose antigen and adjuvants premature.

Preferred is a vaccine composition according to the invention as disclosed *supra* comprising 0.05 μg - 10 μg, preferred 0. 1μg - 1μg, most preferred 0.5 μg. Further preferred dose ranges are: low range 0.1-1.0 μg, most preferred 0.5 μg, middle range 1.1-10 μg, most preferred 15 μg, high range 11μg-20μg, most preferred 15 μg. The artisan knows the criteria for the ideal dose which depends on the chosen route of administration, i.e. with gene gun the vaccine is injected directly into Langerhans cells, thus very little of antigen gets lost, whereas i.m. injection requires much higher doses.

Most preferred is a vaccine composition according to the invention as disclosed *supra* comprising 0.5 μg of individual nucleic acid vector or preferred expression vector and preferably for 10 shots per animal, i.e. 5μg of individual nucleic acid vector (or preferred expression vector) per vaccination, e.g. if seven nucleic acid vectors (or preferred expression vectors) are used, 35 μg per vaccination and animal (see example 2).

Also preferred is a vaccine composition according to the invention as disclosed *supra* comprising: low range 10-100 μg, most preferred 50 μg, middle range 101-500 μg, most preferred 200 μg, high range 501 μg-2000 μg, most preferred 1000 μg. Again, the artisan knows the criteria for the ideal dose which depends on the chosen route of administration, i.e. with gene gun the vaccine is injected directly into Langerhans cells, thus very little of antigen gets lost, whereas i.m. injection requires much higher doses.

Also most preferred is a vaccine composition according to the invention as disclosed *supra* comprising 50 μg of individual nucleic acid vector or preferred expression vector and preferably for 4 injections per animal, i.e. 200 μg of individual nucleic acid vector (or preferred expression vector) per vaccination, e.g. if seven nucleic acid vectors (or preferred expression vectors) are used, 1,4 mg per vaccination and animal (see example 2).

In yet another important embodiment the invention relates to a nucleic acid vector consisting of nucleic acids ORF 2b ORF 5, and ORF7 of EAV, and a vector backbone and regulatory sequences. Preferably, said ORFs are as disclosed in SEQ ID No. 2, SEQ ID No. 5, and SEQ ID No. 7.

A preferred aspect of the invention is a nucleic acid vector according to the invention as disclosed *supra,* wherein said nucleic acid is DNA.

A more preferred aspect of the invention is a nucleic acid vector according to the invention as disclosed *supra,* wherein said nucleic acid vector is an expression vector.

Another more preferred aspect of the invention is a nucleic acid vector according to the invention as disclosed *supra,* wherein said expression vector comprises a eukaryotic cis-acting transcription/translation sequence functionally linked to said ORF(s). To accomplish expression, a wide variety of vectors have been developed and are commercially available which allow inducible (e.g., LacSwitch expression vectors, Stratagene, La Jolla, CA) or cognate (e.g., pcDNA3 vectors, Invitrogen, Chatsworth, CA) expression of EAV ORF nucleotide sequences under the regulation of an artificial promoter element. Such promoter elements are
often derived from CMV of SV40 viral genes, although other strong promoter elements which are active in eukaryotic cells can also be employed to induce transcription of EAV ORF nucleotide sequences. Typically, these vectors also contain an artificial polyadenylation sequence and 3' UTR which can also be derived from exogenous viral gene sequences or from other eukaryotic genes. Furthermore, in some constructs, artificial, non-coding, spliceable introns and exons are included in the vector to enhance expression of the nucleotide sequence of interest (in this case, EAV ORF sequences). These expression systems are commonly available from commercial sources and are typified by vectors such as pCDNA3 and pZeoSV (Invitrogen, San Diego, CA). Innumerable commercially-available as well as custom-designed expression vectors are available from commercial sources to allow expression of any desired EAV ORF transcript in more or less any desired cell type, either constitutively or after exposure to a certain exogenous, stimulus (e.g., withdrawal of tetracycline or exposure to IPTG).

A most preferred aspect of the invention is a nucleic acid according to the invention as disclosed *supra,* wherein said expression vector is selected from the group of pCR3.1, pcDNA3.1/His A, pcDNA3.1/His B, pcDNA3.1/His C, and pDisplay (pD).

Another most preferred aspect of the invention is a nucleic acid according to the invention as disclosed *supra,* wherein said nucleic acid vector consists of a nucleic acid selected from the group of SEQ ID No. 2, SEQ ID No. 5, SEQ ID No. 9 and/or SEQ ID No. 7, and a vector backbone and regulatory sequences.

Gene gun is a ballistic system that can propel DNA-coated microparticles directly into the skin. Plasmid DNA is affixed to gold particles of about 0,45 μm. The total amount of DNA per sheet is a function of the DNA/gold ratio, i.e. 5 μg DNA/mg gold. If about 0,4 mg of gold particles are shoot into the epidermis/dermis driven by helium (helium discharge pressure of 400-450 psi), about 2 μg of DNA is inoculated into the skin.
Therefore, the fundamental difference of gene gun vs. i.m. injection is the amount of DNA required to produce an equivalent level of gene expression. This apparent difference could most likely related to the fact that i.m. or i.d. injections by needle places the DNA also into extracellular spaces, exposure of DNA to nucleases in the intestitial fluid or that the hydrostatic pressure that results from the injection of several μl of saline into a muscle rapidly drives out the DNA of protein producing cells so that only a little amount of injected DNA will be expressed in protein.

Interestingly, the gun is in general limited by the little amount of plasmid that can be delivered in one inoculation. This limit is about 2,5 μg of plasmid per shot. Exceeding this amount causes the particles to clump together; creating 'macroparticles' which cause increased damage to the target tissue. By contrast, milligrams of plasmid DNA can be delivered by i.m. injections using needles and the success of i.m. injection can be easily monitored by swelling of the injected muscle bundle. Qualitative differences in immunisation by the gene gun and i.m. injection can be circumvented by increasing the amount of DNA. A DNA inoculation by gene gun needs almost hairless skin. So the most animals to be vaccinated have to get shaved.

Such problems should be overcome by new generations of gene guns which also may be used according to the invention. A new device, the PowderJect® system driven by helium gas, is the first one which is able to deliver both metallic and non-metallic particles into tissues. Most preferred is the use of the PowderJect® system.

Thus, another important embodiment of the invention is the use of one or several nucleic acid vector(s) according to the invention as disclosed supra, in the manufacture of a vaccine for the prophylaxis and treatment of EAV infections wherein said one or several nucleic acid vectors and compositions

(i) are to be coated onto carrier particles;

(ii) the coated carrier particles are to be accelerated into epidermal cells of the horse in *vivo* to induce a protective or therapeutic immune response in said horse upon of after exposure to EAV, where the reduction of EAV-associated symptoms or the reduction of horizontal or vertical transmission can be monitored.

Preferably, in said use as disclosed *supra,* the carrier particles are gold.

Any of the above-disclosed devices such as gene gun or the PowderJect ® system may be used. The injection may be carried out as disclosed *supra.* Most preferably, said method may be carried out repeatedly. An appropriate vaccination scheme may be preferably on day 0 (basic vaccination), 2 weeks therafter, 4 weeks after the basic vaccination and 7 weeks after the basic vaccination. This is exemplified in example 2.

Also preferred vaccination schemes are:

1) only one base immunization without booster;or
2) base immunization, 1st boost after 8-12 weeks; or
3) base immunization, 1st boost after 8-12 weeks, 2nd boost after 12 months.

Said vaccine, DNA molecule(s) according to the invention as disclosed *supra*, or one or several nucleic acid vector(s) according to the invention as disclosed *supra* may be administered by any known route of administration : preferably orally, nasally, lingually, intravenously (i.v.), intradermally (i.d.), intraepidermally (by rubbing into the skin), intranasally, vaginally, subcutaneously (s.c.), intramuscular (i.m.).

According to the invention, various vehicles for administration of the vaccine, DNA molecule(s) according to the invention as disclosed *supra,* or one or several nucleic acid vector(s) according to the invention as disclosed *supra* may be used: only plasmid 'naked' DNA inoculation by needle - liposomes - gold beads - biodegradable nanoparticles - virus like particles (VLP) - aerosol.

Preferred modes of administration for the vaccine, DNA molecule(s) according to the invention as disclosed *supra,* or one or several nucleic acid vector(s) according to the invention as disclosed *supra* are: injection by needle, gene gun, encapsulated in liposomes or rubbing into the skin.

Preferred doses are 0.5 μg of individual nucleic acid vector or preferred expression vector and 10 shots per animal, i.e. 5 μg of individual nucleic acid vector (or preferred expression vector) per vaccination, e.g. if seven nucleic acid vectors (or preferred expression vectors) are used, 35 μg per vaccination and animal (see example 2).

Preferred are 1 to 10, more preferably 5 or 7 or 10 shots per aninmal, most preferred are 10 shots per animal.

One preferred method of vaccination is the direct injection of plasmid DNA into skeletal muscle. Long-lasting immune responses are obtained in many cases without boost.

For the i.m. route, DNA preferably is injected by needle, whereas for the i.e. route, a gene gun preferably is used (see *supra).*

Yet another important embodiment of the invention is the use of one or several nucleic acid vector(s) according to the invention as disclosed supra, in the manufacture of a vaccine for the prophylaxis and treatment of EAV infections wherein said one or several nucleic acid vectors and compositions

(i) are to be injected into muscular cells of the horse *in vivo* to induce a protective or therapeutic immune response in said horse upon of after exposure to EAV, where the reduction of EAV-associated or the reduction of horizontal or vertical transmission can be monitored.

Most preferably, said use may be carried out repeatedly. An appropriate vaccination scheme may be preferably on day 0 (basic vaccination), 2 weeks therafter, 4 weeks after the basic vaccination and 7 weeks after the basic vaccination. This is exemplified in example 2. Preferred doses are 50 μg of individual nucleic acid vector or preferred expression vector and 4 inoculations per animal, i.e. 200 μg of individual nucleic acid vector (or preferred expression vector) per vaccination, e.g. if seven nucleic acid vectors (or preferred expression vectors) are used, 1,4 mg per vaccination and animal (see example 2).

Also preferred is a vaccine composition according to the invention as disclosed *supra* comprising: low range 10-100 μg, most preferred 50 μg, middle range 101-500 μg, most preferred 200 μg, high range 501 μg-2000μg, most preferred 1000μg. Again, the artisan knows the criteria for the ideal dose which depends on the chosen route of administration, i.e. with gene gun the vaccine is injected directly into Langerhans cells, thus very little of antigen gets lost, whereas i.m. injection requires much higher doses.

Again, preferred vaccination schemes are:

1) only one base immunization without booster;or
2) base immunization, 1st boost after 8-12 weeks; or
3) base immunization, 1st boost after 8-12 weeks, 2nd boost after 12 months.

Said vaccine, DNA molecule(s) according to the invention as disclosed *supra,* or one or several nucleic acid vector(s) according to the invention as disclosed *supra* may be administered by any known route of administration: preferably orally, intravenously (i.v.), intradermally, intraepidermally (by rubbing into the skin), intranasally, vaginally, subcutaneously (s.c.), intramuscular (i.m.).

Preferred modes of administration for the vaccine, DNA molecule(s) according to the invention as disclosed *supra,* or one or several nucleic acid vector(s) according to the invention as disclosed *supra* are: injection by needle, gene gun, encapsulated in liposomes or rubbing into the skin.

Preferred are 1 to 10, more preferably 3 or 4 or 5 inoculations per aninmal, most preferred are 4 inoculations per animal.

The invention further relates to the use of one or several DNA molecule(s) according to the invention as disclosed *supra* or one or several nucleic acid vector(s) according to the invention as disclosed *supra* in the manufacture of a vaccine for the prophylaxis and treatment of EAV infections. Preferred is the use of DNA molecule(s) or nucleic acid vector(s) according to the invention as disclosed *supra* in the manufacture of a vaccine suitable for intraepidermal, intradermal or intramuscular administration for the prophylaxis and treatment of EAV infections.

More preferred is the use of DNA molecule(s) or nucleic acid vector(s) according to the invention as disclosed *supra* in the manufacture of a vaccine suitable for intraepidermal or intradermal administration for the prophylaxis and treatment of EAV infections, wherein the vaccine is to be administered on day 0 (basic vaccination), 2 weeks therafter, 4 weeks after the basic vaccination and 7 weeks after the basic vaccination. This is exemplified in example 2.

More preferred is the use of DNA molecule(s) or nucleic acid vector(s) or expression vector according to the invention as disclosed *supra* in the manufacture of a vaccine suitable for intraepidermal or intradermal administration for the prophylaxis and treatment of EAV infections, wherein the vaccine comprises 0.5 μg of individual nucleic acid vector or preferred expression vector and preferably for 10 shots per animal, i.e. 5μg of individual nucleic acid vector (or preferred expression vector) per vaccination, e.g. if seven nucleic acid vectors (or preferred expression vectors) are used, 35 μg per vaccination and animal (see example 2).

Preferred is the use of DNA molecule(s) or nucleic acid vector(s) or expression vector according to the invention comprising 0.05 μg - 10 μg, preferred 0.1μg - 1 μg, most preferred 0.5 μg. Further preferred dose ranges are: low range 0.1-1.0 μg, most preferred 0.5 μg, middle range 1.1-10 μg, most preferred 15 μg, high range 11μg-20μg, most preferred 15 μg. The artisan knows the criteria for the ideal dose which depends on the chosen route of administration, i.e. with gene gun the vaccine is injected directly-into Langerhans cells, thus very little of antigen gets lost, whereas i.m. injection requires much higher doses.

Again, preferred vaccination schemes are:

1) only one base immunization without booster;or
2) base immunization, 1st boost after 8-12 weeks; or
3) base immunization, 1st boost after 8-12 weeks, 2nd boost after 12 months.

Said vaccine, DNA molecule(s) according to the invention as disclosed *supra,* or one or several nucleic acid vector(s) according to the invention as disclosed *supra* may be administered by any known route of administration: preferably orally, intravenously (i.v.), intradermally, intraepidermally (by rubbing into the skin), intranasally, vaginally, subcutaneously (s.c.), intramuscular (i.m.).

Preferred modes of administration for the vaccine, DNA molecule(s) according to the invention as disclosed *supra,* or one or several nucleic acid vector(s) according to the invention as disclosed *supra* are: injection by needle, gene gun, encapsulated in liposomes or rubbing into the skin.

Preferred are 1 to 10, more preferably 3 or 4 or 5 administrations per aninmal, most preferred are 4 administrations per animal.

More preferred is the use of DNA molecule(s) or nucleic acid vector(s) according to the invention as disclosed *supra* in the manufacture of a vaccine suitable for intraepidermal or intradermal administration for the prophylaxis and treatment of EAV infections, wherein the vaccine is to be administered on day 0 (basic vaccination), 2 weeks therafter, 4 weeks after the basic vaccination and 7 weeks after the basic vaccination. This is exemplified in example 2.

Preferred is the use of DNA molecule(s) or nucleic acid vector(s) or expression vector according to the invention comprising 0.05 μg - 10 μg, preferred 0.1μg - 1 μg, most preferred 0.5 μg. Further preferred dose ranges are: low range 0.1-1.0 μg, most preferred 0.5 μg, middle range 1.1-10 μg, most preferred 15 μg, high range 11μg-20μg, most preferred 15μg. The artisan knows the criteria for the ideal dose which depends on the chosen route of administration.

Again, preferred vaccination schemes are:

1) only one base immunization without booster;or
2) base immunization, 1st boost after 8-12 weeks; or
3) base immunization, 1st boost after 8-12 weeks, 2nd boost after 12 months.

Said vaccine, DNA molecule(s) according to the invention as disclosed *supra,* or one or several nucleic acid vector(s) according to the invention as disclosed *supra* may be administered by any known route of administration: preferably orally, intravenously (i.v.), intradermally, intraepidermally (by rubbing into the skin), intranasally, vaginally, subcutaneously (s.c.), intramuscular (i.m.).

Preferred modes of administration for the vaccine, DNA molecule(s) according to the invention as disclosed *supra,* or one or several nucleic acid vector(s) according to the invention as disclosed *supra* are: injection by needle, gene gun; encapsulated in liposomes or rubbing into the skin.

Preferred are 1 to 10, more preferably 3 or 4 or 5 administrations per aninmal, most preferred are 4 administrations per animal.

Also more preferred is the use of DNA molecule(s) or nucleic acid vector(s) or expression vector according to the invention as disclosed *supra* in the manufacture of a vaccine suitable for intramuscular administration for the prophylaxis and treatment of EAV infections, wherein the vaccine comprises 50 μg of individual nucleic acid vector or preferred expression vector and preferably for 4 shots per animal, i.e. 200 μg of individual nucleic acid vector (or preferred expression vector) per vaccination, e.g. if seven nucleic acid vectors (or preferred expression vectors) are used, 1,4 mg per vaccination and animal (see example 2).

Also preferred is use-of DNA molecule(s) or nucleic acid vector(s) or expression vector according to the invention as disclosed *supra* comprising: low range 10-100 μg, most preferred 50 μg, middle range 101-500 ug, most preferred 200 μg, high range 501μg-2000μg, most preferred 1000μg. Again, the artisan knows the criteria for the ideal dose which depends on the chosen route of administration, i.e. with gene gun the vaccine is injected directly into Langerhans cells, thus very little of antigen gets lost, whereas i.m. injection requires much higher doses.

Again, preferred vaccination schemes are:

1) only one base immunization without booster; or
2) base immunization, 1$^{st}$ boost after 8-12 weeks; or
3) base immunization, 1$^{st}$ boost after 8-12 weeks, 2$^{nd}$ boost after 12 months.

Said vaccine, DNA molecule(s) according to the invention as disclosed *supra,* or one or several nucleic acid vector(s) according to the invention as disclosed *supra* may be administered by any known route of administration: preferably orally, intravenously (i.v.), intradermally, intraepidermally (by rubbing into the skin), intranasally, vaginally, subcutaneously (s.c.), intramuscular (i.m.).

Preferred modes of administration for the vaccine, DNA molecule(s) according to the invention as disclosed *supra,* or one or several nucleic acid vector(s) according to the invention as disclosed *supra* are: injection by needle, gene gun, encapsulated in liposomes or rubbing into the skin.

Preferred are 1 to 10, more preferably 3 or 4 or 5 administrations per aninmal, most preferred are 4 administrations per animal.

The invention also relates to the a kit of parts comprising a vaccine composition according to the invention as disclosed *supra* or one or several EAV ORF DNA molecule(s) according to the invention as disclosed *supra* or one or several nucleic acid vector(s) according to the invention as disclosed *supra.* Said kit is ready-to-use for vaccination of horses. Said kit may further contain, but is not limited to test tubes, other suitable containers, washing reagents and reaction buffers (which may vary in pH and magnesium concentrations), sterile water, liposomal preparations, transfection reagent such as DOTAP Liposomal (Roche) or Lipofectin, BME (Eagle's basal medium), ethanol, gold, spermidine, $CaCl_2$, carrier proteins and further compounds known to the skilled artisan.

The following examples serve to further illustrate the present invention; but the same should not be construed as limiting the scope of the invention disclosed herein.

**Example 1 Neutralization tests** (example not falling under the scope of the invention)

## MATERIALS AND METHODS

Viruses and cells

The equine arteritis virus (EAV) used in this study was kindly provided by Professor H. Ludwig, Berlin and propagated on rabbit kidney cells (RK13, ATCC number CCL-37). The cell cultures were obtained from the American Type Culture Collection and propagated in Basal Medium Eagle (BME) supplemented with 10% fetal calf serum, 100 IE/ penicillin G; 100 IE /ml streptomycin. Medium and serum were purchased from GibcoBRL (Eggenstein, Germany).

Production of EAV-specific antisera

Antiserum against EAV was induced in New Zealand white rabbit. The animal was inoculated subcutaneously with 0.5 ml purified EAV. Inoculation was repeated for four times. The experimental protocol is summarized in Table 1. The sensitivity of rabbit antisera was determined by western blot analysis. It was found that the rabbit antiserum raised against EAV is able to recognize viral specific protein at the dilution of about 1:2000 and higher.

Preparation of viral RNA

Virion RNA and total infected cell RNA was prepared from EAV-infected RK13 cell cultures at 12, 24, 36, and 48 h p.i. using a guanidinium iso-thiocyanate/cesiumchloride procedure based on the method described by Glisin *et al.*

(1974). Infected cells or virions from clarified infected cell culture supernatants were dissolved in a 4.0 M guanidinium thiocyanate (GTC) solution. Cellular DNA in the infected cell preparation was sheared by repeatedly passing the solution through a 23-gauge needle. CsCl and sarcosyl (30% aqueous solution) were added to the GTC preparation to final concentrations of 0.15 g/ml and 3.0%, respectively. In volumes of 8 ml the preparation was transferred onto a 3 ml 5.7 M CsCl cushion and centrifuged at 29,000 rpm in a Beckman SW41 rotor for 24 h at 20°C. The supernatant was discarded and the RNA pellet was dissolved in RNase free $H_2O$ to a final concentration of about 10 μg/ml. RNA preparations were stored at-20°C in 80% ethanol containing 100 mM sodium acetate. As an alternative total RNA of EAV-infected cells was prepared using the RNeasy Mini Kit (Qiagen, Hilden, Germany) according to the instructions of the manufacturer.

First-strand cDNA Synthesis

For each first-strand cDNA synthesis reaction approximately 0.5 μg of purified RNA were pelleted and dissolved in 10 μl RNase free $H_2O$ containing 20 U RNase inhibitor (Takara Shuzo -Co., Ltd., Shiga, Japan). The reaction was prepared in 20 μl volumes using enzymes and reagents from the RNA LA PCR Kit Ver.1.1 (Takara Shuzo Co., Ltd., Shiga, Japan) according to the instructions of the manufacturer. The reaction included 5 mM $MgCl_2$, 1 mM of each dNTP, 10 pmol of a specific reverse oligonucleotide primer, and 5 U AMV reverse transcriptase XL. The reaction was incubated in an automated temperature cycling reactor (Genius, Techne, Cambridge, UK) for 2 min at 60°C followed by 15 min at 50°C. Then the temperature was gradually lowered to 42°C at a speed of 1°C/min. As a final step the reaction was incubated for 2 min at 80°C and rapidly cooled to 4°C. RNase free $H_2O$ was added to the reaction products to obtain a final volume of 100 μl. The first-strand cDNA stocks were stored at -20°C.

Oligonucleotides and polymerase chain reaction (PCR)

Specific oligonucleotides were synthesized with an Oligo 1000M DNA. Synthesizer (Beckman Instruments GmbH, München, Germany). The properties of the individual oligonucleotides primers are summarized in Table 2. Polymerase chain reaction (PCR) was performed in 100 μl volumes using TaKaRa LA *Taq* DNA polymerase (supplied with reaction buffer, Takara Shuzo Co., Ltd., Shiga, Japan). Each reaction contained 1.5-2.5 mM $MgCl_2$, 12.5 nmol of each dNTP (Boehringer Mannheim Biochemica, Mannheim, Germany), 50 pmol of each oligonucleotide primer, and 1 μl of a first-strand cDNA stock solution (see above). An improved PCR protocol was developed based on a combination of commonly used hot-start and touch-down procedures. Briefly, before adding the dNTP mixture and the DNA polymerase the samples were preheated for 5 min at 94°C and rapidly cooled to 4°C. Then dNTPs and DNA polymerase were added at 4°C and the reaction tubes were directly transferred to a preheated temperature cycling reactor (Genius, Techne, Cambridge, UK) at 94°C. PCR reactions were incubated for 35 cycles under cycling conditions of 94°C for 30 sec, 70-56°C for 1 min (starting at 70°C and decreasing by 0.4°C per cycle), and 72°C for 1-5 min, depending on the size of the expected PCR product. As a final step the reaction mixture was incubated for 7 min at 72°C. Reaction products were analyzed by polyacrylamide slab gel electrophoresis and ethidium bromide staining.

Molecular cloning of viral cDNA and preparation of plasmid DNA

PCR products representing EAV-specific cDNA sequences were subjected to restriction endonuclease treatment and restriction fragments were purified using preparative low melting point agarose gel electrophoresis. Specific DNA bands were extracted from the gel by a hot phenol procedure followed by gel filtration. Restricted and purified EAV cDNA was inserted into one of the following mammalian expression vectors: pCR3.1, pcDNA3.1, pcDNA3.1/His, pDisplay (Invitrogen). Vector plasmids were prepared using restriction endonucleases and purified as described above. In addition, restricted vector DNA was dephosphorylated using calf intestine phosphatase (CIP). Ligation of specific EAV cDNA fragments with expression vector DNA was performed as described previously (Rösen-Wolff *et al.,* 1991). The resulting recombinant plasmid constructs are listed in Table 2. The specificity of the reaction products was confirmed by nucleotide sequence analysis of the insert and flanking vector regions.

Nucleotide sequence analysis

PCR products were treated with 1 vol phenol:chloroform (5:1) and precipitated with 3 vol 95% ethanol containing 100 mM sodium acetate. The DNA was then washed with 70% ethanol and dissolved in bidestilled water to a final concentration of 20 ng/μl. Plasmid DNA was prepared using the Qiagen tip100 Kit (Qiagen, Hilden, Germany) according to the instructions of the manufacturer. Purified DNA was adjusted in $H_2O$ to a final concentration of 1 μg/μl. Purified DNA was automatically sequenced with a 373A "Extended" DNA sequencer using the BigDye Terminator-*Taq* cycle sequencing technique (Applied Biosystems GmbH, Weiterstadt, Germany). Each sequencing reaction was performed in a volume of 20 μl containing 100 ng of a PCR product or 0.5 μg of plasmid DNA, 50 pmol of the sequencing primer,

and 5 μl of the BigDye Terminator reaction mixture. The cycle sequencing reaction was incubated for 28 cycles in an automated temperature cycling reactor (GeneE, Techne, Cambridge, UK) under cycling conditions of 96˚C for 30 sec and 60˚C for 4 min per cycle. The samples were prepared for electrophoresis as described by the manufacturer. The electrophoresis of the samples was carried out on a 36-well 48-cm WTR (well to read) polyacrylamide gel. The nucleotide sequences obtained from individual sequencing reactions were assembled using the Sequence Navigator software (version 2.1, Applied Biosystems GmbH, Weiterstadt, Germany). Nucleotide and amino acid sequences were compared to current GenBank, EMBL, and SwissProt database sequence entries using the BLAST service of the National Center for Biotechnology Information (National Library of Medicine, Bethesda, MD, USA). Physico-chemical properties of proteins were determined and conserved sequence motifs were identified with the PHYSCHEM and PROSITE programs included in the PC/Gene software (release 6.85, A. Bairoch, University of Geneva, Switzerland). The ClustalX program (version 1.64b) (Thompson *et al.,* 1997) was used to generate multiple sequence alignments.

Preparation of viral RNA and Northern blot analysis

Total cellular RNA was isolated at different times after infection using the guanidium/cesium chloride method as described previously (Rösen-Wolff *et al.,* 1988, 1989; Rösen-Wolff and Darai, 1991). The northern blot analyses of these RNAs were carried out using formaldehyde agarose gel (1%) electrophoresis as described elsewhere (Rösen-Wolff *et al.,1988,* 1989; Rösen-Wolff and Darai, 1991).

DNA vaccination of animals

The immunogenic potential of the EAV translation units were *investigated in vivo* using BALB/c mice that were administered with DNA of the constructed expression vectors harboring and expressing the cDNA of the individual ORFs of EAV. The BALB/c mice were injected with about 100 μg/DNA diluted in 100 μl PBS. The DNA was injected subcutaniously and into the *tibialis cranialis* muscle *(Musculus gastrocnemius)* and with a 27 gauge needle. The mice were boosted 3 to 5 times with the same quantities of DNA and under the same conditions at about two week intervals. Control mice received the same amount of parental expression vectors via an identical route and frequency.

Neutralization test

Neutralization tests were carried out by diluting EAV-specific mouse or rabbit sera with PBS (1:2 to 1:1024) in a Falcon microtiter plate. Serum dilutions (50 μl) were mixed with 100 $TCID_{50}$ of EAV (50 μl). The serum-virus mixture was incubated in a 5% $CO_2$-air atmosphere at 37˚C for 2 h. Subsequently, $5 \times 10^3$ RK13 cells in suspension were added to each sample of the serum-virus mixture. After 12 h the infected cultures were overlayed with BME containing 10% FCS and 0.5% carboxymethylcellulose. Then the cultures were incubated for 3-4 days at 37˚C in a 5% $CO_2$-air atmosphere. Titers of infectious units were determined after staining with 1 % crystal violet.

Immunoblot analysis

Confluent monolayers of cells were harvested by scraping the cells from the culture well, petri dishes, and/or flasks after being washed three times with PBS (pH 7.2). The final cell pellet was resuspended in distilled water. Protein concentration was measured under the standard method (Bradford, 1976). Samples were dissolved in an equal volume of lysis buffer (0.01 M Tris HCl, 10% glycerol, 2% SDS, 5% β-mercaptoethanol, 0.1% (w/v) bromophenol blue, pH 8), heated for five minutes at 95˚C, and subjected to SDS-PAGE according to the method of Laemmli (1970). Proteins derived from infected and transfectant cells, as well as recombinant N protein were separated by SDS-PAGE and electroblotted onto nitrocellulose filters using semi-dry electroblotting chambers (Renner, Dannstadt, Germany). Transfer efficiency was monitored by Ponceau staining (Sigma, Munich, Germany). Filters were blocked for 1 h and incubated with a 1:1000 and 1:2000 dilution of the rabbit antisera mentioned above. Alkaline phosphatase conjugated antibodies (anti rabbit or mouse Ig-AP, Boehringer Mannheim, Germany) were used to detect interaction of the rabbit or mouse antiserum with EAV protein.

Immunofluorescence assay

Indirect Immunofluorescence assay was performed essentially as described earlier (Welzel *et al.,* 1998). Briefly, RK13 cell lines were seeded on tissue chamber slides (Nunc Inc., Naperville, USA). The monolayer cell culture were infected with EAV at the MOI of 2 PFU/cell and 48 h after infection the cells were fixed with acetone-methanol and the slides were stored at -20˚C. Fluorescein isothiocyanate (FITC)-conjugated anti-rabbit or mice IgG (F(ab')2 fragment immunoglobulin (Boehringer, Mannheim, FRG) was used as second antibody. In addition, rhodamine B-isothiocyanate (Merck, Darmstadt, Germany) was used at a final concentration of 10 ng/µl for counterstaining of the cells, together with the second antibody.

Enzyme linked immunosorbent assay (ELISA)

Polysorp F8 Microtiter Plates (Nunc, Wiesbaden, Deutschland) were coated with 50µl EAV Protein (EAV+Host (RK13)) at a concentration of 2 µg × $ml^{-1}$ in PBS (+ 0.05% $N_3Na$) over night at room temperature, followed by three cycles of washing with $H_2O$ and then postcoated with 300 µl Blocking Buffer (0.017 M $Na_2B_4O_7$ x 10 $H_2O$, 0.12 M Na Cl, 0.05 % Tween 20, 1 mM EDTA, 5 % BSA, 0.05 % $NaN_3$) for 3h at 28°C followed by three cycles of washing with $H_2O$. For the assay, the following reagents were successively used: rabbit anti EAV serum at a reciprocal dilution up to 16000 or mouse anti EAV serum at a reciprocal dilution up to 800. The dilutions were made in Sample buffer POD (DADE Behring, Marburg, Deutschland). After three cycles of washing with 300 µl/well, horse-radish peroxidase labeled rabbit-anti IgG second antibody or the horse-radish peroxidase labeled mouse-anti IgG second antibody (Boehringer, Mannheim, Deutschland) at a predetermined optimum dilution of 1:3000 each was added. The dilutions were made in Blocking buffer. Incubation steps were done for 1h at 28°C, each followed by three cycles of washing with 300 µl/well Washing Buffer (DADE Behring, Marburg, Deutschland). Color was developed by adding 200 µl/well of freshly prepared Buffer/Substrate TMB and Chromogen TMB (10:1) (DADE Behring, Marburg, Deutschland). The assay was stopped after 30 min by the addition of 50 µl/well Stopping Solution POD (DADE Behring, Marburg, Deutschland) and read according to standard procedures at 450 nm on an automatic ELISA reader (MR5000, DYNATECH, Denkendorf Deutschland).

Lymphocyte activation assay

A Lymphocyte activation assay for the eventual use of the cellular response of the immunized mice was established. BALB/c mouse, female, anti-mouse CD3ε (500A2, PharMingen, Cat. No. 01511D), anti-mouse CD69 (H1.2F3, PharMingen, Cat. No. 01505B),
Geys buffer (140 mM $NH_4Cl$, 2.7 mM KCl, 6.5 mM $Na_2HPO_4$, 1.5 mM $KH_2PO_4$, 0.7 mM $CaCl_2$, 0.5 mM $MgCl_2$), FACS buffer (PBS, 2% FCS, 0.01% $NaN_3$), Polystyrene round bottom tubes (Falcon, Becton Dickinson, Cat. No. 2052), and six well plate (TPP, Cat. No. 9206) were used. The mesurement of the samples was performed using FACScan (Becton Dickinson), five measurable parameters: three high performance secondary photomultiplier with bandpass filter: 530 nm for fluorescein isothiocyanate (FITC), 585 nm for phycoerythrin (PE) and 650 nm (red fluorescence), forward and side scatter.

One mouse was killed, spleenectomy was performed and spleen was in a sterile tube with 1x BME medium. Spleen was passed through a sterile homogeniser for intact spleen cell separation. The homogeniser was washed with 1x BME medium. The solution was centrifuged for 3 min with 1500 rpm. The pellet was dissolved in 1.5 ml Geys buffer. After that the solution was centrifuged for 3 min with 1500 rpm. The pellet was dissolved in 9 ml BME medium with 10% FCS. One 6 well plate was filled with 1 ml 1x BME medium with 10% FCS, in 3 wells of the plate additional 1 µg/ml anti-mouse CD3ε was added. 1.5 ml cell solution was added to each well. The 6 well plate was incubated at 37°C, 5% $CO_2$ for three days. Solution with activated and not activated cells were each divided in four Polystyrene round bottom tubes. The tubes were filled with FACS buffer and centrifuged for 3 min with 1500 rpm. The supernatant was discarded and the pellet was resuspended in two of four tubes with 100µl anti-mouse CD3ε (1:100 diluted in FACS buffer) and incubated for 30 minutes at 4°C. For double fluorescence the cells were centrifuged for 3 min with 1500 rpm, the pellet was resuspended with 100µl anti-mouse CD69 (1:100 diluted in FACS buffer) and incubated for 30 minutes at 4°C again. The cells were centrifuged for 3 min, with 1500 rpm, the pellet was resuspended in 2 ml FACS buffer, centrifuged again for 3 min with 1500 rpm, and resuspended in 500 µl FACS buffer each, vortexed and measured in the FACScan.

Computer-assisted sequence analysis

Nucleotide sequences were compiled using the ABI sequence navigator software version 1.2. Nucleotide and amino acid sequences were analyzed using the PC/GENE program release 6.85 (Intelligenetics Inc. Mountain View, California, U.S.A.) and OMIGA program release 11.3 (Oxford Molecular Group Ltd., Oxford, UK).

**RESULTS**

Establishment of a cell culture system for virus propagation, isolation of virus particles, and extraction of viral RNA

The equine arteritis virus (EAV) used in this study was grown and propagated on rabbit kidney cells (RK-13). Virions of EAV were prepared, purified, and the viral RNA was extracted.

Generation of specific rabbit antisera against whole virus

In order to specifically detect viral gene products by serological assays a polyclonal hyperimmune rabbit antiserum was raised against complete EAV virion components in New Zealand white rabbits. The protocol of the immunization experiment is given in Table 1. The titer of the antiserum was found to be >1:2000 as determined by Western Blot analysis.

Generation of rabbit antisera against synthetic polypeptides

The biophysical analysis of the nucleocapsid protein (ORF 7) of EAV indicated that a strong antigenic domain was located within the first 38 amino acids of the ORF 7 gene product. In order to investigate the immunogenic potential of the N-terminus of the viral nucleocapsid protein a synthetic polypeptide corresponding to amino acid residues 1-38 was synthesized and linked to keyhole limpet hemocyanine (KLH) and used to raise polyclonal monospecific antiserum in rabbits. The antiserum was found to detect specific EAV gene product in Western Blot assay at a dilution of 1:200.

Amplification of viral genes by RT-PCR and molecular cloning of viral genes in expression vectors

The EAV genome consists of about 12.287 nucleotides with a short 3'poly- (A) tail. For molecular cloning of the viral genome specific oligonucleotides were synthesized (Table 2) and purified viral RNA was used in 3'-RACE experiments to generate a cDNA bank from the genomic RNA including cDNA of viral mRNA transcripts. The amplified cDNA fragments include the viral open reading frames (ORFs) 2b to 7, which encode the small glycoprotein, nonstructural proteins, large glycoprotein, membrane protein, and nucleocapsid protein, respectively (Table 3). The viral genes were molecularly cloned into a suitable expression vector *(pCR3.1, pDisplay, and pcDNA3.1/His A, B, C; Invitrogen).* The identity of the cDNA of the individual viral ORFs was confirmed by nucleotide sequence analysis. The properties of the constructed mammalian expression vectors harboring and expressing EAV-specific cDNA of the individual viral translation units are summarized in Table 3.

As an additional goal for the development of efficient EAV DNA vaccines in future it is envisaged to construct expression vectors harboring and expressing overlapping or full-length viral cDNA. An expression library of cloned high molecular weight viral cDNA was constructed spanning the entire viral genome. Using optimized long-range RT-PCR protocols a number of overlapping viral cDNA fragments ranging from 2,000 to over 8,500 bp were generated. The identities of the RT-PCR products were confirmed by nucleotide sequence analysis. This library is essential to establish full-length cDNA clones expressing the entity of viral antigens simultaneously in *in vivo* and *in vitro* when necessary. The properties of the individual RT-PCR products are summarized in Table 4.

DNA vaccination of mice with different vector constructs and evaluation of the corresponding immune response

DNA vaccination of mice with vector construct expressing viral ORFs 5 and 7

Balb/c mice were used as a model system for the evaluation of the immune responses against individual gene products of EAV raised by administration of recombinant plasmid DNA. The capability of the expression vectors harboring and expressing EAV ORF5 and ORF7 that encode the viral nucleocapsid protein and the viral major glycoprotein to induce immune response in the mouse system was investigated. The animals were inoculated subcutaneously and intramuscularly for five times with 100 μg of the particular DNA in 14-day intervals. Analysis of the individual mouse sera revealed that the administered DNAs of the vectors expressing both viral gene products are able to induce significant immune response in mice as tested by neutralization test (NT). The results of these studies are summarized in Table 5 and are shown in Fig. 3 for ORF 5. A significant immune response was detected when the animals were immunized with the recombinant plasmids pCR3.1-EAV-07-BX-C3 and pCR3.1-EAV-OS-C14 harboring ORFs 7 and 5 coding for viral nucleocapsid protein and large envelope glycoprotein ($G_L$), respectively. As shown in Table 5 it was found that the recombinant plasmids pCR3.1-EAV-O7-BX-C3 are able to raise antibodies against EAV in mice (80 % immune response) as detected by Neutralization Test. Similar results (50-70 % immune response) were obtained in two independent experiments when the animals were inoculated with the recombinant plasmids pCR3.1-EAV-O5-BX-C14 (Table 5 and Fig. 3A and B).

In the next step of this investigation the animals were administered with the DNAs of the recombinant plasmids pCR3.1-EAV-O7-BX-C3 and pCR3.1-EAV-O5-C14 simultaneously under the same conditions described above. The results of this study that is in agreement with the corresponding results obtained from the analysis of the gene products of the EAV ORFs 7 and 5 (Table 5 and Fig. 3) are summarized in Table 5 and shown in Fig. 4. These data indicate that the native DNA of recombinant plasmids pCR3.1-EAV-07-BX-C3 (harboring ORF 7) and pCR3.1-EAV-O5-C14 (harboring ORF 5) is able to express the corresponding gene products (viral nucleocapsid protein and large envelope glycoprotein

($G_L$)) *in vivo.*

The results of these studies unambiguously underline that the gene products of the EAV ORFs 7 and 5 are suitable candidates for development of a DNA vaccine protecting an EAV infection.

DNA vaccination of mice with vector construct expressing viral ORFs 5, 7, and IL-2

In order to determine whether or not the IL-2 is able to enhance the immunogenic potential of the gene product of the EAV ORFs 5 and 7 by DNA vaccination the following experiments were performed. An expression vector (pWS2ms) containing the murine IL-2 - expression cassette (kindly provided by Dr. W. Schmidt, Intercell, Vienna) was co-inoculated with two novel constructed recombinant plasmids pDP-EAV-O7-BgS-C2 and pDP-EAV-05-C1 harboring and expressing the EAV ORFs 7 and 5 that encode the viral nucleocapsid protein and large envelope glycoprotein ($G_L$), respectively. The construction of these expression vectors based on pDisplay system that is similar to pCR3.1. This system additionally allows the cell surface expression of inserted epitopes by fusion to an N-terminal signal peptide and a C-terminal transmembrane domain. Balb/c mice were inoculated subcutaneously and intramuscularly for four times with 100 $\mu$g of each of the vector constructs in 14-day intervals. Under the conditions used an enhanced EAV-specific immune response in mice was detectable as shown in Table 5.

DNA vaccination of mice with vector construct expressing viral ORFs 5 and 6

The EAV consists of an unglycosylated membrane protein (M, 17 kDa, gene product of ORF 6). It was of particular interest to proof whether or not this viral gene product can eventually influence and/or enhance the immunogenic potential of the gene products of EAV ORF 5 *in vivo.* A recombinant plasmid pCR3.1-EAV-06-BE-C3 was constructed in which the translation unit of the EAV ORF 6 was inserted into the corresponding site of the expression vectors pCR3.1. Balb/c mice were inoculated with recombinant plasmids pCR3.1-EAV-06-BE-C3 and pCR3.1-EAV-O5-C14 under the conditions described above. The sera obtained from the Balb/c mice (pre and post DNA vaccination) were analyzed using neutralization test and the results are given in Table 5 and shown in Fig. 6. A significant enhanced potential of the gene product of the EAV-ORF 6 on the functional activities of the viral large envelope glycoprotein (gene product of EAV ORF 5) in vivo was not observed.

DNA vaccination of mice with vector construct expressing viral ORFs 3 and 4

Although the functional activity of the gene products of two EAV ORFs 3 and 4 is not known, the eventual activity of these viral proteins in vivo was investigated. Two recombinant plasmids pCR3.1-EAV-03-BX-C1 and pCR3.1-EAV-O4-BX-C3 was constructed (Table 3) in which the cDNA of the EAV ORFs 3 and 4 were inserted into the corresponding site of the expression vector pCR3.1. The sera obtained from the Balb/c mice (pre- and post DNA-vaccination) that were inoculated with these recombinant plasmids were analyzed using neutralization test. The results of these studies are summarized in Table 5 and shown in Fig. 7 and 8 for the gene products of EAV ORFs 3 and 4, respectively. No neutralizing antibodies were detected in the immunized Balb/c mice under the conditions used.

DNA vaccination of mice with vector construct expressing EAV N-terminal hydrophilic ectodomain of large envelope glycoprotein ($G_L$)

Balasuriya and coworkers (1995) found that the neutralization determinants of EAV are located on the gene product of ORF 5 (large envelope glycoprotein) within the N-terminal ectodomain (amino acid positions 1-121; Balasuriya *et al.,* 1997). Accordingly, a novel expression vector based on pcDNA3.1/His system was constructed. This vector is similar to pCR3.1, but allows the specific detection and purification of the expressed fusion proteins using N-terminal amino acid sequence tags. The strategy of this experiment is shown in Fig. 2. A recombinant plasmid was constructed and termed pC31-HIS-EAV-O5-del-121. The insert of this expression vector possesses the coding region of EAV ORF 5 corresponding to amino acids 1-121 of the viral large envelope glycoprotein which was inserted into the corresponding sites of the parental vector pcDNA3.1-HIS-C (Table 5 and Fig. 2). Balb/c mice were inoculated with recombinant plasmid pC31-HIS-EAV-05-del-121 under the conditions described above. The sera obtained from the Balb/c mice (pre and post DNA vaccination) were analyzed using neutralization test. As shown in Table 5 and Fig. 9 it was found that the majority of the DNA vaccinated animals (90 %) developed a significant neutralizing antibody against EAV.

DNA vaccination of mice with vector constructs expressing viral membrane and glycoproteins in combination with IL-2 gene expression

As described above, it had been shown that the viral N-glycosylated major membrane protein ($G_L$), the N-glycosylated minor membrane protein ($G_S$, 25 kDa), and unglycosylated membrane protein (M, 17 kDa) are able to develop significant immune response *in vivo* as detected by DNA vaccination of Balb/c mice. Furthermore, the enhancer function of IL-2 gene expression during DNA vaccination is known. However, an intermolecular interaction of these proteins during gene expression in *vivo* e.g. by DNA vaccination is not known, so far. Therefore it was of particular importance to proof whether or not these proteins are capable to induce an adequate immune response by simultaneous expression *in vivo.* Pour recombinant plasmids pCR3.1-EAV-06-BE-C4, pCR3.1-EAV-O2-BX-C5, pC3.1-EAV-O5-BX-C14, and pWS2ms were used. These vector constructs are able to express viral membrane protein, glycoproteins $G_s$, $G_L$, and mice interleukin 2, respectively. Balb/c mice were inoculated with a DNA mixture containing the four recombinant plasmids under the standard conditions described above. The sera obtained from the Balb/c mice (pre- and post DNA vaccination) were analyzed using neutralization test and the corresponding data are summarized in Table 5 and shown in Fig. 10. Under the conditions used in this experiment 90% of the DNA vaccinated mice developed significant immune response against EAV.

DNA vaccination of mice with vector constructs expressing viral small glycoprotein and the gene product of the ORF 4

The capability of the EAV small glycoprotein (Gene products of ORF2b and the gene product of ORF 4 to develop immune *response in vivo* was investigated by DNA vaccination of Balb/c mice. Two recombinant plasmids pCR3.1-EAV-O2-BX-C5 and pCR3.1-EAV-O4-BX-C3 were used. These vector constructs are able to express viral small glycoproteins $G_S$, and the gene product of EAV ORF 4, respectively. Balb/c mice were inoculated with a DNA mixture containing the two recombinant plasmids under the standard conditions described above. The sera obtained from the Balb/c mice (pre and post DNA vaccination) were analyzed using neutralization test. As shown in Fig. 11 and Table 5, it was found that about 50% of the DNA vaccinated animals developed NT-titer against EAV.

Logistics for DNA vaccination of horses

In addition-to the objective of our studies, it was necessary to develop and establish a novel screening system for detection of humoral and cellular immune response in vaccinated horses. An enzyme linked immunosorbent assay (ELISA) and a lymphocyte activation assay were established as described in the session of material and methods. Furthermore, it was necessary to prepare a convenient kit for vaccination of horses with the constructed expression vectors harboring and expressing the viral gene products of ORFs 2b to 7.

Development of a novel Enzyme linked immunosorbent assay (ELISA)

Polysorp F8 Microtiter Plates were coated with Protein of EAV infected RK-13 cells at a concentration of 2 $\mu g$ x $ml^{-1}$. In this system antibodies against EAV (rabbit, mouse, and horse serum, etc.) can be detected. Horse-radish peroxidase labeled rabbit-anti IgG or horse-radish peroxidase labeled mouse-anti IgG served as second antibody. The color was developed by adding Buffer/Substrate TMB and Chromogen TMB and the adsorbance was determined according to standard procedures at 450 nm on an automatic ELISA reader (for detail see session material and methods). An example of the results of this analysis is shown in Fig. 12. The viral antigens prepared for ELISA can be used for performance of about 20,000 ELISA assays.

Development of a Lymphocyte activation assay

A Lymphocyte activation assay for the eventual use of the cellular response of the immunized mice was established. BALB/c mouse, female, anti-mouse CD3ε (500A2, PharMingen, Cat. No. 01511D), anti-mouse CD69 (H1.2F3, PharMingen, Cat. No. 01505B), Geys buffer (140 mM $NH_4Cl$, 2.7 mM KCl, 6.5 mM $Na_2HPO_4$, 1.5 mM $KH_2PO_4$, 0.7 mM $CaCl_2$, 0.5 mM $MgCl_2$), FACS buffer (PBS, 2% FCS, 0.01% $NaN_3$), Polystyrene round bottom tubes (Falcon, Becton Dickinson, Cat. No. 2052), and six well plate (TPP, Cat. No. 9206) were used. The measurement of the samples was performed using FACScan (Becton Dickinson), five measurable parameters: three high performance secondary photo multiplier with band pass filter: 530 nm for fluorescein isothiocyanate (FITC), 585 nm for phycoerythrin (PE) and 650 nm (red fluorescence), forward and side scatter.

One mouse was killed, spleenectomy was performed and spleen was transferred to a sterile tube with 1x BME medium. Intact spleen cells were separated and were washed with 1x BME medium. The solution was centrifuged for 3 min with 1500 rpm. The pellet was dissolved in 1.5 ml Geys buffer. After that the solution was centrifuged for 3 min with 1500 rpm. The pellet was dissolved in 9 ml BME medium with 10% FCS. One 6 well plate was filled with 1 ml 1x BME medium with 10% FCS, in 3 wells of the plate additional 1 $\mu g/ml$ anti-mouse CD3ε was added. 1.5 ml cell solution was added to each well. The 6 well plate was incubated at 37°C, 5% $CO_2$ for three days. Solutions with activated and not activated cell were each divided in four Polystyrene round bottom tubes. The tubes were filled with FACS buffer and centrifuged for 3 min with 1500 rpm. The supernatant was discarded and the pellet was resuspended in two of four tubes with 100μl anti-mouse CD3ε (1:100 diluted in FACS buffer) and incubated for 30 minutes at 4°C. For double fluorescence the cells were centrifuged for 3 min with 1500 rpm, the pellet was resuspended with 100μl anti-mouse CD69 (1:100 diluted in FACS buffer) and incubated for 30 minutes at 4°C again. The cells were centrifuged for 3 min with 1500 rpm, the pellet was resuspended in 2 ml FACS buffer, centrifuged again for 3 min with 1500 rpm, and resuspended in 500 μl FACS buffer each, vortexed and measured in the FACScan.

Table 1: An example of experimental protocol used for production of polyclonal antibodies against equine arteritis virus in New Zealand white rabbit

| Date | Treatment |
|---|---|
| **09.06.1998** | Preimmun-serum<br>10 x 620 μl |
| **10.06.1998** | 1st inoculation*<br>0.5 ml/s.c. |
| **24.06.1998** | 2nd inoculation*<br>0.5 ml/s.c. |
| **08.07.1998** | 3 rd inoculation*<br>0.5 ml/s.c. |
| **22.07.1998** | 4th inoculation*<br>0.5 ml/s.c.<br>**First bleeding (10 ml)**<br>**Titer by immunoblot analysis = 1:200** |
| **30.07.1998** | 5th inoculation*<br>0.5 ml/s.c. |
| **11.08.1998** | **Final bleeding (30 x 1.5 ml)**<br>**Titer by immunoblot analysis = >1 :2000** |
| Virus: Equine arteritis virus: Virion in 0.5 ml PBS, purified by sucrose gradient centrifugation<br>Animal: New Zealand white rabbit (ca 2 kg/female) | |

Table 2: List of oligonucleotide primers that were used for the amplification and molecular cloning of equine arteritis virus cDNA. Artificial sequences containing restriction endonuclease recognition sites (underlined) are shown in boldface letters.

| Primer Name | Nucleotide Sequence |
|---|---|
| P-XbaI-EAV-O5-R1 | 5'-GGGTCTAGAGTCACCACAAAATGAATCTATGGC-3' |
| P-BamHI-EAV-O5-F1 | 5'-GGGGGATCCTGGTACGTTGGGCTCAACGATG-3' |
| P-XbaI-EAV-O7-R1 | 5'-GGGTCTAGACCACACAGGAGAATATCCACGTC-3' |
| P-BamHI-EAV-O7-F1 | 5'-GGGGGATCCCGCAGTTGGTAACAAGCTTGTCG-3' |
| P-XbaI-EAV-O5-R1 | 5'-GGGTCTAGAGTCACCACAAAATGAATCTATGGC-3' |
| P-BamIII-EAV-O5-F1 | 5'-GGGGGATCCTGGTACGTTGGGCTCAACGATG-3' |
| P-BamHI-EAV-O2-F1 | 5'-GGGGGATCCATTTCTGTGATTGATGCAGCGC -3' |
| P-XbaI-EAV-O2-R1 | 5'-GGGTCTAGAGGCATATTCATAACCCGTGTGCACTAC-3' |
| P-BamHI-EAV-O3-F1 | 5'-GGGGGATCCTTTGACCGGACCGGCCACATGGGTC-3' |
| P-XbaI-EAV-O3-R1 | 5'-GGGTCTAGAGTAAGTAAAATTACGAGCCTCTGCAGC-3' |
| P-BamHI-EAV-O4-F1 | 5'-GGGGGATCCCCTTTGTAGATGAAGATCTACGGC-3' |
| P-XbaI-EAV-O4-R1 | 5'-GGGTCTAGAGCAATACAATCATAGATAACATCGTTGAGCCC-3' |

(continued)

| Primer Name | Nucleotide Sequence |
|---|---|
| P-BamHI-EAV-O6-F1 | 5'-GGGGGATCCCAGCTGAGGTATGGGAGCCATAG-3' |
| P-EcoRI-EAV-O6-R1 | 5'-CGGGAATTCCATGCGCAGTAGGTCATTGTAGC-3' |
| P-BamHI-EAV-O5-F1n | 5'-GGGGGGATCCTGGTACGTTGGGCTCAACGATG-3' |
| P-XbaI-EAV-O5-R1n | 5'-GGGGGTCTAGAGTCACCACAAAATGAATCTATGGC-3' |
| P-BamHI-EAV-O7-F1n | 5'-GGGGGGATCCCGCAGTTGGTAACAAGCTTGTCG-3' |
| P-XbaI-EAV-O7-R1n | 5'-GGGGGTCTAGACCACACAGGAGAATATCCACGTC-3' |
| P-SalI-EAV-O5-R1 | 5'-GGGGTCGACGTCACCACAAAATGAATCTATGGC-3' |
| P-EcoRI-EAV-O7-R1 | 5'-GGGGAATTCCACACAGGAGAATATCCACGTC-3' |
| P-EAV-O5-R1 | 5'-CAACTATGCCGAATTCACGGCC-3' |
| P-EAV-O5-F1 | 5'-GGCCGTGAATTCGGCATAGTTG-3' |
| P-BamHI-EAV-O2-F1n | 5'-GGGGGGATCCATTTCTGTGATTGATGCAGCGC -3' |
| P-XbaI-EAV-O2-R1n | 5'-GGGGGTCTAGAGGCATATTCATAACCCGTGTGCACTAC-3' |
| P-BamHI-EAV-O3-F1n | 5'-GGGGGGATCCTTTGACCGGACCGGCCACATGGGTC-3' |
| P-XbaI-EAV-O3-R1n | 5'-GGGGGTCTAGAGTAAGTAAAATTACGAGCCTCTGCAGC-3' |
| P-BamHI-EAV-O4-F1n | 5'-GGGGGGATCCCCTTTGTAGATGAAGATCTACGGC-3' |
| P-XbaI-EAVO4-R1n | 5'-GGGGGTCTAGAGCAATACAATCATAGATAACATCGTTGAGCCC-3' |
| P-BamHI-EAV-O6-Fln | 5'-GGGGGGATCCCAGCTGAGGTATGGGAGCCATAG-3' |
| P-EcoRI-EAV-O6-Rln | 5'-GGGGGGAATTCCATGCGCAGTAGGTCATTGTAGC-3' |
| P-SalI-EAV-O5-R1n | 5'-GGGGGGGTCGACTGGCTCCCATACCTCAGCTGC-3' |
| P-BglII-EAV-O7-Fln | 5'-GGGGGGAGATCTATGGCGTCAAGACGATCACGTCCG-3' |
| P-SalI-EAV-O7-R1n | 5'-GGGGGGGTCGACCGGCCCTGCTGGAGGCGCAAC-3' |
| P-BglII-EAV-O2-Fln | 5'-GGGGGGAGATCTATGCAGCGCTTTTCTTTCTCATGC-3' |
| P-SalI-EAV-O2-Rln | 5'-GGGGGGGTCGACCAAAATCTTGCGCCGCGGCAAAG-3' |
| P-BglII-EAV-O6-Fln | 5'-GGGGGGAGATCTATGGGAOCCATAGATTCATTTTGTGG-3' |
| P-SalI-EAV-O6-R1n | 5'-GGGGGGGTCGACTTGTAGCTTGTAGGCTGTCGCCG-3' |
| P-EcoRI-EAV-O1-Fln | 5'-GGGGGGGAATTCATGGCAACCGCTACTGG-3' |
| P-KbaI-EAV-Ol-R1n | 5'-GGGGGGTCTAGATCACACGGGCCCAATGACTGAACC-3' |
| P-AflII-TOEAV-F1 | 5'-CCCCTTAAGTGCCATATACGGCTCACCACCATATACAC-3' |
| P-MroI-TOEAV-R1 | 5'-CCCTTCCGGAGGTTCCTGGGTGGCTAATAACTACTTC-3' |
| PR-BAM-5102 | 5'-GGGGGGATCCCAGTACTTTGGC-3' |
| PF-BAM-5097 | 5'-GGGGGGATCCCAGCGACACCCGAGGCACG-3' |
| PR-SPH-8570 | 5'-GGGGGGCATGCTGTAGGTGGGCCATGGATCAAGTCC-3' |
| PF-SPH-8565 | 5'-GGGGGGCATGCCACCTGGGCCAAGAAATTGACC-3' |
| PR-EAV-6959-6946 | 5'-GCTGGCCGCGTAATGCTGGTTGGG-3' |
| PF-EAV-5948-5973 | 5'-CCGTGCTGGATGTGAGGCCGTTACCG-3' |
| P-EAV-GL-ECTO-F | 5'-GGGGGGATCCATGTTATCTATGATTGTATTGCTATTC-3' |

(continued)

| Primer Name | Nucleotide Sequence |
|---|---|
| P-EAV-GL-ECTO-R | 5'-GGGGGGTCTAGACAACACAACTATGCCGAATTCAC-3' |
| P-pcDNA3.1/HisC-F | 5'-ATGGTATGGCTAGCATGACTGGT-3' |
| P-pcDNA3.1/HisC-R | 5'-CAAACAACAGATGGCTGGCAACT-3' |

Table 3: Properties of the constructed mammalian expression vectors harboring EAV-specific cDNA of the individual translations units.

| Construct Name | EAV-Specific Insert (Nucleotide Position) | Expression Vector (Restriction Sites) | Oligonucleotide Primers |
|---|---|---|---|
| **pCR3.1-EAV-O2-BX** | **ORF 2b: small glycoprotein (9807-10490)** | pCR3.1 (*Bam*HI/*Xba*I) G/GATCC/T/CTAGA | P-BamHI-EAV-02-Fln P-XbaI-EAV-02-Rln |
| **pCR3.1-EAV-O3-BX** | **ORF 3** (10289-10780) | pCR3.1 *(Bam*HI/*XbaI)* G/GATCC / T/CTAGA | P-BamHI-EAV-03-Fln P-XbaI-EAV-03-Rln |
| **pCR3.1-EAV-O4-BX** | **ORF4** (10683-11141) | pCR3.1 (*Bam*HI/*Xba*I) G/GATCC / T/CTAGA | P-BamHI-EAV-04-Fln P-XbaI-EAV-04-Rln |
| **pCR3.1-EAV-O5-BX** | **ORF 5: large glycoprotein** (11129-11896) | pCR3.1 *(Bam*HI*IXba*I) G/GATCC / T/CTAGA | P-BamHI-EAV-05-Fln P-XbaI-EAV-05-R1n |
| **pDP-EAV-O5-BgS** | **ORF 5: large glycoprotein** (11129-11896) | PDisplay (*Bgl*II*ISal*I) A/GATCT / G/TCGAC | P-BglII-EAV-05-F1n P-SalI-EAV-05-R1n |
| **pCR3.1-EAV-O6-BE** | **ORF 6: membrane protein** (11884-12372) | pCR3.1 (*Bam*HI/*Eco*RI) G/GATCC / G/AATTC | P-BamHI-EAV-06-F1n P-EcoRI-EAV-06-Rln |
| **pCR3.1-EAV-07-BX** | **ORF 7: nucleocapsid protein** (12296-12628) | pCR3.1 (*Bam*HI/*Xba*I) G/GATCC/T/CTAGA | P-BamHI-EAV-07-Fln P-XbaI-EAV-07-R1n |
| **pDP-EAV-07-BgS** | **ORF 7: nucleocapsid protein** (12296-12628) | PDisplay (*Bgl*II/*SalI*) A/GATCT / G/TCGAC | P-BglII-EAV-07-Fln P-SalI-EAV-07-R1n |
| **pCR3.1-HIS-EAV-O5-del-1-121-BX** | **partial ORF 5: C-terminal ectodomain of large glycoprotein** (11129-11492) | pcDNA3.1/HIS C *(Bam*HI/*XbaI)* G/GATCC / T/CTAGA | P-EAV-GL-ECTO-F P-EAV-GL-ECTO-R |

Table 4: Properties of the equine arteritis virus specific cDNA obtained by long range RT-PCR

| PCR product (viral ORFs) | Nucleotide Position | Size of Viral cDNA Fragment bp | Site (s) of Restriction Enzyme (s) | Oligonucleotide Primers |
|---|---|---|---|---|
| PCR-EAV-1-5102 | 1-5102 | 5102 | ***Afl*II/*Bam*HI** | P-AflII-TOEAV-F1 & |

(continued)

| PCR product (viral ORFs) | Nucleotide Position | Size of Viral cDNA Fragment bp | Site (s) of Restriction Enzyme (s) | Oligonucleotide Primers |
|---|---|---|---|---|
| (truncated ORF 1 a*) | | | **C/TTAAG / G/ GATCC** | PR-BAM-5102 |
| PCR-EAV-5097-8570 **(Part of ORF1 a & b*)** | 5097-8570 | 3473 | ***Bam*HI/*Sph*I** **G/GATCC / GCATG/C** | PF-BAM-5097 & PR-SPH-8570 |
| PCR-EAV-8565-12687 **(Part of ORF1 b & 2-7**)** | 8565-12687 | 4122 | ***Sph*I/*Mro*I** **GCATG/C / T/ CCGGA** | PF-SPH-8565 & P-Mro I-TOEAV-R1 |
| PCR-EAV-1-8570 **(ORF 1 a & truncated 1b*)** | 1-8570 | 8570 | ***Afl*II/*Sph*I** **C/TTAAG / GCATG/C** | P-AflII-TOEAV-F1 & PR-SPH-8570 |
| PCR-EAV-5097-12687 **(Part of ORF1a* -7**)** | 5097-12687 | 7590 | ***Bam*HI/*Mro*I** **G/GATCC / T/ CCGGA** | PF-BAM-5097 & P-Mro I-TOEAV-R1 |
| PCR-EAV-1-6959 **(ORF1 a & part of 1b*)** | 1-6959 | 6959 | ***Afl*II** **C/TTAAG** | P-AflII-TOEAV-F1 & PR-EAV-6959-6946 |
| PCR-EAV-5948-12687 **(Part of ORFIb* -7**)** | 5948-12687 | 6739 | ***Mro*I** **T/CCGGA** | PF-EAV-5948-5973 & P-Mro I-TOEAV-R1 |
| * ORF1: ORF 1a: 208-5391, ORF 1b: 5388-9734 nucleotides<br>** ORF2-7: 9807-12628 nucleotides | | | | |

Table: 5: Summary of the results obtained by DNA immunization of mice using different gene products of equine arteritis virus

| Experime nt (No. of animals) | Expression Vector (ORF/Gene product) | Average of NT-AB[a] titer minimum/ maximum | | % of *Immun*-response [b] |
|---|---|---|---|---|
| | | PIS | PVS | |
| **MV-00-01(10)** 07/27/1998 to 10/19/1998 | **pCR3.1** | <1:10 | <1:10 | 0 |
| MV-07-01(10) **07/27/1998 to 10/19/1998** | **pCR3.1-EAV-07-BX-C3 (ORF 7/nucleocapsid protein =NP)** | **<1:10/1:10** | **1:20/1:640** | **80** |
| MV-05- 01(10) **08/29/1998 11/11/1998** | **pCR3.1-EAV-O5-BX-C14** (**ORF 5/large envelope glycoprotein. to = $G_L$)** | **<1:10** | **1:10/1:160** | **70** |

(continued)

| Experime nt (No. of animals) | Expression Vector (ORF/Gene product) | Average of NT-AB[a] titer minimum/ maximum | | % of *Immun*-response [b] |
|---|---|---|---|---|
| | | PIS | PVS | |
| MV-O5- 02(10) **12/07/1998 to 03/20/1999** | **pCR3.1b-EAV-O5-BX-C14 (ORF 5/$G_L$)** | **<1:10** | **1:10/1:160** | **50** |
| MV-57-01(10) **09/11/1998 to 11/23/1999** | **pCR3.1-EAV-O5-BX-C14** (**ORF** 7/NP) **pCR3.1-EAV-07-BX-C3 (ORF 5/GL)** | **<1:10** | **1:10/1:80** | **30/70[c]** |
| MV-57IL2-1 (10) **10/26/1998 to 01/25/1999** | **pDP-EAV-05-BgS-C1** (ORF 7/NP) **pDP-EAV-07-BgS-C2** (**ORF 5/$G_L$**) **pWS2ms** (IL2) | **<1:10/1:10** | **1:10/1:80** | **40/80[c]** |
| MV-56-01(10) **12/07/1998 to 02/08/1999** | **pCR3.1-EAV-05-BX-C14** (ORF 7/NP) **PCR3.1-EAV-06-BE-C4 (ORF 6/membrane protein)** | **<1:10** | **1:10/1:80** | **20/70[c]** |
| MV-03-01(10) **12/21/1998 to 03/22/1999** | pCR3.1-EAV-03-BX-C1 (ORF 3/?) | <1:10 | <1:10/1:10 | 0 |
| **MV-04-01(10) 12/22/1998 to 03/22/1999** | pCR3.1-EAV-04-BX-C3 (ORF4/?) | <1:10 | <1:10/1:20 | 0 |
| MV-05d-01(10) **12/29/1998 to 03/22/1999** | **pC3.1-HIS-EAV-05-del-121 (amino terminus of large envelope glycoprotein; the first 121 amino acids)** | **<1:10** | **1:20/1:160** | **90/100[c]** |
| MV 256IL2-1 02/12/1999 to 04/07/1999 | **pCR3.1-EAV-O2-BX-C5 (ORF 2b/ small glycoprotein) pCR3.1-EAV-O5-BX-C14 (ORF 5/$G_L$) pCR3.1-EAV-O6-BE-C4 (ORF 6/membrane protein) pWS2ms (IL2 gene)** | **<1:10/1:10** | **1:10/1:80** | **70/90[c]** |

(continued)

| Experime nt (No. of animals) | Expression Vector (ORF/Gene product) | Average of NT-AB[a] titer minimum/ maximum | | % of *Immun*-response [b] |
|---|---|---|---|---|
| | | PIS | PVS | |
| Mv-99/5(10) o5/14/1999 to 07/09/1999 | **pCR3.1-EAV-O2-BX-C5 (ORF 2b/ small glycoprotein) pCR3.1-EAV-O4-BX-C3 (ORF4/?)** | **<1:10/1:10** | **<1:10/1:80** | **40/50[c]** |

**[a] Neutralizing antibodies**
**[b] Neutralizing titer 1:20 excluded**
**[c]Neutralizing titer 1:20 included**
**PIS: Preimmune serum; PVS: Post vaccination serum**
**[?] Protein of unknown function**

**Example 2 DNA vaccination of horses**

Preparation of a DNA vaccine kit for application in horses

A convenient kit for vaccination of horses harboring the individual constructed recombinant plasmid (10 mg DNA) expressing the viral gene products of ORFs 2b to 7 was prepared. The properties of the DNA vaccination kit are summarized in Table 6.

In order to proof the ability of the expression of EAV cDNAs of ORFs 2b and 5 to 7 in the autologous animal system horse it was necessary to screen the horse sera prior to immunization trial. The selection of the suitable animals was based on the results obtained from the analysis of the horse sera. These studies allowed detecting the existence of a previous naturally occurred EAV infection in horses. Five sera were obtained from Prof. Dr. H. Müller (Institut für Virologie, Veterinärmedizinische Fakultät, Universität Leipzig). The horse sera were analyzed by neutralization assay and ELISA as described above. The results of these experiments for sera obtained from Leipzig are summarized in table 7. In contrast, it was found that three horses from the animal farms of Leipzig did not developed specific antibodies against an EAV infection. Consequently, it seem to be rational that the animals from the later farm can be considered for evaluation of EAV DNA vaccine in its natural host.

Preparation of autologous skin fibroblasts

Two skin biopsies were performed from each of the five horses involved in the study (Daggy, Frieda, Friedrich, Jessy, Nelke). Skin samples were cut into (5-8) slices and attached to cell culture flasks. After a 30 min incubation at 37°C and 5% $CO_2$, skin samples were cultured in Dulbecco's modified eagles medium (DMEM) containing 10% fetal calf serum (FCS), antibiotics and antimycotics. Fibroblasts growing out of the skin samples were passaged twice and extended to $2x10^7$ cells. From each horse cells were frozen in liquid nitrogen (N2) in aliquots containing at least $2x10^6$ cells.

Several aliquots were thawed and cultured in DMEM containing 10% FCS and antibiotics to verify the competence of these cells for further growth. Cells were passaged more than 20 times. For transfection experiments, however, cell of passage 15 and more proved to be not suitable.

Purification of plasmid DNA

Plasmids were obtained from Boehringer Ingelheim (named pCR3.1-EAV-02-BX-C3), pCR3.1-EAV-05-BX-C14, and pCR3.1-EAV-07-BX-C3). After selection of ampicillin resistent e.coli K12 colonies, bacterial cultures containing the different plasmids were grown to large scale. From each plasmid 500μl DNA at a concentration of 1.5μg/μl were isolated and stored at -20°C for the transfection experiments.

Transfection experiments

Transfection experiments were initially performed in 12 and 24 well plates. Titration experiments in the 24 well plate format using cell numbers between $15x10^4$ and $3x10^5$ per well revealed that $1.25x10^5$ cells seeded in each well were almost confluent (85%) within 24 hours.

a) Lipofection as transfection reagents

Titration experiments performed in a 24 well plate format using 5-20μl/well Lipofectin showed that amounts >15μl were toxic to the cells. Titration experiments using different amounts (5-80μg/well) of DNA with 12.5 μl Lipofectin revealed that concentrations of 80μg DNA showed the highest transfection efficiencies. However, the efficiency in these transfection experiments did not exceed 10%.

b) LipofectAMINE as transfection reagent

In order to increase the transfection efficiency, transfection experiments were performed using additional transfection reagents (LipofectAMINE, LipofectAMINE *plus,* DMRIE). Compared to the results of the transfection experiments using Lipofectin. DMRIE did not result in higher number of transfected cells. LipotectAMINE showed to be more toxic to the cells, but the transfection efficiencies were higher. The recommdation of the supplier to use in addition to LipofectAMINE the *'plus'* reagent did not increase the transfection rate. Therefore, LipofectAMINE seems to be the reagent of choice to transfect the cultured primary horse skin fibroblasts.

In the initial experiments using 7.5μl LipofectAMINE and 5μg of DNA, about 15% of the target cells were transfected. Currently transfection experiments are being performed to further increase the transfection efficiency. So far, the transfection efficiency is about 20%.

Isolation of peripheral blood lymphocytes

Three weeks and one day before the initial immunisation, 50ml of heparinised blood was collected from each horse by jugular venipuncture. The blood was centrifuged at 400 x g for 5 min and the plasma was removed. Blood calls (10ml) were resuspended with phosphate buffered saline (PBS) to a volume of 25ml, layed on a Ficoll Hypaque gradient (15ml) and centrifuged at 400 x-g for 30min. The PBMC were collected from the interface, washed twice in PBS, counted and frozen in aliquots in $N_2$ in 10% DMSO and 90% FCS. To test the viability of the PBMC, cells were thawed, cultured in Isocove's modified Eagles medium (IMEM) containing 10% FCS and antibiotics. PBMC were stimulated for two days with 2.5μg/ml pookweed mitogen and cultured thereafter in the presence of 200U of human recombinant IL-2. PBMC showed a good proliferation, were grown to $2x10^6$ cells/ml and were frozen again as aliquots in $N_2$.

Detection of EAV-specific antibodies

Serum was collected 4 months, three weeks and one day before the initial immunisation. Serum of the first timepoint was investigated at the Institute of Medical Virology (Prof. Darai) in Heidelberg for the presence of EAV-specific antibodies. The results are summarised in table 10.

Immunisation of the horses

All five horses were immunised by intramuscular (i.m.) injection and intradermal application of the EAV plasmid DNA encoding (parts of) ORF 2b,ORF 5, and ORF 7 using a gene gun. The i.m. inoculations were applied to the musculi semimembranosus/semitendinosus/gluteus. The gene gun application of the DNA was performed on both sides of the neck. The corresponding parts of the skin ($40cm^2$) were shaved before to enable a good DNA application using the gene gun. A detailed protocol of the DNA immunisation (including sedation of the horses, amount of DNA, adjuvants, buffers) is provided in example 4. The horses were investigated 24 and 48 h post immunisation for local and systemic reactions. None of the animals developed fever and the local reactions (thickness of the skin, development and involution of papules) are summarised in tables 12 and 13.

Collection of blood and serum samples

Peripheral blood lymphocytes, serum and plasma of the horses were taken as outlined in Table 12. Up to date, blood and serum samples were taken three times after the $3^{rd}$ booster immunization to measure the kinetics of antibody titres and activities of cytotoxic T-lymphocytes.

Determination of the maximum cellular $^{51}Cr$-uptake

Titration experiments were perfomed using (i) constant amounts of $^{51}Cr$ with different cell-concentrations and incubation times and (ii) constant incubation times with different cell-concentrations and amounts of $^{51}Cr$.

Target cells (5 x $10^3$, 2 x $10^4$, and 5 x $10^4$) were labeled with 100 μCi for each 90 min., 150 min., and 240 min. The cellular $^{51}Cr$-uptake as well as $^{51}Cr$ present in the culture supernatant were determined. In the supernatants of the labeled cultures, a linear increase of $^{51}Cr$ was measured (Figure 14a). The amount of cellular $^{51}Cr$ also increased with longer incubation times and higher numbers of cells.

Similar results were obtained when these experiments were performed using constant incubation times with different cell concentrations and amounts of $^{51}Cr$ (Figure 14a). The substraction of the $^{51}Cr$ present in the cells and the $^{51}Cr$ released into the supernatant is shown in (Figure 14b). For the measurement of cytotoxic T-lymphocyte activities in the blood of the immunized horses, it was decided to label 5 × $10^4$ target cells with 200 μCi for 240 min.

Measurement of cytotoxic T-lymphocyte activities after the 2nd and 3rd booster immunization

The preparation of the target cells including transfection and $^{51}Cr$-labeling was performed as described in detail in Example 3. The isolation of the peripheral blood lymphocytes, the cell culture *in vitro* as well as the restimulation of the effector cells was also performed as outlined in Example 3.

As described above, we labeled 5 x $10^4$ target cells with 200 μCi for 240 min. To avoid spontaneous release of $^{51}Cr$ by the cells the target cells were kept on ice for 45 min after the first washing step. The effector cells were added to the cultures using effector/target cell ratios of 3:1, 25:1, and 50:1. After incubation for 8 h at 37°C and 5 % $CO_2$ culture plates were centrifuged at 1000 RPM for 3 min and each supernatant was measured for the presence of $^{51}Cr$ in a scintillation. Negative control culture supernatants consisted of labelled cells without the addition of effector cells (spontaneous $^{51}Cr$-release of the cells). Positive control culture supernatants consisted of cultures of labeled cells after cell lysis using 10% Triton X-100 (maximum $^{51}Cr$-uptake). The results of the $^{51}Cr$-release of the individual cultures including the effector cells prior to immunization, two weeks after the 2nd and two weeks after the 3rd booster immunisation (see Table 11) are included as table 18. Figures 15a)- 15e) show the calculated specific lysis ≥ 0 according to Hammond SA, Issel CJ, and Montelaro RC (1998): General method for the detection and in vitro expansion of equine cytolytic T lymphocytes. J Immunol Method 213: 73-85.

The data sheets containing the $^{51}Cr$-release of the individual cultures (table 18) show the relative small standard deviations between the four individually handled cultures of each sample. Compared with the earlier cytotoxic T-cell assays performed with cells after the first immunization and the 1st booster immunization (see example 3), the differences between the negative control culture supernatants (spontaneous $^{51}Cr$-release of the cells) and the positive control culture supernatants (maximum $^{51}Cr$-uptake by the cells) are greater in the current assay. In general, e measured higher percentages of the calculated specific lysis (Fig. 15). In the cultures containing effector cells obtained prior to immunization, however, we also measured relatively high percentages of specific lysis. It would be expected to have higher amounts of $^{51}Cr$ released in the supernatants when higher number of effector cells were added to the targets. The values measured in the supernatants of the cultures with effector/target cell ratios of 3:1, 25:1, and 50:1, however, did not show such a consistence in specific lysis. Possibly, the number of antigen expressing target cells (approximately 20 %) are not high enough to enable the measurement of speicific lysis. To enlarge the number of antigen positive cells, we used in the current experiments 5 x $10^4$ target cells. Other possible reasons for the inconsistency of specific lysis are not efficient restimulations of the effector cells *in vitro* or other unknown methodical details.

Summary of the measured cytotoxic T-lymphocyte activities

Due to the inconsistency of the measured specific lysis it is difficult to draw conclusions concerning the antigen (s) most suited for the induction of cytotoxic T-lymphocytes. In an attempt to give an overview of the measured specific lysis we calculated for each ORF the average value (X) of the specific lysis of all different effector/target cell ratios (3: 1, 25:1, 50:1) and subtracted the average value (Y) of the negative controls (results of the cells before immunization), which were calculated similarly.

$$X = \frac{X_1(3:1)+X_2(25:1)+X_3(50:1)}{3} \qquad Y = \frac{X_1(3:1)+Y_2(25:1)+Y_3(50:1)}{3}$$

The results show the absolute increase of the cell lysis in percent at the indicated times for each ORF, compared to the values obtained before immunization.

Two different scales were used to express the increase of specific lysis. In example 5, Table 19, measured lysis between 0 and 5 % is given a + (plus). In example 5, Table 20, measured lysis between 0 and 5 % is given a - (minus). Horse Daggy was serologically positive before immunization. This explains the measured specific lysis already two weeks after the first immunization. Horse 'Frieda' had a weak antibody response before the first immunization. Cytotoxic T-lymphocytes directed against the expression product of ORF 2b and 7 were also measured in the blood of this animal two weeks after the first immunization. The serologically negative animals Nelke' and 'Friedrich' did not develop cytotixic T-lymphocytes after the first immunization. Only in horse 'Jessy', also serologically negative before immunization, cytotoxic T-lymphocytes directed against the expression product of ORF 7 were measured. Unfortunately, the specific lysis was not consistent at the following time points. After the 3$^{rd}$ booster immunization, however, specific lysis (> 10 %) eas measured in all animals. The activity of cytotoxic T-lymphocytes was directed in horse 'Frieda' and 'Jessy' against the expression product of ORF 5, in horse 'Daggy' and 'Friedrich' against the expression product of ORF 7 and in horse 'Nelke' against the expression product of ORF 7.

Table: 6: Content of a kit produced for DNA-vaccination of horses using expression vectors harboring and expressing individual gene of equine arteritis virus

| **Expression vectors** | **Concentration plasmid DNA μg/μl** | **Total of DNA mg** |
|---|---|---|
| pCR3.1-EAV-02-BX-C5<br>**Gene product: small glycoprotein** | 1 | 10 |
| pCR3.1-EAV-03-BX-C1<br>**Gene product: unknown** | 1 | 10 |
| pCR3.1-EAV-04-BX-C3<br>**Gene product: unknown** | 1 | 10 |
| pCR3.1-EAV-O5-BX-C14<br>**Gene product: large envelope glycoprotein** | 1 | 10 |
| pCR3.1-EAV-06-BE-C4<br>**Gene product: membrane protein** | 1 | 10 |
| pCR3.1-EAV-07-BX-C3<br>**Gene product: nucleocapsid protein** | 1 | 10 |
| pDP-EAV-05-BgS-Cl<br>**Gene product: large envelope glycoprotein** | 1 | 10 |
| pDP-EAV-07-BgS-C2<br>**Gene product: nucleocapsid protein** | 1 | 10 |
| pC3.1-HIS-EAV-O5del-121-C12Gene<br>**product: The N-terminal (121 aa) hydrophilic ectodomain of large envelope glycoprotein** | 1 | 10 |
| GD-HD-07-12-1999 | | |

Table: 7:

| Test of EAV specific antibodies in preimmune serum |
|---|
| The results of neutralization test (NT) and ELISA test in which the preimmune sera of 5 horses from Leipzig were screened for existence of a humoral immune response developed during a natural infection with wild type equine arteritis virus (EAV) |

(continued)

| Horse | Titer of serum NT[a] | Titer of serum ELISA | Remarks |
|---|---|---|---|
| Daggi | **1:40** | **1:100** | **positive** |
| Frieda | 1:20 | 1:50 | boundary |
| Friedrich | 1:10 | <1:50 | |
| Jessy | 1:10 | <1:50 | |
| Nelke | 1:10 | <1:50 | |
| Positive<br>Control Serum | 1:160[b]<br>1:80[c] | <br>1:200[c] | **positive**<br>**positive** |
| [a] Multiplicity of infection (MOI) : 100 PFU of EAV/1000 RK-13 3 cells/well<br>[b] Rabbit antiserum raised against EAV<br>[c] Horse serum was obtained from Prof. Dr. Ludwig, Berlin | | | |

Table 8:

| Duration of Immunity: | | | | | | | |
|---|---|---|---|---|---|---|---|
| Determination of antibody titer by neutralization test (NT) in the serum of 5 horses after DNA vaccination with the cDNA EAV ORF 2b,5 and 7 and cDNA encoding equine IL 2. | | | | | | | |
| Horse | Pre-vaccine Serum 22.5.2000 NT-Titer: | 1.Post-vaccine Serum 5.6.2000 NT-Titer: | 2.Post-vaccine Serum 28.7.2000 NT-Titer: | 3.Post-vaccine Serum 22.9.2000 NT-Titer: | 4. Post-vaccine Serum 19.1.2001 NT-Titer: | 5. Post-vaccine Serum 16.3.2001 NT-Titer: | 6. Post-vaccine Serum 11.5.2001 NT-Titer: |
| Daggy | 1:64 | 1:256 | 1:256 | 1:264 | 1:128 | 1:96 | 1:16 |
| Frieda | <1:2 | 1:256 | 1:256 | 1:264 | 1:64 | 1:32 | 1:16 |
| Friedrich | <1:2 | 1:64 | 1:128 | 1:512 | 1:48 | 1:32 | 1:8 |
| Jessy | <1:2 | 1:256 | 1:128 | 1:128 | 1:16 | 1:16 | 1:8 |
| Nelke | 1:8 | 1:256 | 1:256 | 1:128 | 1:16 | 1:16 | 1:16 |

Table 9:

| Immunisation schedule with the cDNA EAV ORF 2b,5 and 7 and with cDNA encoding equine IL 2. Combined application of cDNA (in μg) via Gene Gun (GG) and intramuscular injection (i.m.) | | | | |
|---|---|---|---|---|
| Horse | 1. Basic immunisation: 23.Mai 2000 | 1. Booster 6.Juni 2000 | 2. Booster 21.Juni 2000 | 3. Booster 14.Juli 2000 |
| Daggy | 35 μg GG + 1400 μg i.m | 35 μg GG + 1400 μg i.m | 35 μg GG + 1400 μg i.m | 35 μg GG + 1400 μg i.m |
| Frieda | 35μg GG+1400 μg i.m | 35 μg GG + 1400 μg i.m | 35 μg GG + 1400 μg i.m | 35 μg GG + 1400 μg i.m |
| Friedrich | 35 μg GG+ 1400 μg i.m | 35 μg GG + 1400 μg i.m | 35 μg GG + 1400 μg i.m | 35 μg GG + 1400 μg i.m |
| Jessy | 35 μg GG + 1400 μg i.m | 35 μg GG + 1400 μg i.m | 35 μg GG + 1400 μg i.m | 35 μg GG + 1400 μg i.m |
| Nelke | 35 μg GG + 1400 μg i.m | 35 μg GG + 1400 μg i.m | 35 μg GG + 1400 μg i.m | 35 μg GG + 1400 μg i.m |

Table 10: Age of Horses

| horses | age (years) | anti-EAV antibodies* |
|---|---|---|
| Daggi | 22 | ++ |
| Frieda | 16 | ? |
| Jessy | 16 | - |
| Friedrich | 10 | - |
| Nelke | 6 | - |

*before immunisation (10/99)

Table 11: Sample collection and immunisation regimen

| | DNA application | collection of plasma samples | isolation of PBMC* |
|---|---|---|---|
| prae-immunisation | n.d. | 02.03.00 | 02.03.00 |
| prae-immunisation | n.d. | 23.03.00 | 23.03.00 |
| prae-immunisation | n.d. | 22.05.00 | 22.05.00 |
| Immunisation | 23.05.00 | 05.06.00 | 05.06.00 |
| 1. Booster | 06.06.00 | 20.06.00 | 20.06.00 |
| 2. Booster | 21.06.00 | 13.07.00 | 13.07.00 |
| 3. Booster | 14.07.00 | 28.07.00 | 28.07.00 |
| | n.d. | 25.08.00 | 25.08.00 |
| | n.d. | 22.09.00 | 22.09.00 |

* peripheral blood mononuclear cells
n.d.: not done

Table 12

Skin Reaktions of Horses: 24 hours post injectionem after first immunisation*

| | Daggy | | Frieda | | Nelke | | Friedrich | | Jessy | |
|---|---|---|---|---|---|---|---|---|---|---|
| Numbers of papules | le: 6<br>ri: 6 | | le: 4<br>ri: 6 | | le: 2<br>ri: 1 | | le: 5<br>ri: 5 | | le: 6<br>ri: 6 | |
| Description of papules | le: severe swollen, epidermis becomes eroded<br>ri: severe swollen, 1 papule without epidermis | | le: severe swollen, epidermis becomes severe eroded<br>ri: moderate swollen, Str.c. mild eroded | | le: moderate swollen, Str.c. normal<br>ri: see left | | le: not to mild swollen, Str.c. normal<br>ri: moderate swollen, Str.c. normal | | le: mild to moderate swollen, Str.c. scaled<br>ri: mild swollen, Str.c. scaled | |
| Skin thickness | le: | ri: | le: | ri: | le: | ri: | le: | ri: | le: | ri: |
| i) normal | 0.25 cm | 0.30 cm | 0.30 cm | 0.30 cm | 0.20 cm | 0.20 cm | 0.30 cm | 0.35 cm | 0.40 cm | 0.40 cm |
| ii) Papules | 0.60 cm | 0.60 cm | 0.60 cm | 0.60 cm | 0.45 cm | 0.40 cm | 0.40 cm | 0.40 cm | 0.60 cm | 0.60 cm |

(continued)

| | | | | | | | | | | |
|---|---|---|---|---|---|---|---|---|---|---|
| Skin thickness | le: | ri: | le: | ri: | le: | ri: | le: | ri: | le: | ri: |
| Diameter | le: 1.1-1.2cm<br>ri: 1.0 cm | | le: 0.8-1.0cm<br>ri: 0.7-0.8cm | | le: 0.6-0.8cm<br>ri: 0.7cm | | le: 1.0-1.1cm<br>ri: 0.9-1.2 cm | | le: 0.5-0.6 cm<br>ri: 0.5 cm 1 papule: 1.0 cm | |
| Features | | | On both sides: gnat bites with allergic oedema | | Very thin skin, le: 2 white circles | | 1papule: Str.c. mild swollen, congested and folding of the skin | | 1 papule with erosin, strong oedema and pruitus | |
| Le: left side of neck; ri: right sight of neck; Str.c.: Stratum corneum;<br>* Immunisation date: 23/05/2000; Skin reaction protocol date: 24/05/2000 | | | | | | | | | | |

Table 13

| Skin Reaktions of Horses: 48 hours post injectionem after first immunisation* | | | | | | | | | | |
|---|---|---|---|---|---|---|---|---|---|---|
| | Daggy | | Frieda | | Nelke | | Friedrich | | Jessy | |
| Numbers of papules | le: 6<br>ri: 6 | | le: 4<br>ri: 6 | | le: 2<br>ri: 1 | | le: 5<br>ri: 5 | | le: 6<br>ri: 6 | |
| Description of papules | le: severe swollen, epidermis becomes eroded<br>ri: severe swollen, 1 papule without epidermis | | le: severe swollen, epidermis becomes severe eroded<br>ri: moderate swollen, Str.c. mild eroded | | le: moderate swollen, Str.c. normal<br>ri: see left | | le: no to mild swollen, Str.c. normal<br>ri: moderate swollen, Str.c. normal | | le: mild to moderate swollen, Str.c. scaled<br>ri: mild swollen, Str.c. scaled | |
| Skin thickness | le: | ri: | le: | ri: | le: | ri: | le: | ri: | le: | ri: |
| i) normal | 0.25 cm | 0.30 cm | 0.30 cm | 0.30 cm | 0.20 cm | 0.20 cm | 0.30 cm | 0.35 cm | 0.40 cm | 0.40 cm |
| ii) Papules | 0.70 cm | 0.60 cm | 0.50 - o. 60cm | 0.45 cm | 0.45 cm | 0.40 cm | 0.40 cm | 0.40 cm | 0.60 cm | 0.60 cm |
| Diameter | le: 1.1 - 1.2 cm<br>ri: 1.0 cm | | le: 0.8 - 1.0 cm<br>ri: 0.7 - 0.8 cm | | le: 0.6 - 0.8 cm<br>ri: 0.7 cm | | le: 1.0 -1.1 cm<br>ri: 0.9 - 1.2 cm | | le: 0.5<br>ri: 0.9 -1.2 cm | |
| Features | | | On both sides: gnat bites with allergic oedema | | Very thin skin, le: 2 white circles | | 1 papule: Str.c. mild swollen, congested and folding of the skin | | 1 papule with erosin, strong oedema and pruitus | |
| Le: left side of neck; ri: right sight of neck; Str.c.: Stratum corneum; * Immunisation date: 23/05/2000; Skin reaction protocol date: 24/05/2000 | | | | | | | | | | |

**Example 3 Cytotoxic T-lymphocyte (CTL Assay)**

**1. Immunisation of horses**

1.1. DNA application and observed skin reactions

The horses (Daggy, Frieda, Friedrich, Jessy, Nelke) were immunised according to the immunisation regimen (Table 14) by intramuscular (i.m.) injection and intradermal gene gun application of EAV ORF-2b,EAV ORF-5, and EAV ORF-7 expression plasmids. The i.m. inoculations were applied to the musculi semimembranosus/semitendinosus/ gluteus. The gene gun applications of the DNA were performed on both sides of the neck. To enable an optimal gene gun DNA application the corresponding parts of the skin (40 $cm^2$) had been shaved before. The horses were investigated 24 h after the DNA applications for systemic and local reactions. None of the animals developed fever or local reactions such as thickening of the skin, development and involution of papules. Protocols are included as Tables 12 and 13 (see above). Photographs of the skin areas involved were taken 24 h after DNA application.

Table 14. Sample collection and immunisation regimen

| | DNA application | collection of plasma samples | isolation of PBMC* |
|---|---|---|---|
| Prae-immunisation | n.d. | 02.03.00 | 02.03.00 |
| Prae-immunisation | n.d. | 23.03.00 | 23.03.00 |
| Prae-immunisation | n.d. | 22.05.00 | 22.05.00 |
| Immunisation | 23.05.00 | 05.06.00 | 05.06.00 |
| 1. Booster | 06.06.00 | 20.06.00 | 20.06.00 |
| 2. Booster | 21.06.00 | 13.07.00 | 13.07.00 |
| 3. Booster | 14.07.00 | 28.07.00 | 28.07.00 |

*peripheral blood mononuclear cells
n.d.: not done

1.2. Detection of EAV-specific antibodies

Serum and plasma samples of the immunised animals were taken two/three weeks after the DNA applciations as outlined in Table 14.

**2. Preparation of target cells**

2.1. Transfection experiments in T25 cell culture flasks

Skin fibroblasts (1.43 x $10^6$) cells) were incubated at 37˚C and 5 % and 5 % $CO_2$ in T25 (25 $cm^2$) cell culture flasks using Dulbecco's modified Eagles medium (DMEM) containing 10 % fetal calf serum (FCS) and antibiotics. Cells were transfected with 100 μg DNA of a Green Fluorescent Protein (GFP) expression plasmid in 1.3 ml Optimem® (Gibco) by the addition of 87.5 μl LipofectAMINE® (Gibco). After 24 h cells were treated with trypsin and seeded in a concentration of 5 x $10^3$ to 5 x $10^4$ cells per well in 96-well-plates. About 20 % of the cells were transfected after 24 h of incubation at 37˚C and 5 % $CO_2$ as determined by immunofluorescence.

2.2. Titration experiments using polyclonal EAV-specific rabbit serum (11.08.98)

Vero-cells ($10^4$) were seeded in each well of a 96-well-plate and grown over night (o/n) in DMEM culture medium containing 10 % FCS and antibiotics at 37˚C and 5 % $CO_2$. The monolayers were subsequently infected with a 1:100 or 1:1000 diluted Vero-cell propagated EAV stock (Institute of Virology, University of Leipzig). The first EAV-specific cytopathic effects (CPE) were observed 36 h post infection and the cultures were immediately fixed for 30 min with ethanol at 4˚C. The 96-well-plate was stored with ethanol at -70˚C.

For immunohistochemical detection of viral antigens in infected cells, cultures were rinsed for 5 min with phosphate buffered saline (PBS) at room remperature (RT). In order to block endogenous peroxidases, cultures were incubated for 5 min with 7.5 % $H_2O_2$ in methanol. Cells were rinsed with PBS and incubated for 1 h with serum dilutions (polyclonal EAV-specific rabbit serum from 11.08.98, pre-immune [rabbit #98/8] from 10.06.98 ranging from 1:100 and 1:200 up to 1:1600 in 0.05 % Tween 20-PBS. Cultures were rinsed twice with

0.05 % Tween 20-PBS and once with destilled water and incubated for 1 h with a biotinylated anti-rabbit antibody diluted 1:750 at 37˚C. Cells were rinsed as before with Tween 20-PBS and destilled water and incubated for 30 min at 37˚C with streptavidin-peroxidase (1:500 in Tween 20-PBS). Cultures were rinsed again and the substrate AEC (14,25 ml Na-Acetat buffer 0.75 μl AEC, 75 μl $H_2O_2$ 1:10 diluted in destilled water) was added. Even in the highest antibody dilution (1:1600) clear positive reactions were visible.

2.3. Transfection of skin fibroblasts with EAV-specific expression plasmids

Transfection experiments were performed similarly to the protocol described in example 2 using the 24-well-plate format. Immunohistochemical analysis using the polyclonal EAV-specific rabbit serum (11.08.98), diluted 1:800 in 0.05 % Tween 20-PBS, showed that about 20 % of the cells were transfected with the EAV ORF-2b, EAV ORF-5, and EAV ORF-7 expression plasmids.

2.4. Culturing of target cells prior to CTL assay

Skin fibroblasts of the horses were cultured in Iscove's modified Eagles medium (IMEM) containing 10 % FCS and antibiotics. Cultures were extended to three T25 cell culture flasks and transfected when being confluent for 80-90 % with each 100 μg of the EAV ORF-2b, EAV ORF-5, and EAV ORF-7 expression plasmids. After 24 h, cultures were treated with trypsin, cells were counted and labeled in suspension with $^{51}$Cr.

**3. Preparation of effector cells**

3.1. Inactivation of EAV using $^{137}$Cs γ-rays

In order to obtain viral antigen for the restimulation of EAV-specific effector cells, Vero-cells were infected with EAV at a multiplicity of infection (moi) of 1. After 36 h, cell cultures were freeze/thawed two times and the cultures were centrifuged for 5 min at 1750 x g. The supernatant was pelleted by centrifugation o/n at 19.000 RPM. The pellet was resuspended in 3 ml PBS and the protein concentration was measured using a BCA-Protein Assay® (Pierce). Aliquots of the samples containing 2 mg/ml were stored at -70˚C.

Samples were exposed on dry ice to $^{137}$Cs γ-rays ranging from 15 Gy up to 1 k Gy. Virus titration on Vero-cells revealed that the infectious titre had decreased only by < log1, even after exposure to 1 k Gy.

3.2 Inactivation of EAV using ultraviolet radiation

Because EAV could not be inactivated by $^{137}$Cs γ-rays, samples were exposed to ultraviolet radiation (254 nm) at a distance of 5 cm for 5 min up to 80 min. Virus titration on Vero-cells revealed that no infectious virus could be detected after a 5 min exposure. To completely inactivate EAV in the samples used for the restimulation of the effector cells, these samples were exposed for 30 min and stored aliquots containing 2 mg of protein per ml at -70˚C.

3.2. Culturing of effector cells prior to CTL assay

PBMC were cultured in IMEM containing 10 % FCS and antibiotics. After stimulation for two days with 2.5 μg/ml pookweed mitogen, cells were cultured for two days in the presence of 200 U of human recombinant IL-2 (Amersham-Pharmacia). EAV-specific cytotoxic T-cells were restimulated for four days with 30 μg/ml inactivated EAV in the presence of 100 U IL-2. Thereafter, cultures were extended for four days in the presence of 200 U of IL-2. Cells were counted and used in the CTL assay.

**4. Measurement of EAV-specific cytotoxic T-cells**

4.1. $^{51}$Cr-labeling of target cells

After trypsin treatment, transfected target cells were resuspended in 1.5 ml and labeled for 2 h with 100 μCi $^{51}$Cr/$10^7$ cells. Cells were washed three times with culture medium and 7.7 x $10^3$ cells were seeded in each well of a 96 U-bottom cell culture plate in a volume of 150 μl. Cultures were incubated for 4 h at 37˚C and 5 % $CO_2$ to let cells adhere to the plate.

4.2. Addition of effector cells

PBMC were counted and added to the cultures in 100 μl volumes and effector/target cell ratios of 3:1, 25:1, and 50:1. Cells were incubated for 8 h in a total volume of 250 μl at 37°C and 5 % $CO_2$.

4.3. Measurement of $^{51}$Cr release in cultures with different effector/target cell ratios

Culture plates were centrifuged at 1000 RPM for 3 min and each supernatant was measured for the presence of $^{51}$Cr in a scintillation counter for 60 sec. Negative control culture supernatants consisted of labeled cells without the addition of effector cells (spontaneous $^{51}$Cr release of the cells). Positive control culture supernatants consisted of cultures of labeled cells after cell lysis using 10 % Triton X-100 (maximum $^{51}$Cr uptake). The results of the $^{51}$Cr release of the individual cultures including the effector cells prior to immunisation, two weeks after the 2nd immunisation, and two weeks after the 3$^{rd}$ booster immunisation (Table 14) are included s. table 18. Figures 9a-e) show the calculated specific lysis ≥ 0 according to Hammond SA, Issel CJ, and Montelaro RC (1998): General method for the detection and in vitro expansion of equine cytolytic T lymphocytes. J. Immunol Methods 213: 73-85.

The data sheets of the $^{51}$Cr release of the individual cultures show a relative small difference between the negative control culture supernatants (spontaneous $^{51}$Cr release of the cells) and the positive control culture supernatants (Maximum $^{51}$Cr uptake by the cells). The specific lysis is therefore difficult to measure, even if the standard deviations between the four individually handled cultures of each sample are small (Table 18).

**Example 4 DNA-Vaccination of horses**

1. Schedule for DNA for Vaccination of horses

| | | |
|---|---|---|
| May 23, 2000 | 1$^{st}$ immunization | day 0 |
| June 6, 2000 | 2$^{nd}$ immunization | day 14 |
| June 21, 2000 | 3$^{rd}$ immunization | day 29 |
| July 14,2000 | 4$^{th}$ immunization | day 51 |

2. Strategy and preparation of DNA for Vaccination

2.1. Application of DNA using Helios Gene Gun System (BIO-RAD):
2.1.1. Number of shots per animal and per vaccination: 10
2.1.2. Total DNA per shot: 3.5 μg
2.1.3. Number of expression vectors used: 7 (the properties and source of the DNA used for vaccination are summarized in Table 15)

| | |
|---|---|
| 1. pCR3.1-EAV-02-BX-C5 | 5. pCR3.1-EAV-07-BX-C3 |
| 2. pCR3.1-EAV-O5-BX-C14 | 6. pDP-EAV-07-BgS-C2 |
| 3. pDP-EAV-O5-BgS-C1 | 7. pCR3.1-Horse-IL2 |
| 4. pCR3.1-HIS-EAV-O5del-121-C12 | |

2.1.4. DNA of individual expression vector per shot: 0.5 μg = 500 ng
2.1.5. Total DNA of individual expression vector per animal and per vaccination: 5 μg
2.1.6. Total DNA per vaccination per animal: 7 x 5 = 35 μg
2.1.7. Number of animals: 5
2.1.8. Total DNA of individual expression vector per vaccination: 5 x 35 = 175 μg
2.1.9. Number of vaccination: 4
2.1.10. Total DNA of individual expression vector administered: 175 x 4 = 700 μg
**2.2. Application of DNA through intramuscular route.**
2.2.1. Number of inoculations per animal and per vaccination: 4
2.2.2. DNA of individual expression vector per inoculation: 50 μg

2.2.3. Total DNA of individual expression vector per animal and per vaccination: 50 x 4 =200 μg
2.2.4. Number of expression vectors used: 7 (see Table 15)
2.2.5. Total DNA per vaccination per animal: 7 x 200= 1.4 mg
2.2.6. Number of animals: 5
2.2.7. Total DNA of expression vector administered: 5 x 1.4 = 7.0 mg
2.2.8. Number of vaccination: 4
2.2.9. Total DNA of individual expression vector administered: 200 x 5 x 4 = 4 mg
2.2.10. Transfection reagent:

DOTAP Liposomal (Roche; Cat.# 1811177) 50 μg/ml BME (3 ml/animal)
Buffer I containing DNA (yellow cup tube)
Lipofectin (Life Technology; Cat.# 18292-011) 50 μg/ml BME (3 ml/animal)
Buffer II (white cup tube) add to DNA prior to application

**Table: 15: Properties of the cDNA used for the undividual DNA-vaccination of horses**

| ***Expression vectors*** | **DNA used per vaccination by μg/horse** | |
|---|---|---|
| | **Gene GUN** | **Classical i.m.** |
| PCR3.1-EAV-O2-BX-C5 (1μg/μl)* 1.clone/5. Passage 16.06.1999<br>**Gene product: small glycoprotein** | **5** | **200** |
| pCR3.1-EAV-O3-BX-C1 (1μg/μl)* 1.clone/6. Passage 17.06.1999<br>**Gene product: unknown** | | |
| pCR3.1-EAV-O4-BX-C3 (1μg/μl)* 1.clone/6. Passage 18.06.1999<br>**Gene product: unknown** | | |
| pCR3.1-EAV-O5-BX-C14 (1μg/μl) 1.clone/5. Passage 05.06.1999<br>pDP-EAV-O5-BgS-Cl (1μg/μl) 1.clone/5. Passage 07.06.1999<br>pCR3.1-HIS-EAV-O5del-121-C12 (1μg/μl) 1.clone/6. Passage 04.06.1999<br>**Gene product: large envelope glycoprotein** | **5**<br>**5**<br>**5** | **200**<br>**200**<br>**200** |
| pCR3.1-EAV-O6-BE-C4 (1μg/μl) 1.clone/5. Passage 15.06.1999<br>**Gene product: membrane protein** | | |
| pCR3.1-EAV-O7-BX-C3 (1μg/μl) 1.clone/6. Passage 09.06.1999<br>pDP-EAV-O7-BgS-C2 (1μg/μl) 1.clone/5. Passage 10.06.1999<br>**Gene product: nucleocapsid protein** | **5.**<br>**5** | **200**<br>**200** |
| pCR3.1-Horse-IL2 (1μg/2μl) 2.clone/2. Passage 06.02.2000<br>**Gene product: Horse interleukin-2** | **5** | **200** |

3. Preparation of the DNA for vaccination of horses using Helios Gene Gun System (BIO-RAD) and optimization Kit (Catalog 165-2424)
3.1. Calculation:

3.1.1. MLQ=1(1mgAu/shot)

3.1.2. DRL = 3.5 (3.5 μg DNA/ mg Au, particle =1.6 μm)
3.1.3. Cartridge length = 13 mm
3.1.4. Nalgene tubing = 750 mm
3.1.5. Factor for Au = 750/13 = 58 mg Au
3.1.6. 58 x-3,5-μg DNA =203 μg DNA ~ 210 μg DNA

3.2. Procedure:

3.2.1. In a 1.5 ml microfuge tube, weigh out 60 mg Au.
3.2.2. To the measured Au add 210 μl 0.05 M Spermidine.
3.2.3. Vortex # 3.2.2. for few seconds, then sonicate 3-5 seconds.
3.2.4. To the Au and Spermidine mixture, add 210 μg total DNA of seven expression vectors = 30 μg of individual expression vectors).
3.2.5. Mix DNA, Spermidine and Au by vortexing ~ 5 seconds.
3.2.6. Add 210 μl $CaCl_2$ dropwise into # 3.2.5. while vortexing at a moderate rate
3.2.7. Precipitate for 10 min in room temperature.
3.2.8. Most of the Au will now be in a pellet, but some may be on the sides of the tube.
The supernatant should be relatively clear. Spin the microcarrier solution in a microfuge for 15 seconds to pellet the Au.
3.2.9. Remove the supernatant and discard.
3.2.10. Resuspend the pellet in the remaining supernatant by vortexing briefly. Wash the pellet three times with 1 ml 100 % ethanol each time.
3.2.11. Spin for 5 seconds in a microfuge between each wash. Discard the supernatants.
3.2.12. After the final ethanol wash, resuspend the pellet in 200 μl of the ethanol solution containing 0.05 mg/ml PVP in ethanol.
3.2.13. Add # 3.2.12 in 2800 μl 0.05 mg/ml PVP in ethanol.
3.2.14. Preparation of cartridges:

May 20, 2000: 300 cartridges were prepared according to the above protocol and the Helios Gene Gun system Instruction Manual. Each cartridge = single shot contains 3.5 μg DNA corresponding to 0.5 μg = 500 ng DNA of individual expression vectors.

4. Preimmune sera

The preimmune sera of 5 horses (Daggi, Frieda, Friedrich, Jessy, and Nelke) that were obtained in October 1999 and analysed by NT and ELISA tests developed in our laboratory for screening of antibodies against equine arteritis virus (EAV). The results are summarized in Table 16.

Table 16: The results of neutralization (NT) and ELISA tests in which the preimmune sera of 5 horses were screened for existence of a humoral immune response developed during a natural infection with wild type equine arteritis virus (EAV)

| No. | Horse | Titre of serum NT° | Titre of serum ELISA | Remarks |
|---|---|---|---|---|
| 1 | Daggi | 1:40 | 1:100 | **positive** |
| 2 | Frieda | 1:20 | 1:50 | boundary |
| 3 | Friedrich | 1:10 | <1:50 | |
| 4 | Jessy | 1:10 | <1:50 | |
| 5 | Nelke | 1:10 | <1:50 | |
| | Positive Control Serum | 1:160[b]<br>1:80[c] | 1:200 | **positive**<br>**positive** |

[a] Multiplicity of infection (MOI): 100 PFU of EAV/1000 RK-13 cells/well
[b] Rabbit antiserum raised against EAV
[c] Horse serum was obtained from Prof. Dr. Ludwig, Berlin

Vaccination protocol
Tuesday, May 23, 2000
1. Race of horses: Jessy: Shetland Pony, Rest: so called " Warm-blooder"
2. Age of horses

2.1. Daggi: 22 years old
3.2. Frieda: 16 years old
3.3. Friedrich: 10 years old
3.4. Jessy: 16 years old
3.5. Nelke: 6 years old

3. Vaccination:

| | | | | |
|---|---|---|---|---|
| 3.1. Jessy | start | 12.15 | end | 12.22 (10 × shot: 5 per neck side) |
| 3.2. Nelke | start | 12.30 * | end | 12.38 (10 x shot: 5 per neck side) |
| 3.3. Daggi | start | 12.45 §& | end | 12.55 (12 x shot: 6 per neck side) |
| 3.4. Frieda | start | 12.47 §& | end | 12.57 (12 x shot: 6 per neck side) |
| 3.5. Friedrich | start | 13.10 § | end | 13.15 (12 x shot: 6 per neck side) |

* Sedativum: Domosedan (Pfizer) = Methyl-4-hydrooxybenzoad 1 ml i.v. (10 mg)
§ Rometar (Serum-Werk Bernburg) = Xylazin i.v. Methyl-4-hydrooxybenzoad
& Daggi and Frieda had been vaccinated together

4. Sera: 2nd preimmune serum was taken on Monday, May 22, 2000 (3 ml per animal)
5. May 24, 2000: All vaccinated horses developed skin reactions "DTH-reaction" at the target of the shot.

Tuesday, June 6, 2000

6. Sera: 1st post vaccinated serum was taken on Monday, June 5, 2000 (3 ml per animal)

7. Vaccination:

| | | | | |
|---|---|---|---|---|
| 3.1. Frieda | start | 12.25* | end | 12.30 (10 x shot: 5 per neck side) |
| 3.2. Daggi | start | 12.27* | end | 12.33 (10 x shot: 5 per neck side) |
| 3.3. Jessy | start | 12.37* | end | 12.43 (10 x shot: 5 per neck side) |
| 3.4. Nelke | start | 12.44* | end | 12.47 (10 x shot: 5 per neck side) |
| 3.5. Friedrich | start | 12.49* | end | 12.54 (10 x shot: 5 per neck side) |

* Sedativum: Rometar = Xylazin i.v. Methyl-4 hydrooxybenzoad 2% (Serum-Werk Bernburg)

Vaccination protocol Tuesday, June 21, 2000
8. Sera: 2nd post vaccinated serum was taken on Monday, June 20, 2000 (1.5 ml per animal)

9. Vaccination:

| | | | | |
|---|---|---|---|---|
| 3.1. Frieda | start | 12.10* | end | 12.16 (12 x shot: 6 per neck side) |
| 3.2. Daggi | start | 12.17* | end | 12.20 (12 x shot: 6 per neck side) |
| 3.3. Jessy | start | 12.25* | end | 12.28 (12 x shot: 6 per neck side) |
| 3.4. Nelke | start | 12.29* | end | 12.32 (11 x shot: 5 & 6 per neck side) |

(continued)

| | | | | |
|---|---|---|---|---|
| 3.5. Friedrich | start | 12.36* | end | 12.39 (12 x shot: 6 per neck side) |
| * Sedativum: Rometar = Xylazin i.v. Methyl-4 hydrooxybenzoad 2% (Serum-Werk Bernburg) | | | | |

Vaccination protocol
Friday, July 14, 2000

10. Sera: 3rd post vaccinated serum was taken on July 13, 2000 , 1.5 ml per animal

11. Vaccination:

| | | | | | |
|---|---|---|---|---|---|
| 3.1. Daggi | start | 12.00* | end | 12.07 | (12 x shot: 6 per neck side, 530 psi) |
| 3.2. Frieda | start | 12.08* | end | 12.11 | (12 x shot: 6 per neck side, 530 psi) |
| 3.3. Jessy | start | 12.21* | end | 12.24 | (12 x shot: 6 per neck side, 430 psi) |
| 3.4. Nelke | start | 12.25* | end | 12.27 | (12 x shot: 6 per neck side, 500 psi) |
| 3.5. Friedrich | start | 12.35* | end | 12.42 | (12 x shot: 6 per neck side, 400 psi) |

* Sedativum: Domosedan (Pfizer) = Methyl-4-hydrooxybenzoad 1 ml i.v. (10 mg)
§ Rometar (Serum-Werk Bernburg) = Xylazin i.v. Methyl-4-hydrooxybenzoad

12. Sera: 4th post vaccinated serum was taken on July 28, 2000, 1.5 ml per animal

13. Determination of neutralizing antibody

The determination of neutralizing antibodies of individual horse sera (30 serum samples that was labelled with code number 1-30, see Table 3) was performed. The results of neutralization tests are summarized in Table 17.

Table 17: The results of neutralization tests (NT) in which the sera of five horses were screened for detection of a humoral immune response developed after DNA vaccination with the cDNA of ORFs 2b,5, and 7 of equine arteritis virus (EAV) as indicated in Table 15 and 16

| Horse | NT-Titre of individual horses taken at the different times prior and post DNA-Vaccination[a] | | | | | |
|---|---|---|---|---|---|---|
| | preimmune serum 1<br>20.10.1999 | preimmune serum 2<br>22.05.2000 | post vaccinated serum 1<br>05.06.2000 | post vaccinated serum 2<br>20.06.2000 | post vaccinated serum 3<br>13.07.2000 | post vaccinated serum 4<br>28.07.2000 |
| 1. Daggi | 1:16 (#01) | 1:64 (#06) | 1:256 (#11) 1:256[e] | 1:256 (#16) | 1:256 (#21) 1:256[e] | 1:256 (#26) |
| 2. Frieda | ≤1:2(#02) | ≤1:2 (#07) | 1:256 (#12) 1:256[e] | 1:256 (#17) 1:256[e] | 1:128 (#22) | 1:256 (#27) 1:256[e] |
| 3. Friedrich | ≤1:2 (#03) | ≤1:2 (#08) | 1:64 (#13) | 1:128 (#18) | 1:128 (#23) | 1:128 (#28) |
| 4. Jessy | ≤1:2 (#04) | ≤1:2 (#09) | 1:256 (#14) 1:256[e] | 1:256 (#19) 1:256[e] | 1:256 (#24) 1:256[e] | 1:128 (#29) |

(continued)

| Horse | NT-Titre of individual horses taken at the different times prior and post DNA-Vaccination[a] | | | | | |
|---|---|---|---|---|---|---|
| | preimmune serum 1<br>20.10.1999 | preimmune serum 2<br>22.05.2000 | post vaccinated serum 1<br>05.06.2000 | post vaccinated serum 2<br>20.06.2000 | post vaccinated serum 3<br>13.07.2000 | post vaccinated serum 4<br>28.07.2000 |
| 5. Nelke | ≤1:2 (#05) | 1:8 (#10) | 1:256 (#15)<br>1:512 (#15) | 1:128 (#20) | 1:256 (#25)<br>1:512[e] | ≤1:2 (#30) 1:256[d] |

[a] DNA-Vaccination of horses was performed as follows:
May 23, 2000: 1st immunization (day 0), June 6, 2000: 2nd immunization (day 14),
June 21, 2000: 3rd immunization (day 29), and July 14, 2000: 4th immunization (day 51)
[b] The number in parenthesis indicate the code number of individual sera used by neutralization test, that was performed by Dr. Herzog, Veterinary laboratory at the Institute for clinical analysis, 71611 Ludwigsburg, Germany.
[c] This titre has to be checked again.
[d] The titre 1:2 was a type mistake. The correct titre was found to be 1:256, (Dr. Herzog laboratory, 08/20/2000)
[e] The results of the second analysis obtained from those sera with a titre of 1:256, (Dr. Herzog laboratory, 09/06/2000)

Biological material

14. Sera: 5th post vaccinated serum was taken on August 25, 2000 (2 x 1,5 ml per animal)

15. Sera: 6th post vaccinated serum was taken on September 22, 2000 (2 x 1,5 ml per animal)

16. Sera: 7th post vaccinated serum was taken on October 23, 2000 (2 x 1,5 ml per animal).

Example 5

Table 18: $^{51}$Cr-release of the individual cultures

2. CTL-Assay, 10./11.08.2000

| Horse | Cells | ORF | | Negative Control | Positive Control | Prior to Immunization E:T 3 to 1 | Prior to Immunization E:T 25 to 1 | Prior to Immunization E:T 50 to 1 | After the 2nd Booster E:T 3 to 1 | After the 2nd Booster E:T 25 to 1 | After the 2nd Booster E:T 50 to 1 | After the 3rd Booster E:T 3 to 1 | After the 3rd Booster E:T 25 to 1 | After the 3rd Booster E:T 50 to 1 |
|---|---|---|---|---|---|---|---|---|---|---|---|---|---|---|
| Daggy | Transfected | 2b | | | | | | | | | | | | |
| | | | | 337 | 791 | 448 | 521 | 710 | 503 | 444 | 312 | 558 | 594 | |
| | | | | 381 | 815 | 495 | 661 | 528 | 473 | 512 | 417 | 480 | 840 | |
| | | | | 392 | 858 | 549 | 389 | 509 | 600 | 481 | 402 | 660 | 541 | |
| | | | | 360 | 952 | 542 | 558 | 457 | 709 | 516 | 407 | 415 | 738 | 791 |
| | | | Mean | 362,5 | 854 | 508,5 | 532,25 | 551 | 546,25 | 488,25 | 384,5 | 529,75 | 680 | 791 |
| | | | SD | 22,6757983 | 70,969477 | 46,921921 | 112,378453 | 110,166541 | 109,335483 | 33,3903678 | 48,7339717 | 108,018674 | 139,63972 | #DIV/0! |
| | | | Specif. Lysis | | | 0,29704985 | 0,34537131 | 0,38351984 | 0,37385554 | 0,25584944 | 0,04476094 | 0,34028484 | 0,84598169 | 0,87182098 |
| Daggy | Transfected | 5 | | 211 | 499 | | | | | | | | | |
| | | | | 252 | 594 | | | | | | | | | |
| | | | | 243 | 656 | | | | | | | | | |
| | | | | 221 | 598 | | | | | | | | | |
| | | | | | | 250 | 255 | 297 | 192 | 259 | 263 | 226 | 233 | |
| | | | | 255 | 501 | 277 | 277 | 330 | 228 | 288 | 259 | 232 | 280 | 247 |
| | | | | 270 | 462 | 285 | 298 | 330 | 223 | 264 | 298 | 242 | 279 | 252 |
| | | | | 238 | 637 | 225 | 282 | 236 | 244 | 274 | 301 | 200 | 223 | 247 |
| | | | Mean | 241,142857 | 549,571429 | 261,75 | 277,5 | 298,25 | 221,75 | 270,75 | 280,25 | 225 | 248,75 | 248,666667 |
| | | | SD | 20,3566683 | 64,0178547 | 30,696091 | 17,0195965 | 44,3198601 | 21,7619699 | 11,9268604 | 22,3215143 | 17,9257729 | 25,5130685 | 2,88675135 |
| | | | Specif. Lysis | | | 0,06681334 | 0,11787865 | 0,18515516 | -0,06287633 | 0,09599352 | 0,12679481 | -0,05233905 | 0,0246842 | 0,02439401 |
| Daggy | Transfected | 7 | | | | | | | | | | | | |
| | | | | 350 | 542 | 612 | 449 | 450 | 465 | 493 | 407 | 271 | 249 | 251 |
| | | | | 308 | 719 | 564 | 553 | 445 | 363 | 499 | 439 | 296 | 290 | 350 |
| | | | | 281 | 609 | 462 | 445 | 362 | 453 | 630 | 391 | 241 | 265 | 484 |
| | | | | 270 | 578 | 577 | 435 | 385 | 389 | 428 | 430 | 301 | 297 | 493 |
| | | | Mean | 297,25 | 611,5 | 553,75 | 470,5 | 410,5 | 417,5 | 512,5 | 416,75 | 277,25 | 275,25 | 394,5 |
| | | | SD | 40,6398788 | 78,7094084 | 64,4379547 | 65,3142537 | 43,7911711 | 49,3254408 | 84,6738842 | 21,8231520 | 27,5 | 22,2487226 | 115,880886 |
| | | | Specif. Lysis | | | 0,81822912 | 0,65131265 | 0,36036186 | 0,38265712 | 0,6849642 | 0,38027040 | -0,0638436 | -0,07000786 | 0,30946098 |

Table 18 a)

2. CTL-Assay, 10./11.08.2000

Table 18 b)

| Horse | Cells | ORF | Negative Control | Positive Control | Prior to Immunization E:T 3 to 1 | Prior to Immunization E:T 25 to 1 | Prior to Immunization E:T 50 to 1 | After the 2nd Booster E:T 3 to 1 | After the 2nd Booster E:T 25 to 1 | After the 2nd Booster E:T 50 to 1 | After the 3rd Booster E:T 3 to 1 | After the 3rd Booster E:T 25 to 1 | After the 3rd Booster E:T 50 to 1 |
|---|---|---|---|---|---|---|---|---|---|---|---|---|---|
| Jessy | Transfected | 2b | 516 | 404 | | | | | | | | | |
| | | | 476 | 514 | | | | | | | | | |
| | | | 488 | 448 | | | | | | | | | |
| | | | 381 | 477 | | | | | | | | | |
| | | | | 1060 | 687 | 526 | 539 | 423 | 462 | 518 | 384 | 302 | 400 |
| | | | | 1082 | 570 | 693 | 539 | 526 | 546 | 497 | 448 | 398 | 484 |
| | | | | 809 | 446 | 513 | 577 | 435 | 496 | 628 | 495 | 395 | 570 |
| | | | | 1235 | 546 | 542 | 431 | 414 | 500 | 508 | 422 | 424 | 383 |
| | | Mean | 460,25 | 766,125 | 557,25 | 568,5 | 521,5 | 449,5 | 501 | 512,75 | 437,25 | 379,25 | 456,5 |
| | | SD | 58,8528803 | 339,304836 | 90,7574607 | 83,8431074 | 62,9364759 | 51,7204022 | 34,5060381 | 13,3010025 | 46,8145542 | 53,2251507 | 98,0850318 |
| | | Specif. Lysis | | | 0,31712301 | 0,35390274 | 0,2002452 | -0,03514508 | 0,13322436 | 0,17163874 | -0,07519412 | -0,26481406 | -0,01225991 |
| Jessy | Transfected | 5 | 225 | 309 | | | | | | | | | |
| | | | 257 | 246 | | | | | | | | | |
| | | | 229 | 404 | | | | | | | | | |
| | | | 242 | 334 | | | | | | | | | |
| | | | 187 | 353 | 253 | 231 | 248 | 216 | 287 | 312 | 248 | 247 | 271 |
| | | | 217 | 280 | 263 | 237 | 268 | 260 | 274 | 293 | 219 | 252 | 289 |
| | | | 216 | 260 | 236 | 278 | 244 | 240 | 292 | 301 | 271 | 244 | 281 |
| | | | 220 | 268 | 249 | 273 | 254 | 253 | 274 | 291 | 250 | 267 | 308 |
| | | Mean | 224,125 | 308,5 | 250,25 | 254,75 | 253,5 | 242,25 | 281,75 | 299,25 | 247 | 252,5 | 287,25 |
| | | SD | 20,4830069 | 54,2428402 | 11,1766125 | 24,1712639 | 10,5039875 | 19,362765 | 8,17877988 | 9,53502318 | 21,3697606 | 10,214369 | 15,6710987 |
| | | Specif. Lysis | | | 0,31714719 | 0,37177542 | 0,35680091 | 0,22003035 | 0,69954476 | 0,91196786 | 0,27769347 | 0,34446131 | 0,76831259 |
| Jessy | Transfected | 7 | 470 | 501 | | | | | | | | | |
| | | | 483 | 599 | | | | | | | | | |
| | | | 460 | 584 | | | | | | | | | |
| | | | 433 | 768 | | | | | | | | | |
| | | | 498 | 539 | 645 | 536 | 546 | 513 | 624 | 577 | 388 | 343 | 401 |
| | | | 517 | 571 | 620 | 588 | 652 | 493 | 571 | 605 | 403 | 407 | 423 |
| | | | 801 | 758 | 585 | 519 | 620 | 482 | 567 | 531 | 423 | 458 | 375 |
| | | | 777 | 1582 | 632 | 595 | 533 | 515 | 607 | 563 | 381 | 398 | 403 |
| | | Mean | 554,625 | 739 | 595,5 | 559 | 587,75 | 500,75 | 592,25 | 569 | 398,75 | 401,5 | 400,5 |
| | | SD | 146,692514 | 358,031124 | 48,9659746 | 37,2111095 | 57,4710072 | 15,9661099 | 27,7773889 | 30,7679487 | 18,5898718 | 47,1062629 | 19,6892526 |
| | | Specif. Lysis | | | 0,22169492 | 0,02372881 | 0,17966102 | -0,28220339 | 0,2040678 | 0,0779661 | -0,84542373 | -0,83050847 | -0,8359322 |

2. CTL-Assay, 10./11.08.2000

| Horse | Cells | ORF | | | Negative Control | Positive Control | Prior to Immunization E:T 3 to 1 | Prior to Immunization E:T 25 to 1 | Prior to Immunization E:T 50 to 1 | After the 2nd Booster E:T 3 to 1 | After the 2nd Booster E:T 25 to 1 | After the 2nd Booster E:T 50 to 1 | After the 3rd Booster E:T 3 to 1 | After the 3rd Booster E:T 25 to 1 | After the 3rd Booster E:T 50 to 1 |
|---|---|---|---|---|---|---|---|---|---|---|---|---|---|---|---|
| Friedrich | Transfected | 2 | | 248 | 2183 | | | | | | | | | | |
| | | | | 259 | 2704 | | | | | | | | | | |
| | | | | 229 | 1633 | | | | | | | | | | |
| | | | | 248 | 2325 | | | | | | | | | | |
| | | | | 220 | 657 | 315 | 327 | 426 | 245 | 299 | 247 | 318 | 226 | 814 | |
| | | | | 314 | 667 | 312 | 274 | 291 | 281 | 508 | 321 | 371 | 315 | 678 | |
| | | | | | 633 | 299 | 289 | 273 | 267 | 693 | 267 | 335 | 363 | 749 | |
| | | | | 289 | 830 | 301 | 283 | 282 | 331 | 295 | 279 | 313 | 238 | 953 | |
| | | | Mean | 257,857143 | 1454 | 306,75 | 293,25 | 318 | 281 | 448,75 | 278,5 | 334,25 | 286 | 798,5 | |
| | | | SD | 33,2338605 | 862,108545 | 7,93200269 | 23,3201683 | 72,3740285 | 36,4783041 | 190,816448 | 31,2569992 | 26,2472221 | 64,3894401 | 117,018941 | |
| | | | Specif. Lysis | | | 0,04087543 | 0,02958916 | 0,05028066 | 0,0193479 | 0,15959035 | 0,01725785 | 0,083868 | 0,02352801 | 0,45198853 | |
| Friedrich | Transfected | 5 | | 272 | 830 | | | | | | | | | | |
| | | | | 354 | 716 | | | | | | | | | | |
| | | | | 304 | 607 | | | | | | | | | | |
| | | | | 281 | 875 | | | | | | | | | | |
| | | | | 233 | 687 | 250 | 327 | 271 | 253 | 269 | 253 | 246 | 239 | 239 | |
| | | | | 270 | 632 | 263 | 279 | 255 | 277 | 288 | 346 | 238 | 290 | 238 | |
| | | | | 220 | 712 | 298 | 278 | 269 | 283 | 252 | 320 | 245 | 282 | 258 | |
| | | | | 242 | 739 | 259 | 319 | 269 | 263 | 292 | 343 | 277 | 295 | 286 | |
| | | | Mean | 272 | 699,75 | 267 | 300,75 | 266 | 269 | 275,25 | 315,5 | 251 | 276,5 | 255,25 | |
| | | | SD | 43,0249097 | 68,5435211 | 20,0831604 | 25,8020591 | 7,393691 | 13,56466 | 18,4639288 | 43,2550575 | 17,907168 | 25,5669057 | 22,4703508 | |
| | | | Specif. Lysis | | | -0,01168907 | 0,06721216 | -0,01402888 | -0,00701344 | 0,0075979 | 0,10169492 | -0,0480941 | 0,01052018 | -0,03915839 | |
| Friedrich | Transfected | 7 | | 290 | 575 | | | | | | | | | | |
| | | | | 266 | 658 | | | | | | | | | | |
| | | | | 318 | 675 | | | | | | | | | | |
| | | | | 258 | 517 | | | | | | | | | | |
| | | | | 503 | 989 | 447 | 457 | 680 | 420 | 425 | 451 | 360 | 319 | 412 | |
| | | | | 537 | 1009 | 414 | 460 | 535 | 478 | 500 | 423 | 376 | 380 | 496 | |
| | | | | 389 | 1058 | 403 | 383 | 465 | 452 | 505 | 435 | 411 | 419 | 355 | |
| | | | | 424 | 999 | 436 | 331 | 590 | 356 | 366 | 438 | 320 | 346 | 336 | |
| | | | Mean | 375,375 | 811,25 | 425 | 407,75 | 642,6 | 426,5 | 449 | 436,75 | 366,75 | 361 | 390,75 | |
| | | | SD | 105,247922 | 225,178501 | 20,0831604 | 62,3397947 | 58,9356382 | 52,6486207 | 66,3375208 | 11,5 | 37,7480684 | 42,2453153 | 71,8346801 | |
| | | | Specif. Lysis | | | 0,11385145 | 0,07427588 | 0,38342415 | 0,1172828 | 0,16891311 | 0,14080672 | -0,01878778 | -0,03287864 | 0,055922 | |

Table 18 c)

Table 18d)

2. CTL-Assay, 10./11.08.2000

| Horse | Cells | ORF | Negative Control | Positive Control | Prior to Immunization E:T 3 to 1 | Prior to Immunization E:T 25 to 1 | Prior to Immunization E:T 50 to 1 | After the 2nd Booster E:T 3 to 1 | After the 2nd Booster E:T 25 to 1 | After the 2nd Booster E:T 50 to 1 | After the 3rd Booster E:T 3 to 1 | After the 3rd Booster E:T 25 to 1 | After the 3rd Booster E:T 50 to 1 |
|---|---|---|---|---|---|---|---|---|---|---|---|---|---|
| Nelke | Transfected | 2b | 674 | 3272 | 837 | 683 | 644 | 825 | 826 | 738 | 480 | 628 | 584 |
| | | | | 2553 | 878 | 646 | 648 | 773 | 819 | 748 | 552 | 565 | 711 |
| | | | | 2264 | 898 | 715 | 708 | 903 | 888 | 693 | 604 | 611 | 666 |
| | | | | 2928 | 844 | 737 | 683 | 758 | 858 | 696 | 596 | 590 | 530 |
| | | Mean | 674 | 2754,25 | 914,25 | 695,25 | 670,25 | 814,75 | 867,75 | 718,75 | 558 | 598,5 | 622,75 |
| | | SD | #DIV/0! | 439,355873 | 71,1354342 | 39,6178673 | 29,5769903 | 65,4643669 | 38,5778434 | 28,3240181 | 58,8037557 | 27,2090671 | 81,1639288 |
| | | Specif. Lysis | | | 0,02140673 | -0,08506715 | -0,10836325 | -0,03151177 | -0,00332403 | -0,08256881 | -0,16806276 | -0,14652307 | -0,13382585 |
| Nelke | Transfected | 5 | 538 | 975 | 606 | 470 | 509 | 476 | 505 | 531 | 392 | 450 | 473 |
| | | | 528 | 921 | 491 | 520 | 455 | 495 | 520 | 469 | 429 | 554 | 551 |
| | | | 640 | 933 | 503 | 479 | 573 | 503 | 480 | 483 | 432 | 475 | 503 |
| | | | 592 | 997 | 523 | 477 | 510 | 494 | 499 | 509 | 459 | 483 | 443 |
| | | Mean | 549,5 | 956,5 | 505,75 | 486,5 | 511,75 | 492 | 501 | 483 | 428 | 492,75 | 492,5 |
| | | SD | 28,8155051 | 35,5668385 | 13,2003788 | 22,6642156 | 48,2450343 | 11,4017543 | 16,5529454 | 32,5371582 | 27,5317998 | 42,0347079 | 46,0543158 |
| | | Specif. Lysis | | | -0,10749388 | -0,15479115 | -0,09275184 | -0,14127764 | -0,11916462 | -0,13882064 | -0,2985258 | -0,13943469 | -0,14004914 |
| Nelke | Transfected | 7 | 399 | 1110 | 406 | 502 | 439 | 349 | 410 | 385 | 523 | 553 | 558 |
| | | | 528 | 822 | 514 | 455 | 432 | 323 | 482 | 524 | 538 | 530 | 627 |
| | | | 441 | 992 | 606 | 487 | 433 | 384 | 355 | 581 | 466 | 544 | 515 |
| | | | 378 | 954 | 632 | 513 | 428 | 451 | 352 | 540 | 649 | 498 | 569 |
| | | Mean | 436 | 969,5 | 539,5 | 489,25 | 433 | 376,75 | 399,75 | 507,5 | 544 | 531,25 | 587,25 |
| | | SD | 65,4675482 | 118,863369 | 102,389778 | 25,1975528 | 4,54606057 | 55,4519311 | 60,9719881 | 85,1214818 | 76,563899 | 24,1022129 | 46,1474087 |
| | | Specif. Lysis | | | 0,19400187 | 0,09981256 | -0,00562324 | -0,11105904 | -0,06794752 | 0,13402062 | 0,20243674 | 0,17853796 | 0,24601887 |

2. CTL-Assay, 10./11.08.2000

| Horse | Cells | ORF | | Negative Control | Positive Control | Prior to Immunization E:T 3 to 1 | Prior to Immunization E:T 25 to 1 | Prior to Immunization E:T 50 to 1 | After the 2nd Booster E:T 3 to 1 | After the 2nd Booster E:T 25 to 1 | After the 2nd Booster E:T 50 to 1 | After the 3rd Booster E:T 3 to 1 | After the 3rd Booster E:T 25 to 1 | After the 3rd Booster E:T 50 to 1 |
|---|---|---|---|---|---|---|---|---|---|---|---|---|---|---|
| Frieda | Transfected | 2b | | 121 | 239 | | | | | | | | | |
| | | | | 144 | 227 | | | | | | | | | |
| | | | | 140 | 222 | | | | | | | | | |
| | | | | 153 | 227 | | | | | | | | | |
| | | | | 207 | 296 | 118 | 137 | 152 | 130 | 126 | 132 | 131 | 163 | 145 |
| | | | | 122 | 212 | 122 | 148 | 137 | 130 | 145 | 145 | | 140 | 150 |
| | | | | 121 | 228 | 139 | 158 | 130 | 134 | 145 | 143 | 136 | 127 | 127 |
| | | | | 172 | 261 | 136 | 131 | 141 | 141 | 144 | 143 | 144 | 161 | 133 |
| | | | Mean | 134 | 239 | 128,75 | 143 | 140 | 133,75 | 140 | 140,75 | 137 | 150 | 138,75 |
| | | | SD | 29,9857109 | 27,1503749 | 10,3077641 | 11,1654228 | 9,20144916 | 5,18812747 | 9,34523051 | 5,90903263 | 6,65743852 | 16,5327957 | 10,5948101 |
| | | | Specif. Lysis | | | -0,05 | 0,08571429 | 0,05714286 | -0,00238095 | 0,05714286 | 0,08428571 | 0,02857143 | 0,15238095 | 0,0452381 |
| Frieda | Transfected | 5 | | 90 | 118 | | | | | | | | | |
| | | | | 67 | 107 | | | | | | | | | |
| | | | | 73 | 136 | | | | | | | | | |
| | | | | 84 | 134 | | | | | | | | | |
| | | | | | 137 | 73 | 81 | 92 | 89 | 72 | 80 | 82 | 106 | 101 |
| | | | | 88 | 127 | 84 | 88 | 73 | | 83 | 88 | 76 | 92 | 90 |
| | | | | 81 | 132 | 89 | 84 | 92 | 82 | 85 | 84 | 93 | 101 | 81 |
| | | | | 86 | 123 | 92 | 101 | | 67 | 83 | 99 | 86 | 95 | |
| | | | Mean | 81,2857143 | 126,75 | 84,5 | 88,5 | 85,6666667 | 79,3333333 | 80,75 | 87,75 | 84,25 | 98,5 | 90,6666667 |
| | | | SD | 8,40068024 | 10,3813292 | 8,34685602 | 8,81286938 | 10,9696551 | 11,2398102 | 5,90903263 | 8,18026079 | 7,13559154 | 6,244998 | 10,0166528 |
| | | | Specif. Lysis | | | 0,07069914 | 0,15868028 | 0,0983603 | -0,04294318 | -0,01178319 | 0,14218382 | 0,06520031 | 0,37863315 | 0,20633674 |
| Frieda | Transfected | 7 | | | 208 | | | | | | | | | |
| | | | | 130 | 387 | | | | | | | | | |
| | | | | 130 | 212 | | | | | | | | | |
| | | | | 112 | 208 | | | | | | | | | |
| | | | | 138 | 210 | 128 | 143 | 140 | 140 | 135 | 158 | 120 | 103 | 131 |
| | | | | 148 | 317 | 156 | 154 | 149 | 144 | 146 | 147 | 115 | 133 | 148 |
| | | | | | 308 | 144 | 138 | 145 | 145 | 142 | 151 | 110 | 127 | 143 |
| | | | | | 264 | 128 | 150 | | 153 | | 143 | 111 | 151 | 130 |
| | | | Mean | 131,6 | 281,75 | 139,5 | 146,25 | 144,666667 | 145,5 | 141 | 149,75 | 114 | 128,5 | 138 |
| | | | SD | 13,2211051 | 62,3417908 | 14,4568323 | 7,13559164 | 4,60024976 | 5,44671155 | 6,68770436 | 6,30681369 | 4,54000057 | 10,8242270 | 8,00002014 |
| | | | Specif. Lysis | | | 0,06068919 | 0,11256243 | 0,10039698 | 0,10679885 | 0,07222436 | 0,13945448 | -0,13522858 | -0,02381687 | 0,04917403 |

Table 18 e)

Table 19: Summary of the measured cytotoxic T-lymphocyte activities

| horse | orf | immunization | 1.booster | 2.booster | 3.booster |
|---|---|---|---|---|---|
| Frieda | 2b | ++ | ++ | - | + |
| | 5 | + | ++ | - | +++ |
| | 7 | ++ | + | + | - |
| Daggy | 2b | +++ | - | - | ++++ |
| | 5 | +++ | ++ | - | - |
| | 7 | +++ | - | - | - |
| Nelke | 2b | + | - | - | - |
| | 5 | + | + | - | - |
| | 7 | - | - | - | +++ |
| Friedrich | 2b | - | - | + | +++ |
| | 5 | + | + | + | - |
| | 7 | + | + | - | - |
| Jessy | 2b | + | + | - | - |
| | 5 | - | - | ++++ | +++ |
| | 7 | ++ | +++ | - | - |

$$x = \frac{x_1(3:1) + x_2(25:1) + x_3(50:1)}{3} = \text{after immunization}$$

$$y = \frac{y_1(3:1) + y_2(25:1) + y_3(50:1)}{3} = \text{before immunization}$$

**x – y = absolute increase of the specific lysis in percent**

x:

- \- = 0%
- \+ = >0-5%
- ++ = >5-10%
- +++ = >10-20%
- ++++ = >20-30%

Table 20: Summary of the measured cytotoxic T-lymphocyte activities

| horse | orf | immunization | 1.booster | 2.booster | 3.booster |
|---|---|---|---|---|---|
| Frieda | 2b | + | + | - | - |
| | 5 | - | + | - | ++ |
| | 7 | + | - | - | - |

(continued)

| horse | orf | immunization | 1.booster | 2.booster | 3.booster |
|---|---|---|---|---|---|
| Daggy | 2b | ++ | - | - | +++ |
| | 5 | ++ | + | - | . - |
| | 7 | ++ | - | - | - |
| Nelke | 2b | - | - | - | - |
| | 5 | - | - | - | - |
| | 7 | - | - | - | ++ |
| Friedrich | 2b | - | - | - | ++ |
| | 5 | - | - | - | - |
| | 7 | - | - | - | - |
| Jessy | 2b | - | - | - | - |
| | 5 | - | - | +++ | ++ |
| | 7 | + | ++ | - | - |

$$x = \frac{x_1(3:1) + x_2(25:1) + x_3(50:1)}{3} = \text{after immunization}$$

$$y = \frac{y_1(3:1) + y_2(25:1) + y_3(50:1)}{3} = \text{before immunization}$$

**x – y = absolute increase of the specific lysis in percent**

**X:**

- \- = **0-5%**
- \+ = **>5-10%**
- ++ = **>10-20%**
- +++ = **>20-30%**

**References**

Balasuriya, U. B. R., Hedges, J. F., Nadler, S. A., McCollum, W.H., Timoney, P. J. and MacLachlan, N. J. (1999) Genetic stability of equine arteritis virus during horizontal and vertical transmission in an outbreak of equine viral arteritis. J. Gen. Virol. 80, 1949-1958.

Balasuriya, U. B. R., MacLachlan N. J., DeVries, A. A. F., Rossitto, P. V., and Rottier, P. J. M. (1995). Identification of a neutralization site in the major envelope glycoprotein (GL) of equine arteritis virus. Virology 207, 518-527.

Balasuriya, U.B., Patton, J.F., Rossitto, P.V., Timoney, P.J., McCollum, W.H., and MacLachlan, N.J.(1997). Neutralization determinants of laboratory strains and field isolates of equine arteritis virus: identification of four neutralization sites in the amino-terminal ectodomain of the G(L) envelope glycoprotein. Virology 232, 114-128.

Barry M.A. and Johnston S.A (1997). Biological features of genetic immunization. Vaccine 15, 788-795.

Bradford, M. (1976). A rapid and sensitive method for the quantitation of microgram quantities of proteins utilizing the principle of protein dye binding. Anal. Biochem. 72, 248-254.
den Boon, J.A., Snijder, E.J., Chimside, E.D., de Vries, A.A., Horzinek, M.C., and Spaan, W.J.(1991). Equine arteritis virus is not a togavirus but belongs to the coronaviruslike superfamily. J Virol 65, 2910-2920.
Gliȿin, V., Crkvenjakov, R., and Byus, C. (1974). Ribonucleic acid isolated by cesium chloride centrifugation. Biochemistry 13, 2633-2637.
Hedges, J.F., Balasuriya, U.B.R., Timoney, P.J., McCollum, W.H., and McLachlan, N.J. (1999). Genetic divergence with emergence of novel phenotypic variants of equine arteritis virus during persistent infection of stallions. J Virol 73, 3672-3681.
Pasquini, S., Xiang, Z., Wang, Y.,.He, Z., Deng, H., Blaszczyk-Thurin, M., and Ert1, H.C. (1997) Cytokines and costimulatory molecules as genetic adjuvants. Immunol. Cel.l Biol. 75, 397-401.
Patton, J.F., Balasuriya, U.B.R., Hedges, J.F., Schweidler, T.M., Hullinger, P.J., and MacLachlan, N.J. (1999). Phylogentic characterization of a highly attenuated strain of equine ateritis virus from the semen of a persistently infected standardbred stallion. Arch Virol 144, 817-827.
Pedersen, K.W., Van Der Meer, Y. Roos, N., and Snijder, E. (1999). Open reding frame 1a-encoded subunit of the arterivirus replicase induce endoplasmic reticulum-derived double-membrane vesicles which carry the viral replication complex. J Virol 73, 2016-2026.
Pirzadeh, B., and Dea, S. (1998). Immune response in pigs vaccinated with plasmid DNA encoding ORF5 of porcine reproductive and respiratory syndrome virus. J. Gen. Virol. 79, 989-999.
Pycock, J.F. (1998). Equine viral arteritis risk from imported semen. The Veterinary Record, 26, 699.
Rosen-Wolff, A., Ben-Hur, T., Becker, Y., and Darai, G. (1988). Comparative analysis of the transcripts mapped in the BamHI DNA fragment B of avirulent HSV-1 HFEM, virulent HSV-1 F, and their intratypic recombinant viruses. Virus Res. 10, 315-324.
Rosen-Wolff, A., Ben-Hur, T., Becker, Y., and Darai, G. (1988) Comparative analysis of the transcripts mapped in the BamHI DNA fragment B of avirulent HSV-1 HFEM, virulent HSV-1 F, and their intratypic recombinant viruses. Virus Res. 10, 315-324.
Rosen-Wolff, A., Scholz, J. and Darai, G. (1989) Organotropism of latent herpes simplex virus type 1 is correlated to the presence of a 1.5 kb RNA transcript mapped within the BamHI DNA fragment B (0.738 to 0.809 map units). Virus Res. 12,43-52.
Rosen-Wolff, A. and Darai, G. (1991) Identification and mapping of the UL56 gene transcript of herpes simplex virus type 1. Virus Res. 19, 115-126.
Stadejek, T., Björklund, H., Ros Bascunana, C., Ciabatti, I.M., Scicluna, M.T., Amaddeo, D., McCollum, W.H., Autorino, G.L., Timoney, P.J. Paton, D. J., Klingebom, B., and Belak, S. (1999). Genetic diversity of equine ateritis virus. J. Gen. Virol. 80, 691-699.
Snijder, E., Van Tol, H., Pedersen, K.W., Raamsman, M.J.B., and De Vries, A.A.F. (1999). Idenification of a novel structural protein of arteriviruses. J Virol 73, 6335-6345.
Thompson, J. D., Gibson, T. J., Plewniak, F., Jeanmougin, F., and Higgins, D. G. (1997). The CLUSTAL_X windows interface: flexible strategies for multiple sequence alignment aided by quality analysis tools. Nucleic Acids Res. 25, 4876-4882.
van der Meer, Y., Van Tol, H. Locker J.K., and Snijder, E. (1998). DORF1a-encoded replicase subunits are involved in the membrane association of the arterivirus replication complex. Journal of Virology 72, 6689-6698.
van Dinten, L.C., Rensen, S., Gorbalenya, A. E. and Snijder, E.J. (1999). Proteolytic processing of the open reading frame 1b-encoded part of arterivirus replicase is mediated by nsp4 Serine protease and is essential for virus replication. J Virol 73, 2027-2037.
Will, H., Cattaneo, R., Koch, H.G., Darai, G., Schaller, H., Schellekens H., van Eerd, P.M.C.A., and Deinhardt, F. (1982). Cloned HBV DNA causes hepatitis in a chimpanzee Nature 299, 740-742.
Will, H., Cattaneo, R., Darai, G., Deinhardt, F., Schellekens H., and Schaller, H. (1985). Infectious hepatitis B virus from cloned DNA of known nucleotide sequence, Proc. Natn. Acad. Sci. U.S.A. 82, 891-895.

## Claims

1. Vaccine composition which is protective against equine arterivirus (EAV) infections in horses and induces a cellular immune response consisting of nucleic acids encoding open reading frame (ORF) 2b, ORF 5 and ORF7 of EAV, said nucleic acids being contained in a single vector backbone or in a plurality of vector backbones, each of which comprises regulatory sequences, wherein ORF2b is the nucleotide sequence set forth in SEQ ID No. 2 or a functional variant thereof wherein said variant carries nucleic acid exchanges, deletions or insertions which amount for up to 10% of the nucleotide acids of the nucleotide acid sequence set forth in SEQ ID No 2.

2. Vaccine composition according to claim 1, wherein said vaccine composition further comprises a single nucleic acid encoding an EAV ORF selected from the group of ORF la, ORF 1b, ORF 3, ORF 4, ORF 6.

3. Vaccine composition according to any one of claims 1 to 2, wherein said nucleic acid is cDNA.

4. Vaccine composition according to claim 1 to 3, wherein said vector(s) is/are expression vector(s).

5. Vaccine composition according to claim 4, wherein said expression vector(s) further comprises(s) a eukaryotic cis-acting transcription/translation sequence functionally linked to said ORF(s).

6. Vaccine composition according to claim 5, wherein said expression vector(s) are selected from the group ofpCR3.1, pcDNA3.1/His A, pcDNA3.1/His B, pcDNA3.1/His C, and pDisplay (pD).

7. Vaccine composition according to any one of claims 1 to 6, further comprising a nucleic acid encoding equine interleukin 2 (IL-2) or a vector or expression vector comprising said nucleic acid encoding IL-2.

8. Vaccine composition according to any one of claims 1 to 7 further comprising a pharmaceutically acceptable carrier or excipient.

9. Vaccine composition according to any one of claims 1 to 8, further comprising one or several adjuvants selected from the group of Muramyl Dipeptide (MDP), Montanide 720, Poly Inosine:Cytosine (Poly I:C) or plasmid DNA comprising unmethylated cytosine, guanine dinucleotide sequence motifs (CpG).

10. Vaccine composition according to claim 1-9, wherein ORF 2b is SEQ ID No. 2, ORF 5 is SEQ ID No. 5 or SEQ ID No. 9 and ORF 7 is SEQ ID No. 7.

11. Vaccine composition according to any one of claims 1 to 10, wherein the nucleic acid vector or expression vector is encapsulated into cationic liposomes.

12. Nucleic acid vector consisting of:

(i) sequences encoding ORF2b, ORF5 and ORF7 of equine arterivirus (EAV), and
(ii) a vector backbone and regulatory sequences, wherein ORF2b is the nucleotide sequence set forth in SED ID No. 2 or a functional variant thereof wherein said variant carries nucleic acid exchanges, deletions or insertions which amount for up to 10% of the nucleotide acids of the nucleotide acid sequence set forth in SED ID No. 2.

13. Nucleic acid vector according to claim 12, wherein said nucleic acid is DNA.

14. Nucleic acid vector according to claim 12 or 13, wherein said nucleic acid vector is an expression vector.

15. Nucleic acid vector according to claim 14, wherein said expression vector further comprises a eukaryotic cis-acting transcription/translation sequence functionally linked to said nucleic acid(s) specific for said ORF(s).

16. Nucleic acid vector according to any one of claims 14 to 15, wherein said expression vector(s) is/are selected from die group of pCR3.1, pcDNA3.1/His A, pcDNA3.1/His B, pcDNA3.1/His C, and pDisplay (pD).

17. Nucleic acid vector according to any one of claims 12 to 16, wherein said nucleic acid vector further comprises nucleic acid SEQ. ID No. 9.

18. Use of one or several nucleic acid vector(s) according to any one of claims 12 to 17 or the compositions according to any of claims 1 to 11 in the manufacture of a vaccine for the prophylaxis and treatment of EAV infections.

19. The use according to claim 18, wherein said one or several nucleic acid vectors and compositions

(i) are to be coated onto carrier particles;
(ii) the coated carrier particles are to be accelerated into epidermal cells of the horse *in vivo* to induce a protective or therapeutic immune response in said horse upon or after exposure to EAV, where the reduction of EAV-associated symptoms or the reduction of horizontal or vertical transmission can be monitored.

20. The use according to claim 19 wherein said carrier particles are gold.

21. The use according to claim 18, wherein said one or several nucleic acid vectors and compositions are to be injected into muscular cells of the horse *in vivo;* and to induce a protective or therapeutic immune response in said horse upon or after exposure to EAV, where the reduction of EAV-associated symptoms or the reduction of horizontal or vertical transmission can be monitored.

## Patentansprüche

1. Vakzine-Zusammensetzung, die gegenüber Equinem Arterivirus (EAV) Infektionen in Pferden protektiv ist, und eine zelluläre Immunantwort induziert, bestehend aus Nukleinsäuren, die für die offenen Leserahmen (ORF) 2b, ORF 5 und ORF 7 von EAV kodieren, wobei die Nukleinsäuren in einem einzelnen Vektor-Rückgrat oder in einer Vielzahl von Vektor-Rückgraten enthalten sind, wobei jedes davon regulatorische Sequenzen umfasst, wobei ORF2B die Nukleotidsequenz ist, die in SEQ ID No. 2 dargelegt ist, oder eine funktionale Variante davon, wobei die Varianten Nukleinsäureaustausche, Deletionen oder Insertionen tragen, die bis zu 10% der Nukleinsäuren der Nukleinsäuresequenz ausmachen, die in SEQ ID No. 2 dargelegt ist.

**2.** Vakzine-Zusammensetzung gemäß Anspruch 1, wobei die Vakzine-Zusammensetzung ferner eine einzelne Nukleinsäure umfasst, die für einen EAV ORF kodiert, ausgewählt aus der Gruppe aus ORF 1a, ORF 1b, ORF 3, ORF 4, ORF 6.

**3.** Vakzine-Zusammensetzung gemäß jedem der Ansprüche 1 bis 2, wobei die Nukleinsäure cDNA ist.

**4.** Vakzine-Zusammensetzung gemäß den Ansprüchen 1 bis 3, wobei der/die Vektor/Vektoren (ein) Expressionsvektor (en) ist/sind.

**5.** Vakzine-Zusammensetzung gemäß Anspruch 4, wobei der/die Expressionvektor(en) weiter eine eukaryotische cis-wirkende Transkriptions-/Translationssequenz umfasst/umfassen, die funktional mit dem/den ORF(s) verknüpft ist.

**6.** Vakzine-Zusammensetzung gemäß Anspruch 5, wobei der/die Expressionsvektor(en) ausgewählt sind aus der Gruppe aus pCR3.1, pcDNA3.1/His A, pcDNA3.1/His B, pcDNA3.1/His C und pDisplay (pD).

**7.** Vakzine-Zusammensetzung gemäß jedem der Ansprüche 1 bis 6, ferner eine Nukleinsäure, die für ein equines Interleukin 2 (IL-2) kodiert, oder einen Vektor oder Expressionsvektor umfassend, der die Nukleinsäure, die für IL-2 kodiert, umfasst.

**8.** Vakzine-Zusammensetzung gemäß jedem der Ansprüche 1 bis 6, ferner einen pharmazeutisch verträglichen Träger oder Hilfsstoff umfassend.

**9.** Vakzine-Zusammensetzung gemäß jedem der Ansprüche 1 bis 8, ferner eines oder mehrere Adjuvanzien umfassend, ausgewählt aus der Gruppe aus Muramyl-dipeptid (MDP), Montanide 720, Poly Inosin:Cytosin (Poly I:C) oder Plasmid DNA umfassend unmethyliertes Cytosin, Guanin-dinukleotid Sequenzmotive (CpG).

**10.** Vakzine-Zusammensetzung gemäß den Ansprüchen 1-9, wobei ORF 2b SEQ ID No. 2, ORF 5 SEQ ID No. 5 oder SEQ ID No. 9 ist und ORF 7 SEQ ID No. 7 ist.

**11.** Vakzine-Zusammensetzung gemäß jedem der Ansprüche 1 bis 10, wobei der Nukleinsäurevektor oder Expressionsvektor in kationische Liposomen eingekapselt ist.

**12.** Nukleinsäurevektor bestehend aus:

(i) Sequenzen, kodierend für ORF 2b, ORF 5 und ORF 7 von Equinem Arterivirus (EAV), und
(ii) einem Vektor-Rückgrat und regulatorischen Sequenzen, wobei ORF 2b die Nukleotidsequenz ist, die in SEQ ID No. 2 dargelegt ist, oder eine funktionale Variante davon, wobei die Varianten Nukleinsäureaustausche, Deletionen oder Insertionen tragen, die bis zu 10% der Nukleinsäuren der Nukleinsäuresequenz ausmachen, die in SEQ ID No. 2 dargelegt ist.

**13.** Nukleinsäurevektor gemäß Anspruch 12, wobei die Nukleinsäure DNA ist.

**14.** Nukleinsäurevektor gemäß Ansprüchen 12 oder 13, wobei der Nukleinsäurevektor ein Expressionsvektor ist.

**15.** Nukleinsäurevektor gemäß Anspruch 14, wobei der Expressionsvektor weiter eine eukaryotische cis-wirkende Transkriptions-/Translationssequenz umfasst, die funktional mit dem/den ORF(s) verknüpft ist.

**16.** Nukleinsäurevektor gemäß jedem der Ansprüche 14 bis 15, wobei der/die Expressionsvektor(en) ausgwählt ist/sind aus der Gruppe aus pCR3.1, pcDNA3.1/His A, pcDNA3.1/His B, pcDNA3.1/His C und pDisplay (pD).

**17.** Nukleinsäurevektor gemäß jedem der Ansprüche 12 bis 16, wobei der Nukleinsäurevektor weiter Nukleinsäure SEQ ID No. 9 umfasst.

**18.** Verwendung von einem oder mehreren Nukeinsäurevektor(en) gemäß jedem der Ansprüche 12 bis 17 oder den Zusammensetzungen gemäß jedem der Ansprüche 1 bis 11 für die Herstellung eines Vakzines für die Prophylaxe und Behandlung von EAV Infektionen.

**19.** Die Verwendung gemäß Anspruch 18, wobei der eine oder mehrere Nukleinsäurevektor(en) und Zusammensetzung (en)

(i) auf Trägerpartikel zu schichten sind;
(ii) die beschichteten Trägerpartikel in Epidermalzellen der *Pferdes in vivo* zu beschleunigen sind, um eine protektive oder therapeutische Immunantwort in dem Pferd auf Exposition gegenüber EAV hin oder nach Exposition gegenüber EAV zu induzieren, wobei die Reduktion von EAV assoziierten Symptomen oder die Reduktion von horizontaler oder vertikaler Übertragung beobachtet werden kann.

**20.** Die Verwendung gemäß Anspruch 19, wobei die Trägerpartikel aus Gold sind.

**21.** Die Verwendung gemäß Anspruch 18, wobei die eine oder mehrere Nukleinsäurevektoren und Zusammensetzungen in muskuläre Zellen des Pferdes *in vivo* zu injizieren sind; und um eine protektive oder therapeutische Immunantwort in dem Pferd auf Exposition gegenüber EAV hin oder nach Exposition gegenüber EAV zu induzieren, wobei die Reduktion von EAV assoziierten Symptomen oder die Reduktion von horizontaler oder vertikaler Übertragung beobachtet werden kann.

**Revendications**

**1.** Composition de vaccin protégeant contre les infections au virus de l'artérite équine (VAE) chez les chevaux et induisant une réponse immunitaire cellulaire consistant en des acides nucléiques codant les cadres ouverts de lecture (ORF) 2b, ORF 5 et ORF 7 du VAE, lesdits acides nucléiques étant contenus dans un squelette de vecteur unique ou dans une pluralité de squelettes de vecteur, chacun d'entre eux comprenant des séquences régulatrices, ORF 2b étant la séquence de nucléotides représentée par la SEQ ID No. 2 ou une de ses variantes fonctionnelles, ladite variante portant des insertions, délétions ou échanges d'acides nucléiques représentant jusqu'à 10 % des acides nucléotidiques de la séquence d'acides nucléotidiques représentée par la SEQ ID No. 2.

**2.** Composition de vaccin selon la revendication 1, ladite composition de vaccin comprenant également un acide nucléique unique codant pour un ORF de VAE choisi parmi le groupe comprenant ORF 1a, ORF 1b, ORF 3, ORF 4, ORF 6.

**3.** Composition de vaccin selon l'une quelconque des revendications 1 à 2, dans laquelle ledit acide nucléique est un ADNc.

**4.** Composition de vaccin selon les revendications 1 à 3, dans laquelle ledit ou lesdits vecteur(s) est/sont un/des vecteur(s) d'expression.

**5.** Composition de vaccin selon la revendication 4, dans laquelle ledit ou lesdits vecteur(s) d'expression comprennent également une séquence eucaryote agissant en cis de transcription/traduction fonctionnellement liée audit ou auxdits ORF.

**6.** Composition de vaccin selon la revendication 5, dans laquelle ledit ou lesdits vecteur(s) d'expression est/sont choisi (s) dans le groupe comprenant pCR3.1, pcDNA3.1/His A, pcDNA3.1/His B, pcDNA3.1/His C et pDisplay (pD).

**7.** Composition de vaccin selon l'une quelconque des revendications 1 à 6, comprenant en outre un acide nucléique codant pour l'interleukine 2 (IL-2) équine ou un vecteur ou un vecteur d'expression comprenant ledit acide nucléique codant pour IL-2.

**8.** Composition de vaccin selon l'une quelconque des revendications 1 à 7, comprenant en outre un excipient ou un véhicule pharmaceutiquement acceptable.

**9.** Composition de vaccin selon l'une quelconque des revendications 1 à 8, comprenant en outre un ou plusieurs adjuvants choisis dans le groupe comprenant le Muramyl Dipeptide (MDP), le Montanide 720, le Poly Inosine:

Cytosine (Poly I:C) ou un ADN plasmidique comprenant des motifs de séquences de dinucléotide guanine-cytosine non méthylée (CpG).

**10.** Composition de vaccin selon les revendications 1 à 9, dans laquelle ORF 2b est représenté par la SEQ ID No. 2, ORF 5 est représenté par la SEQ ID No. 5 ou la SEQ ID No. 9 et ORF 7 est représenté par la SEQ ID No. 7.

**11.** Composition de vaccin selon l'une quelconque des revendications 1 à 10, dans laquelle le vecteur ou le vecteur d'expression de l'acide nucléique est encapsulé dans des liposomes cationiques.

**12.** Vecteur d'acide nucléique consistant en :

(i) des séquences codant pour ORF 2b, ORF 5 et ORF 7 du virus de l'artérite équine (VAE) et
(ii) un squelette de vecteur et des séquences régulatrices, dans lequel ORF 2b est la séquence de nucléotides représentée par la SEQ ID No. 2 ou une de ses variantes fonctionnelles, ladite variante portant des insertions, délétions ou échanges d'acides nucléiques représentant jusqu'à 10 % des acides nucléotidiques de la séquence d'acides nucléotidiques représentée par la SEQ ID No. 2.

**13.** Vecteur d'acide nucléique selon la revendication 12, dans lequel l'acide nucléique est un ADN.

**14.** Vecteur d'acide nucléique selon la revendication 12 ou 13, dans lequel ledit vecteur d'acide nucléique est un vecteur d'expression.

**15.** Vecteur d'acide nucléique selon la revendication 14, dans lequel ledit vecteur d'expression comprend également une séquence eucaryote agissant en cis de transcription/traduction fonctionnellement liée audit ou auxdits acide (s) nucléique(s) spécifique(s) dudit ou desdits ORF.

**16.** Vecteur d'acide nucléique selon l'une quelconque des revendications 14 à 15, dans lequel ledit ou lesdits vecteur (s) d'expression est/sont choisi(s) parmi le groupe comprenant pCR3.1, pcDNA3.1/His A, pcDNA3.1/His B, pcDNA3.1/His C et pDisplay (pD).

**17.** Vecteur d'acide nucléique selon l'une quelconque des revendications 12 à 16, dans lequel ledit vecteur d'acide nucléique comprend également l'acide nucléique de la SEQ. ID No. 9.

**18.** Utilisation d'un ou plusieurs vecteur(s) d'acide nucléique selon l'une quelconque des revendications 12 à 17 ou des compositions selon l'une quelconque des revendications 1 à 11 pour la fabrication d'un vaccin pour la prophylaxie et le traitement d'infections au VAE.

**19.** Utilisation selon la revendication 18, dans laquelle ledit ou lesdits vecteur(s) d'acide nucléique et lesdites compositions

(i) sont destinées à être enduites sur des particules de support ;
(ii) les particules de support enduites sont destinées à être accélérées dans les cellules épidermiques du cheval in *vivo* afin d'induire une réponse immunitaire protectrice ou thérapeutique audit cheval au moment de ou après l'exposition au VAE, la réduction des symptômes associés au VAE ou la réduction de la transmission horizontale ou verticale pouvant être suivie.

**20.** Utilisation selon la revendication 19, dans laquelle lesdites particules de support sont en or.

**21.** Utilisation selon la revendication 18, dans laquelle ledit ou lesdits vecteur(s) d'acide nucléiques et lesdites compositions sont destinés à être injectés dans les cellules musculaires du cheval *in vivo ;* et à induire une réponse immunitaire protectrice ou thérapeutique audit cheval au moment de ou après l'exposition au VAE, la réduction des symptômes associés au VAE ou la réduction de la transmission horizontale ou verticale pouvant être suivie.

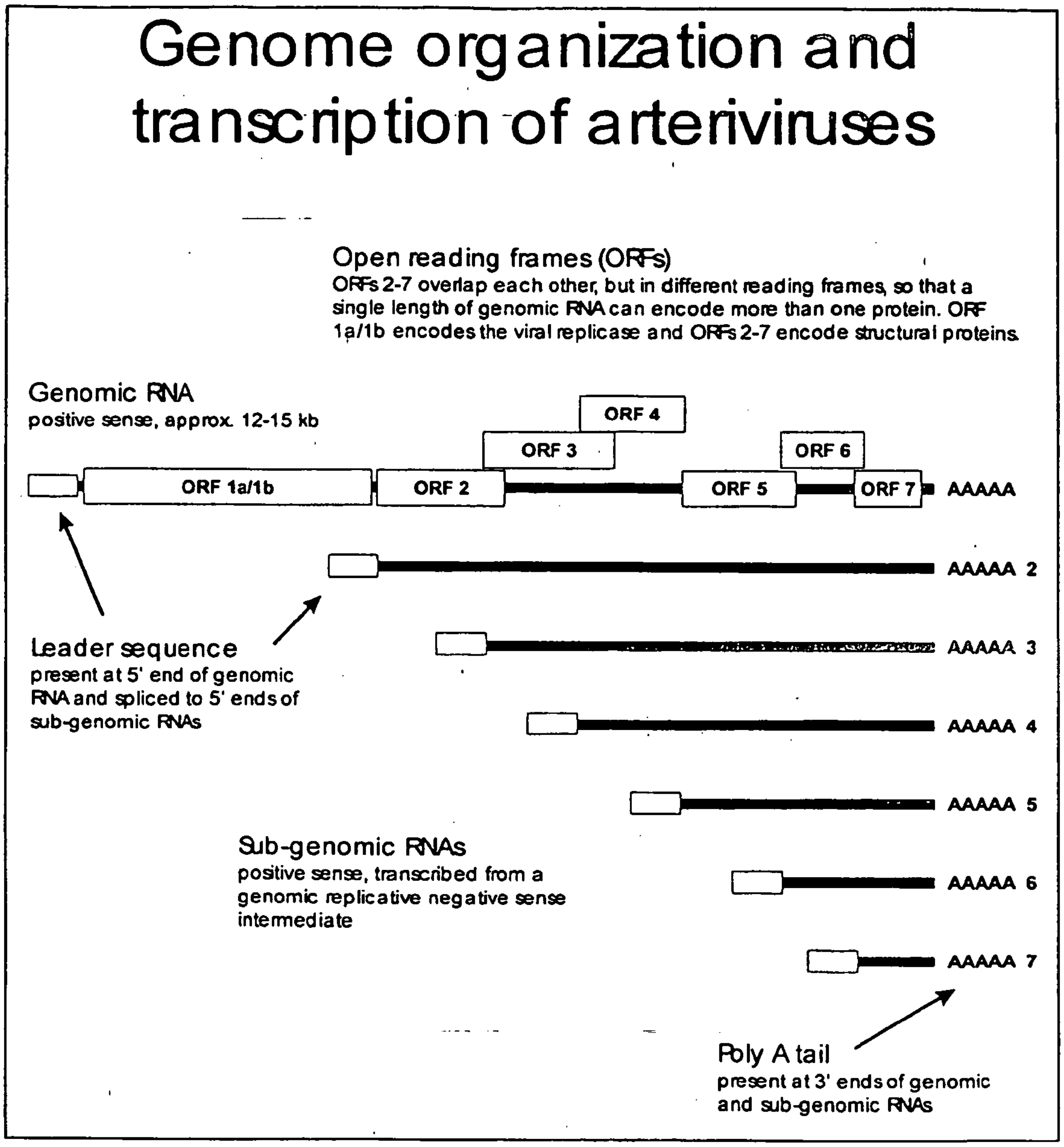


**Fig. 1:**

Schematic diagram of the genomic organization and transcriptional strategy of the family *Arteriviridae*.

# Construction of a vector expressing the neutralization determinant of the viral large glycoprotein

The N-terminal hydrophilic ectodomain (amino acids 1-121) of the G(L) envelope glycoprotein contains the neutralization domains of EAV (Balasuriya et al., 1997, Virology 232, 114-128). The corresponding coding region of the viral ORF 5 (nucleotides 1-363) was inserted into the mammalian expression vector pcDNA3.1/His.

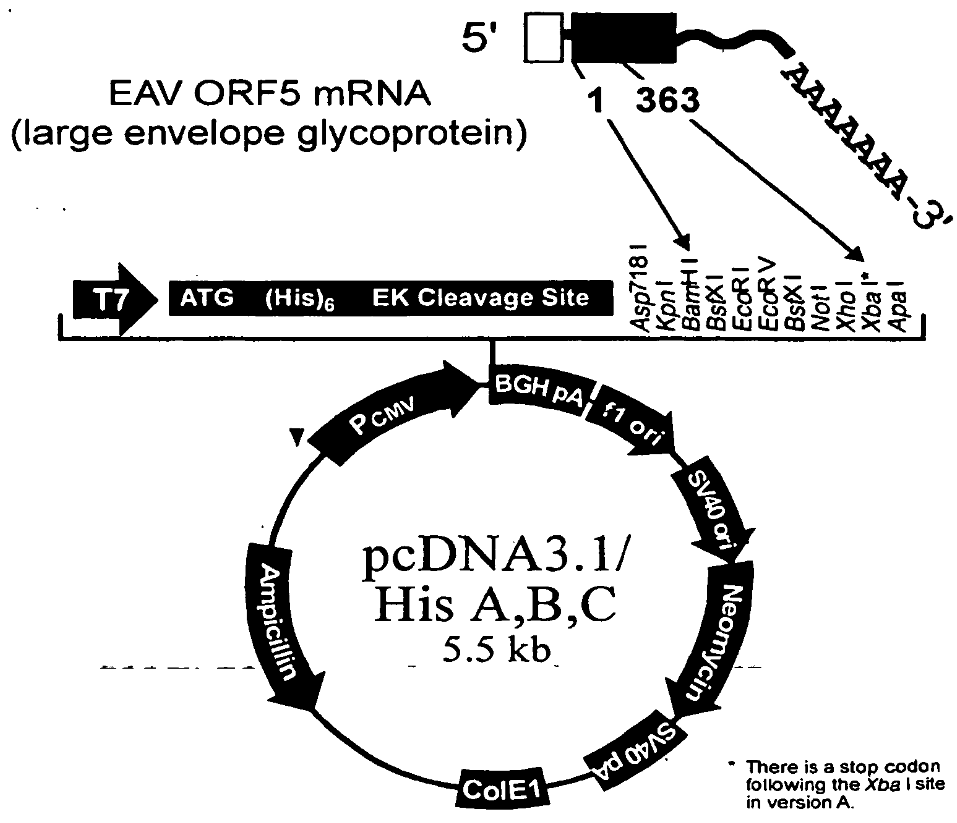


**Fig. 2:**

Schematic diagram of the strategy used for molecular cloning of neutralizing domain of equine arteritis virus (EAV). A part of the cDNA of viral ORF5 expressing the N-terminal hydrophilic ectodomain of the EAV envelope large glycoprotein was inserted into the corresponding sites of mammalian expression vector pcDNA3.1.

**A**

**Diagram presenting the data of DNA immunization of Balb/c mice with recombinant plasmid pCR3.1-EAV-O5-BX-C14 expressing ORF 5 of equine arteritis virus**

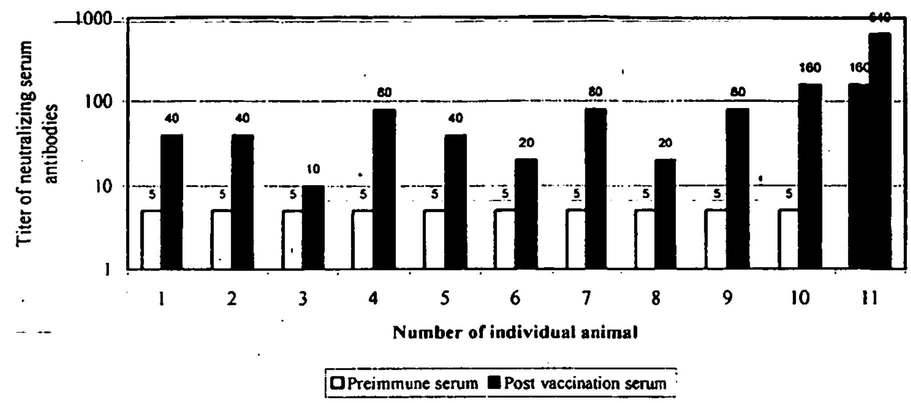


**B**

**Diagram presenting the data of DNA immunization of Balb/c mice with recombinant plasmid pCR3.1-EAV-O5-BX-C14 expressing the ORF 5 of equine arteritis virus**

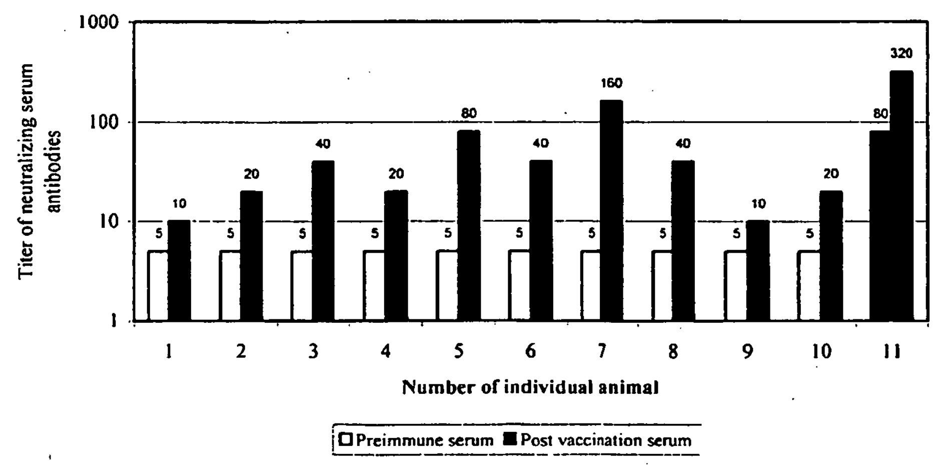


**Fig. 3:**

The results of neutralization tests obtained by the analysis of the sera of the individual Balb/c mice that were inoculated in two independent experiments (A and B) with the DNA of recombinant plasmid pCR3.1-EAV-O5-BX-C14 harboring and expressing ORF 5 of equine arteritis virus (EAV). The individual animals are indicated with number 1 to 10. The white and black columns represent the data of pre and post DNA vaccinated animals, respectively. The column 11 (black color) served as internal positive control and indicates the average of maximum and minimum neutralizing titer obtained from the serum of a New Zealand white rabbit that was immunized with inactivated EAV.

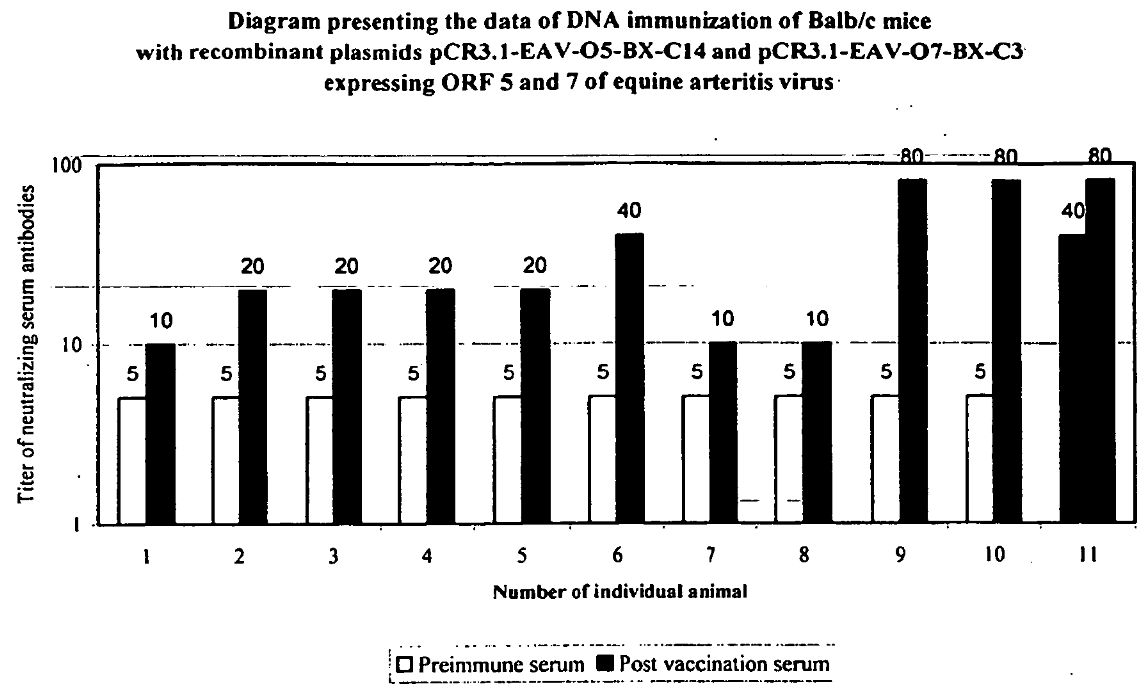


Fig. 4:

The results of neutralization test obtained by the analysis of the sera of the individual Balb/c mice that were inoculated with the DNA of recombinant plasmids pCR3.1-EAV-O5-BX-C14 and pCR3.1-EAV-O7-BX-C3 harboring and expressing ORFs 5 and 7 of equine arteritis virus (EAV). The individual animals are indicated with number 1 to 10. The white and black columns represent the data of pre and post DNA vaccinated animals, respectively. The column 11 (black color) served as internal positive control and indicates the average of maximum and minimum neutralizing titer obtained from the serum of a New Zealand white rabbit that was immunized with inactivated EAV.

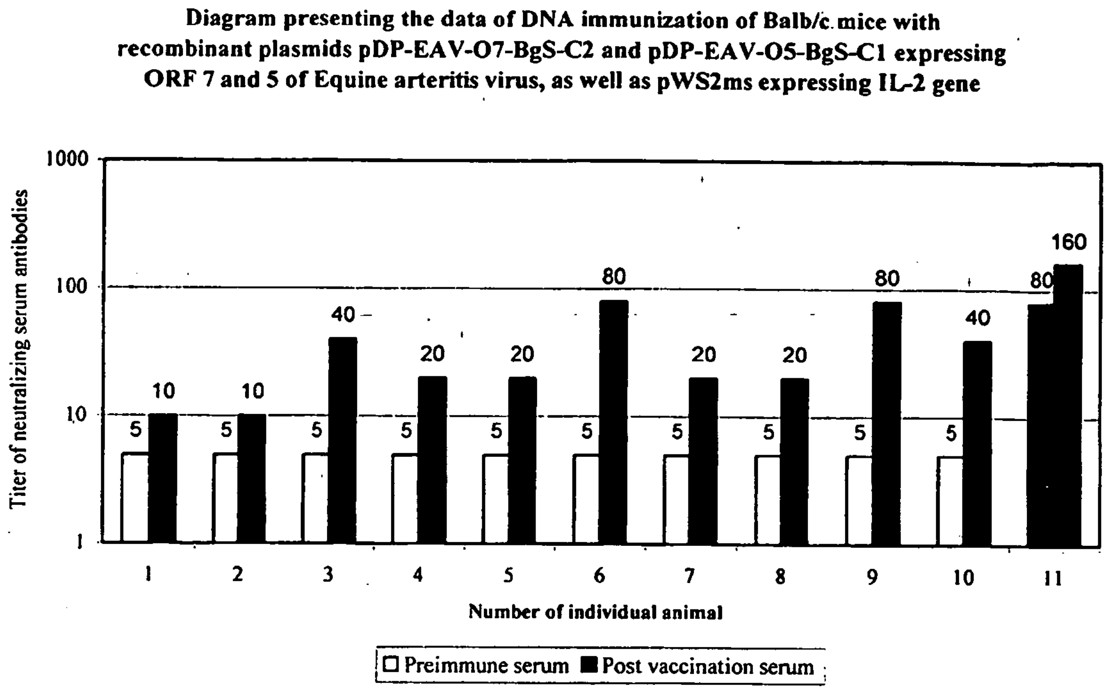


**Fig. 5:**

The results of neutralization test obtained by the analysis of the sera of the individual Balb/c mice that were inoculated with the DNA of recombinant plasmids pDP-EAV-O5-BsS-C2 and pDP-EAV-O7-BsS-C1 harboring and expressing ORFs 5 and 7 of equine arteritis virus (EAV). The recombinant plasmid pWS2ms expressing mouse IL2 gene was administered as immune modulating factor. The individual animals are indicatesd with number 1 to 10. The white and black columns represent the data of pre and post DNA vaccinated animals, respectively. The column 11 (black color) served as internal positive control and indicates the average of maximum and minimum neutralizing titer obtained from the serum of a New Zealand white rabbit that was immunized with inactivated EAV.

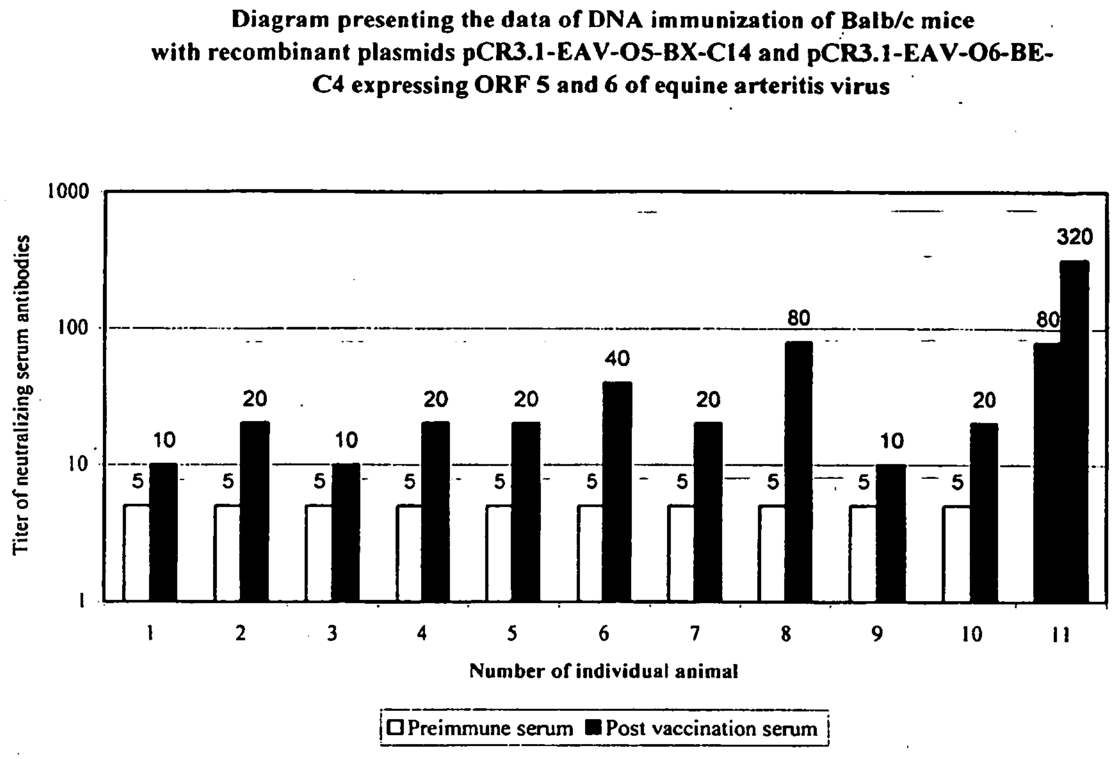


**Fig. 6:**
The results of neutralization test obtained by the analysis of the sera of the individual Balb/c mice that were inoculated with the DNA of recombinant plasmids pCR3.1-EAV-O5-BX-C14 and pCR3.1-EAV-O6-BE-C4 harboring and expressing ORFs 5 and 6 of equine arteritis virus (EAV). The individual animals are indicated with number 1 to 10. The white and black columns represent the data of pre and post DNA vaccinated animals, respectively. The column 11 (black color) served as internal positive control and indicates the average of maximum and minimum neutralizing titer obtained from the serum of a New Zealand white rabbit that was immunized with inactivated EAV.

**Diagram presenting the data of DNA immunization of Balb/c mice with recombinant plasmids pCR3.1-EAV-O3-BX-C1 expressing ORF 3 of equine arteritis virus**

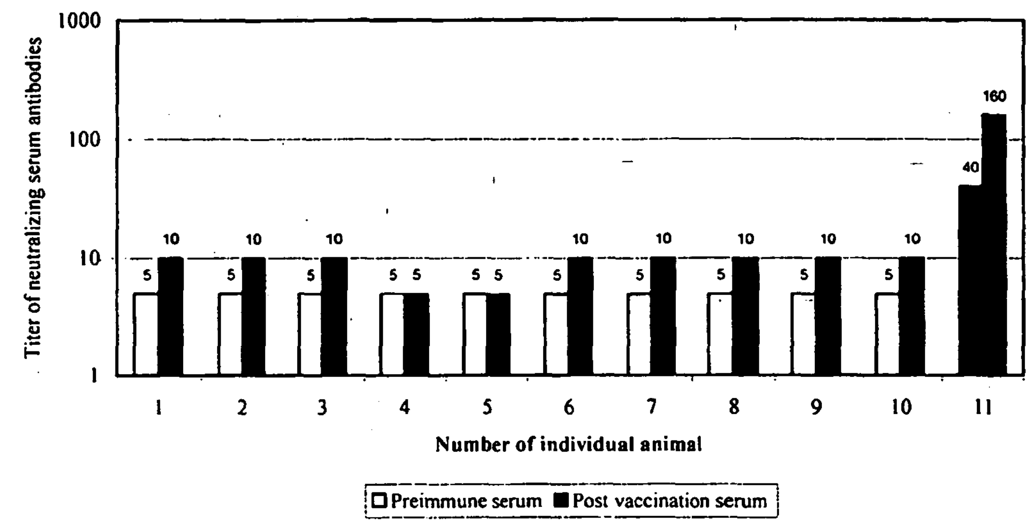


**Fig. 7:**

The results of neutralization test obtained by the analysis of the sera of the individual Balb/c mice that were inoculated with the DNA of recombinant plasmid pCR3.1-EAV-O4-BX-C3 harboring and expressing ORF 4 of equine arteritis virus (EAV). The individual animals are indicated with number 1 to 10. The white and black columns represent the data of pre and post DNA vaccinated animals, respectively. The column 11 (black color) served as internal positive control and indicates the average of maximum and minimum neutralizing titer obtained from the serum of a New Zealand white rabbit that was immunized with inactivated EAV.

**Diagram presenting the data of DNA immunization of Balb/c mice with recombinant plasmids pCR3.1-EAV-O4-BX-C3 expressing ORF 4 of equine arteritis virus**

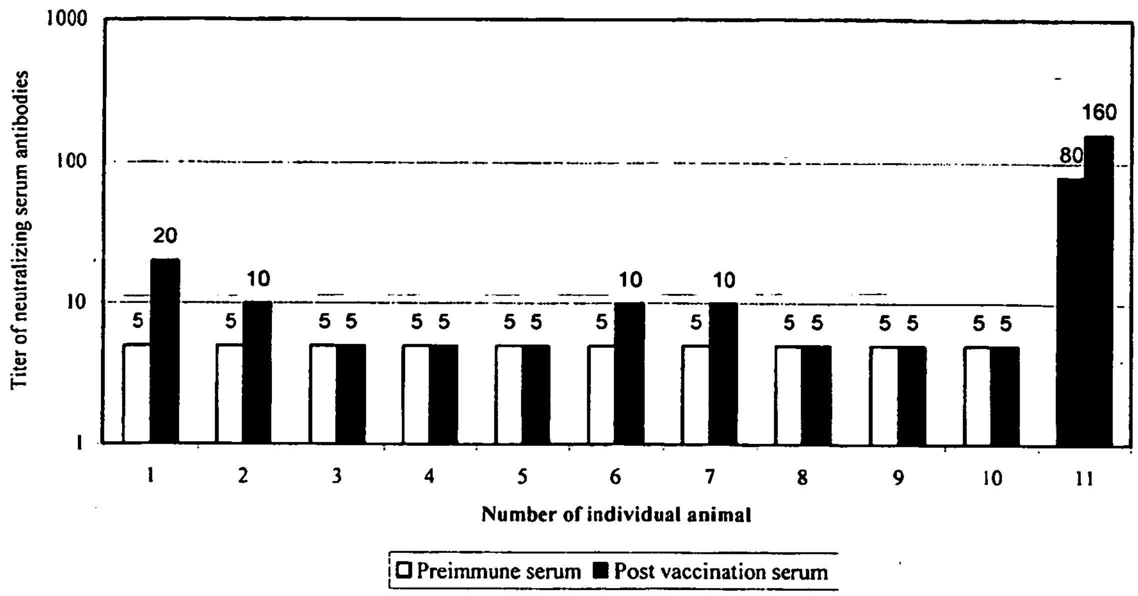


**Fig. 8:**

The results of neutralization test obtained by the analysis of the sera of the individual Balb/c mice that were inoculated with the DNA of recombinant plasmid pCR3.1-EAV-O4-BE-C3 harboring and expressing ORF 4 of equine arteritis virus (EAV). The individual animals are indicated with number 1 to 10. The white and black columns represent the data of pre and post DNA vaccinated animals, respectively. The column 11 (black color) served as internal positive control and indicates the average of maximum and minimum neutralizing titer obtained from the serum of a New Zealand white rabbit that was immunized with inactivated EAV.

**Diagram presenting the data of DNA immunization of Balb/c mice with recombinant plasmid pC3.1-EAV-O5-del-121 expressing the amino terminus (aa 1-121) of ORF 5 of equine arteritis virus**

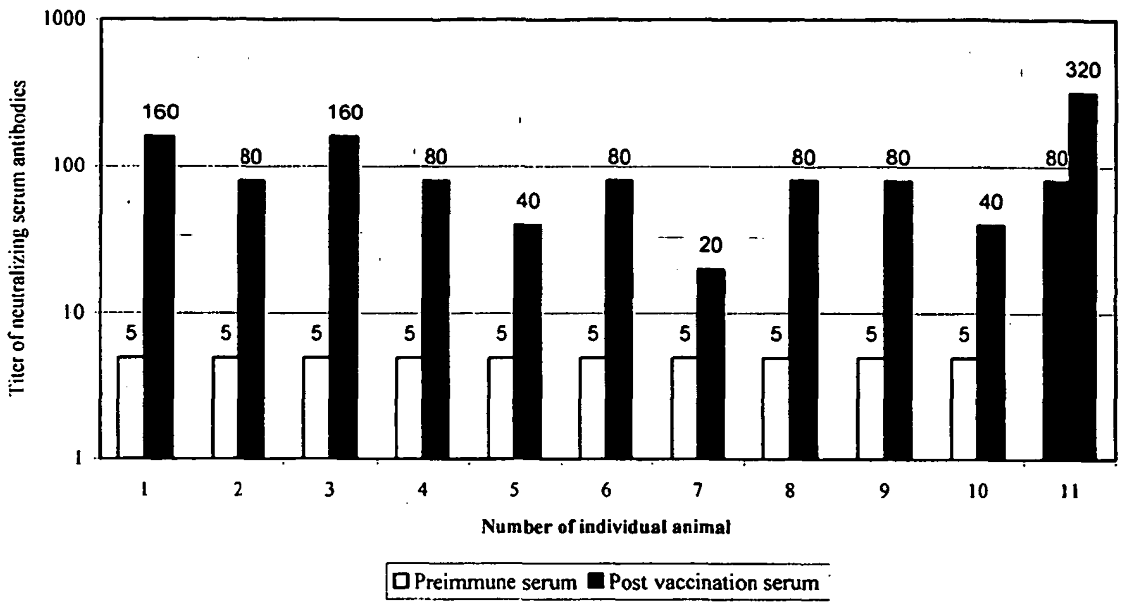


**Fig. 9:**

The results of neutralization tests obtained by the analysis of the sera of the individual Balb/c mice that were inoculated with the DNA of recombinant plasmid pCR31-EAV-O5-del-121 harboring and expressing the N-terminal hydrophilic ectodomain of $G_L$ envelope glycoprotein (amino acid residue 1-121 of ORF 5) of equine arteritis virus (EAV). The individual animals are indicated with number 1 to 10. The white and black columns represent the data of pre and post DNA vaccinated animals, respectively. The column 11 (black color) served as internal positive control and indicates the average of maximum and minimum neutralizing titer obtained from the serum of a New Zealand white rabbit that was immunized with inactivated EAV.

**Diagram presenting the data of DNA immunization of Balb/c mice with recombinant plasmids pCR3.1-EAV-O2-BX-C5, pCR3.1-EAV-O5-BX-C14, and pC31-EAV-BE-O6 expressing ORFs 2b, 5, and 6 of EAV, as well as pWS-2ms-C1 expressing IL-2**

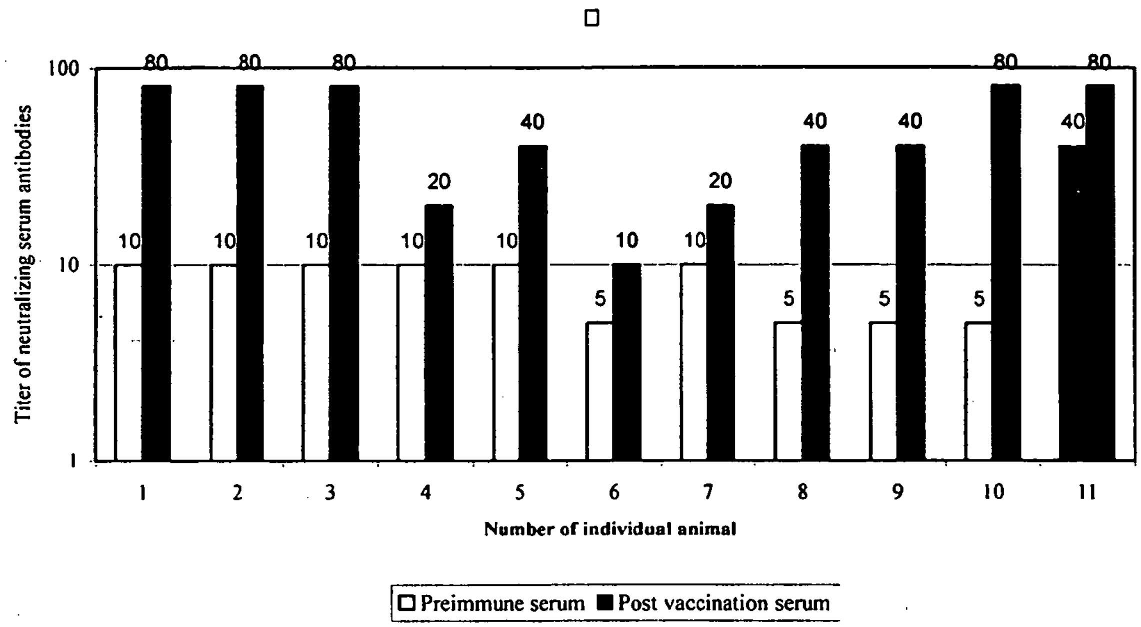


**Fig. 10:**

The results of neutralization test obtained by the analysis of the sera of the individual Balb/c mice that were inoculated with the DNA of recombinant plasmids pCR3.1-EAV-O2-BX-C5, pCR3.1-EAV-O5-BX-C14, and pCR3.1-EAV-O6-BE-C4 harboring and expressing ORFs 2b(small glycoprotein), 5 (large envelope glycoprotein), and 6 (membrane protein), of equine arteritis virus (EAV). The recombinant plasmid pWS2ms (expressing mouse IL2 gene) was administered as immune modulating factor. The individual animals are indicated with number 1 to 10. The white and black columns represent the data of pre and post DNA vaccinated animals, respectively. The column 11 (black color) served as internal positive control and indicates the average of maximum and minimum neutralizing titer obtained from the serum of a New Zealand white rabbit that was immunized with inactivated EAV.

Diagram presenting the data of DNA immunization of Balb/c mice
with recombinant plasmids pCR3.1-EAV-O2-BX-C5 and pCR3.1-EAV-O4-BX-C3
expressing ORF2b and 4 of equine arteritis virus

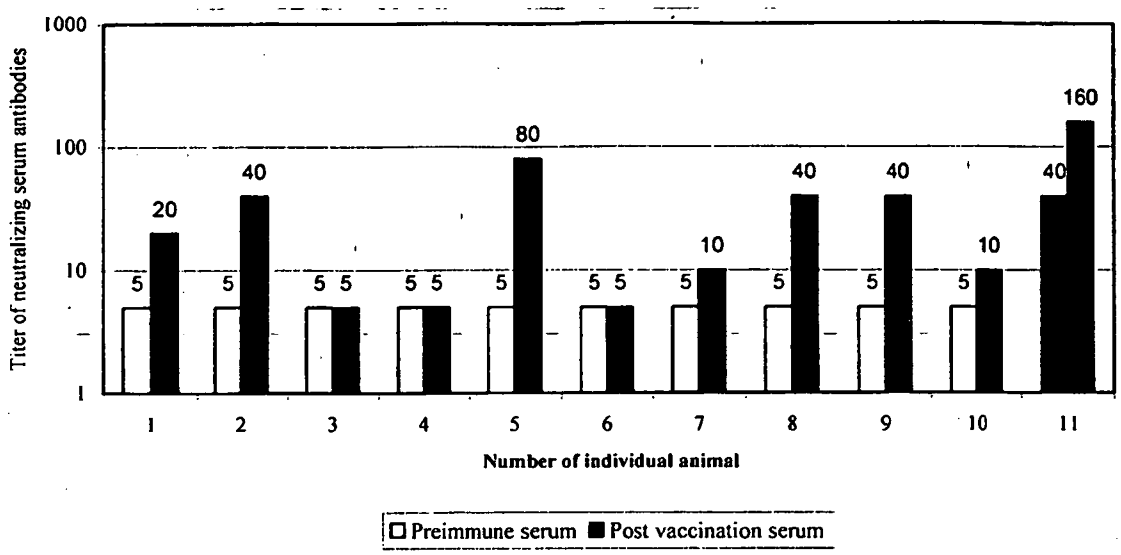


**Fig. 11:**

The results of neutralization test obtained by the analysis of the sera of the individual Balb/c mice that were inoculated with the DNA of recombinant plasmids pCR3.1-EAV-O2-BX-C5 and pCR3.1-EAV-O4-BX-C3 harboring and expressing ORFs 2b and 4 of equine arteritis virus (EAV). The individual animals are indicated with number 1 to 10. The white and black columns represent the data of pre and post DNA vaccinated animals, respectively. The column 11 (black color) served as internal positive control and indicates the average of maximum and minimum neutralizing titer obtained from the serum of a New Zealand white rabbit that was immunized with inactivated EAV.

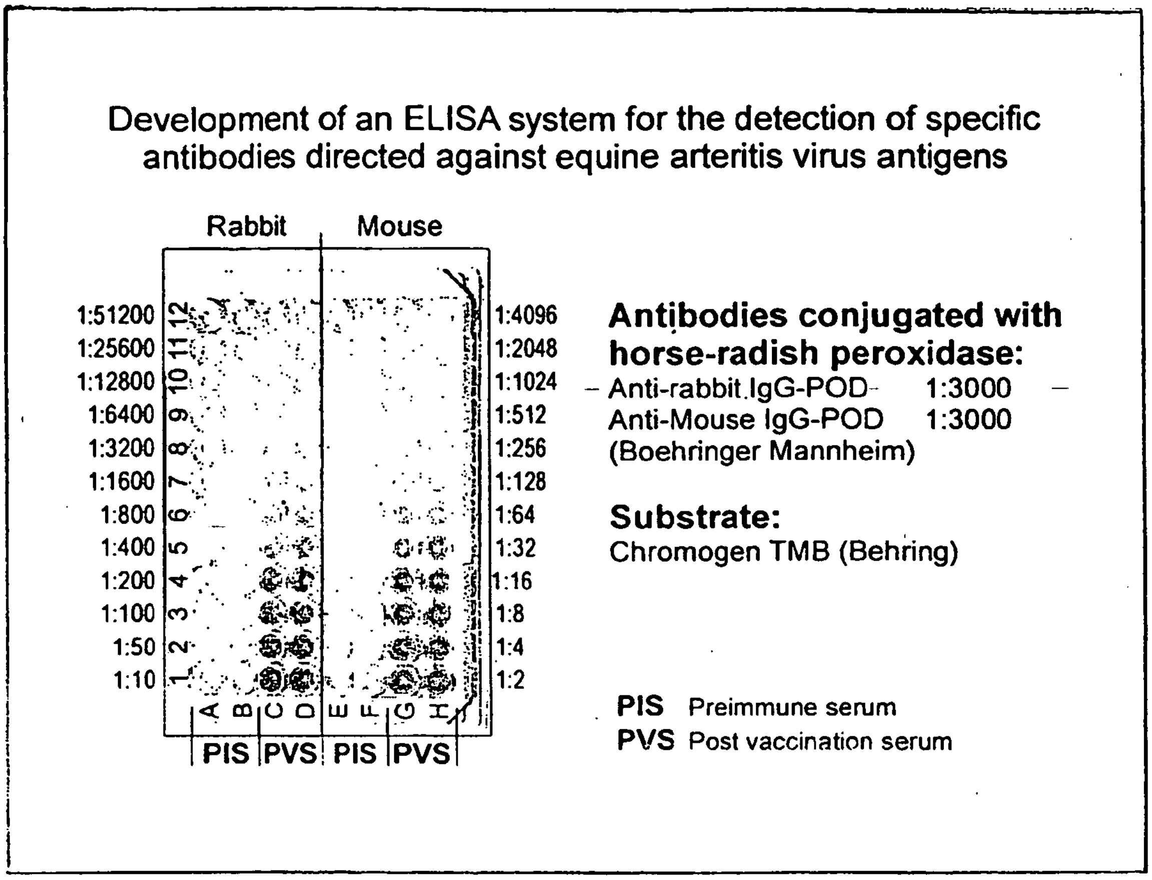


**Fig. 12:**

An example of the results obtained by enzyme linked immunosorbent assay (ELISA) for the detection of EAV specific antibodies. Polysorp F8 microtiter plates were coated with EAV Protein (EAV+ Host (RK13)) at a concentration of 2 µg x $ml^{-1}$ in PBS (+ 0.05% $N_3Na$) over night at room temperature (for detail see session material and methods). The assay was stopped after 30 min by the addition of 50 µl/well stopping solution POD and read according to standard procedures at 450 nm on an automatic ELISA reader (MR5000, DYNATECH, Denkendorf, Deutschland). Photograph was taken prior to the analysis of the assay by 450 nm by ELISA reader.

Establishing of a lymphoproliferation assay for detection of cellular immune response

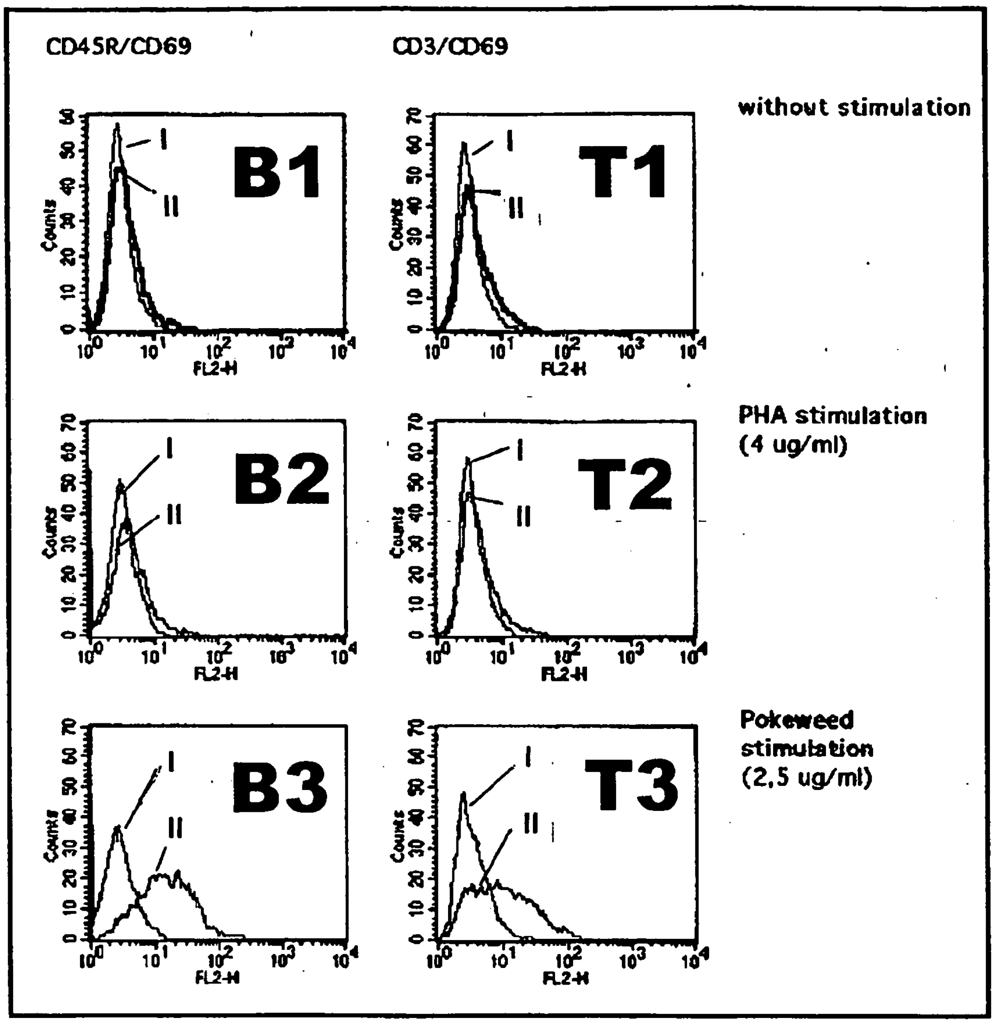


Fig. 13:

Representative FACS profiles of expression of various adhesion molecules on spleen cells from a female BALB/c mouse. B- and T cells were activated for three days with 8 g/ml PHA (Lectin from Phaesolus vulgaris Sigma, Cat.No.L-8754) (Fig. T2 and B2) or 2.5 g/ml Pokeweed (Lection from Phytolacca Americana, Sigma Cat.No.L-9379) (Fig.T3 and B3) and only FACS buffer (PBS, 2% FCS, 0,01% $NaN_3$) as negative control (Fig.T1 and B1) respectively. B cells were stained for single fluorescence with anti-mouse CD45R/ B220 (diluted 1:100 FACS buffer, RA3-6B2, PharMingen Cat.No.01124A, FITC) (Fig. B1, B2 and B3, lane I) and for double fluorescence with anti-mouse CD69 (diluted 1:100 in FACS buffer, H1.2F3, PharMingen Cat.No01505B, PE) and anti-mouse CD45R/ 220 (Fig.B1, B2, and B3, lane II, showing activated B cells.) T cells were stained for single fluorescence anti-mouse CD3 (diluted 1:100 in FACS buffer, 145-2C11, PharMingen Cat.No.01088A, Cy-Chrome) (Fig.T1, T2, and T3, lane I) and for double fluorescence with anti-mouse CD69 (diluted 1:100 in FACS buffer, H1.2F3, PharMingen Cat.No.01505B, PE) and anti-mouse CD3. Cells were sorted and measured in FACScan (Becton Dickinson) (Fig.T1, T2, and T3, lane II, showing the activated T cells).

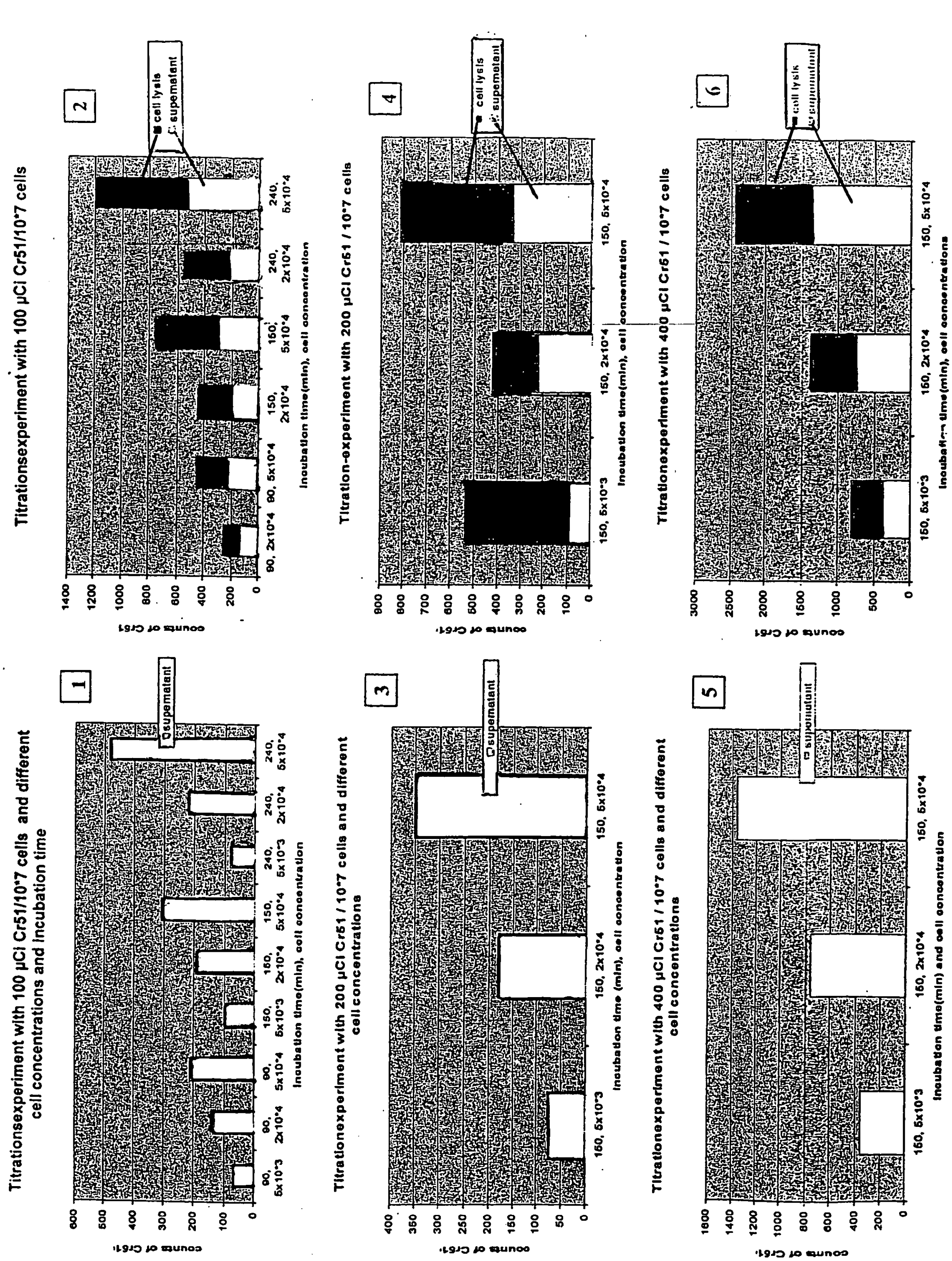


Fig

Newly filed

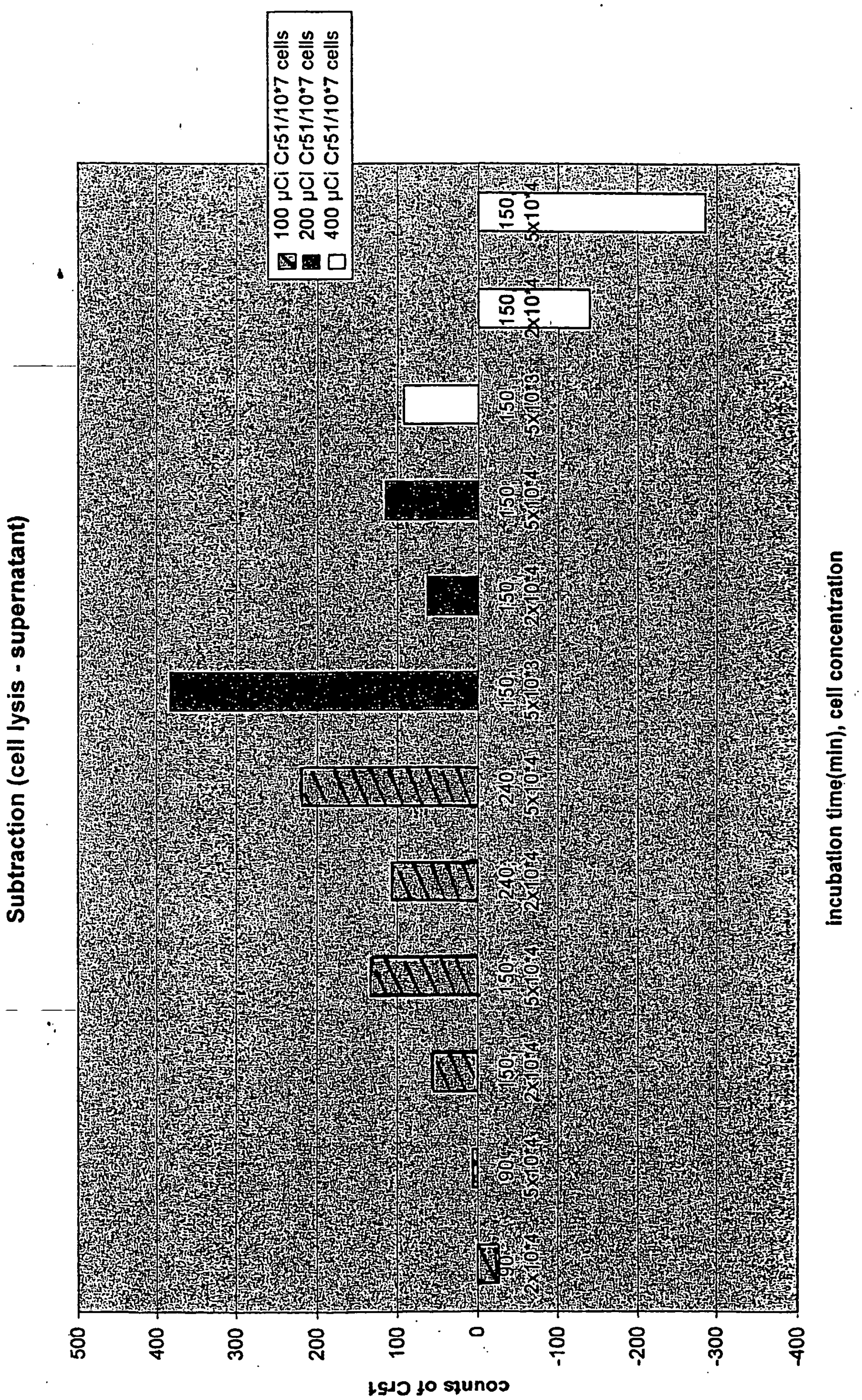

Subtraction (cell lysis - supernatant)
100 µCi Cr51/10*7 cells
200 µCi Cr51/10*7 cells
400 µCi Cr51/10*7 cells
counts of Cr51
500
400
300
200
100
0
-100
-200
-300
-400
90 2x10'4
90 5x10'4
150 2x10'4
150 5x10'4
240 2x10'4
240 5x10'4
150 5x10'3
150 2x10'4
150 5x10'4
150 5x10'3
150 2x10'4
150 5x10'4
incubation time(min), cell concentration


Fig. 14 b)

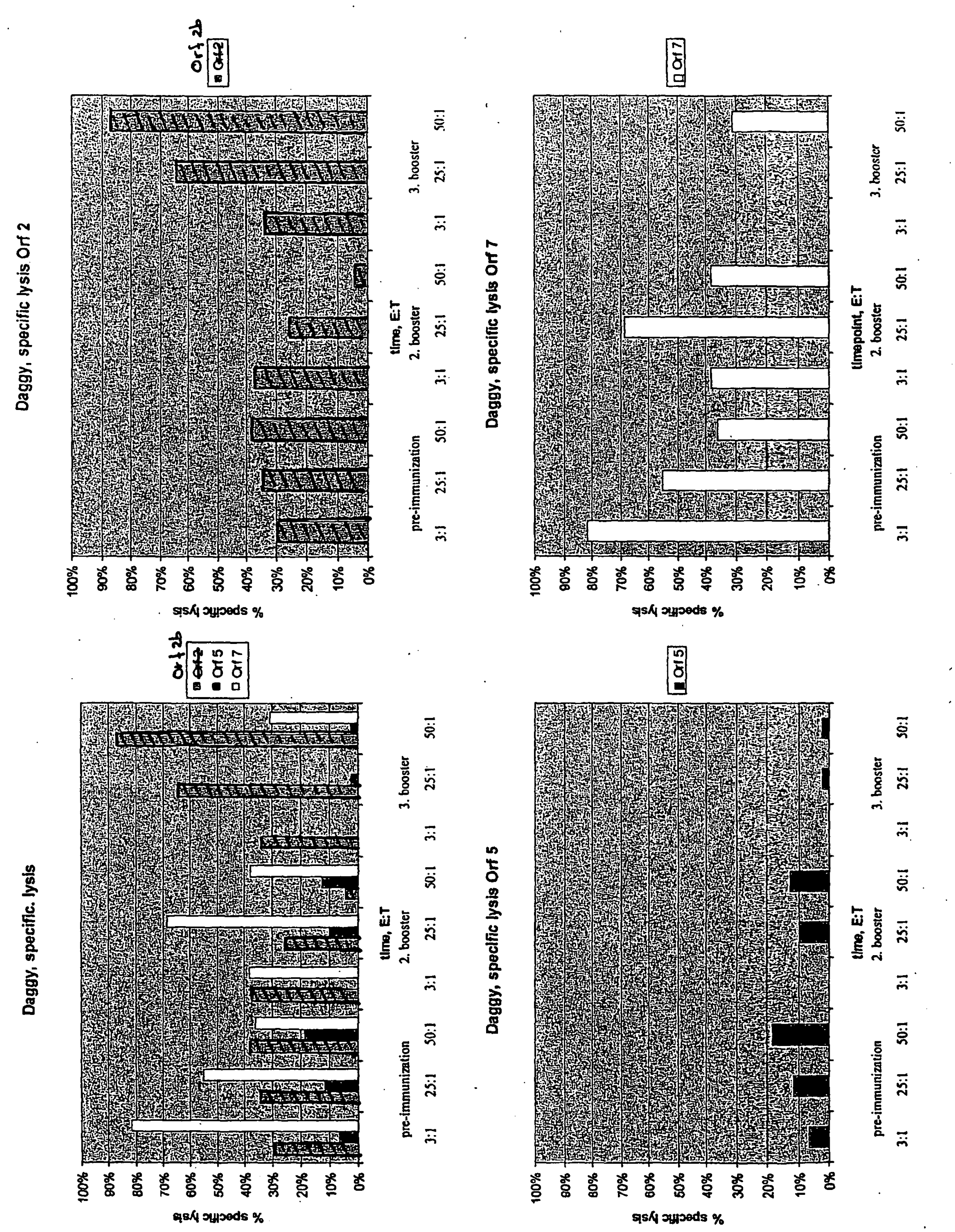
Daggy, specific lysis Orf 2
Daggy, specific. lysis
Daggy, specific lysis Orf 7
Daggy, specific lysis Orf 5
% specific lysis
Orf 5
Orf 7
time, E:T
timepoint, E:T
pre-immunization
2. booster
3. booster
3:1
25:1
50:1
100%
90%
80%
70%
60%
50%
40%
30%
20%
10%
0%

Fig. 15 a)

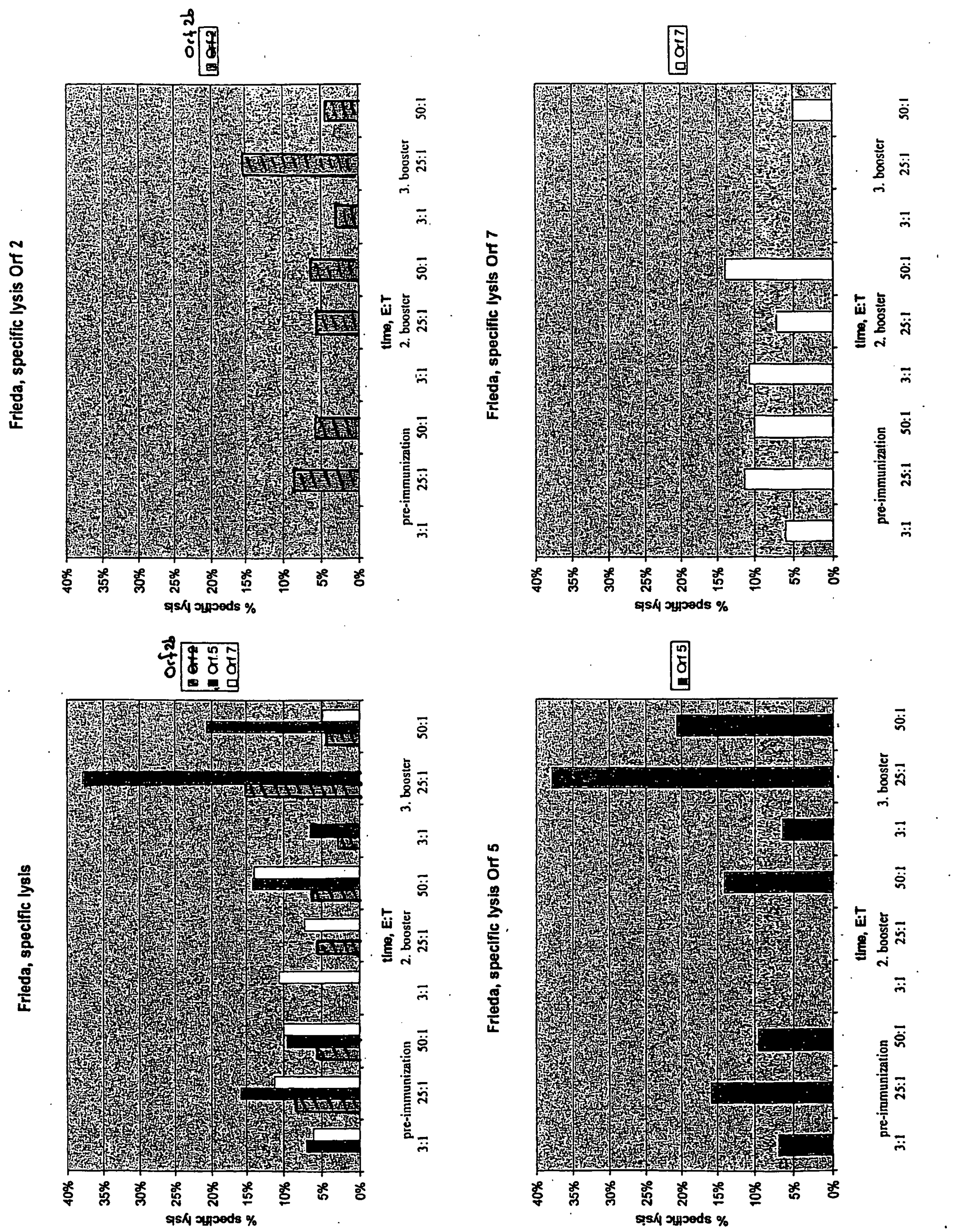
Frieda, specific lysis
Frieda, specific lysis Orf 2
Frieda, specific lysis Orf 5
Frieda, specific lysis Orf 7
% specific lysis
40%
35%
30%
25%
20%
15%
10%
5%
0%
Orf 2
Orf 2b
Orf 5
Orf 7
3:1
25:1
50:1
pre-immunization
2. booster
3. booster
time, E:T

Fig. 15 b)

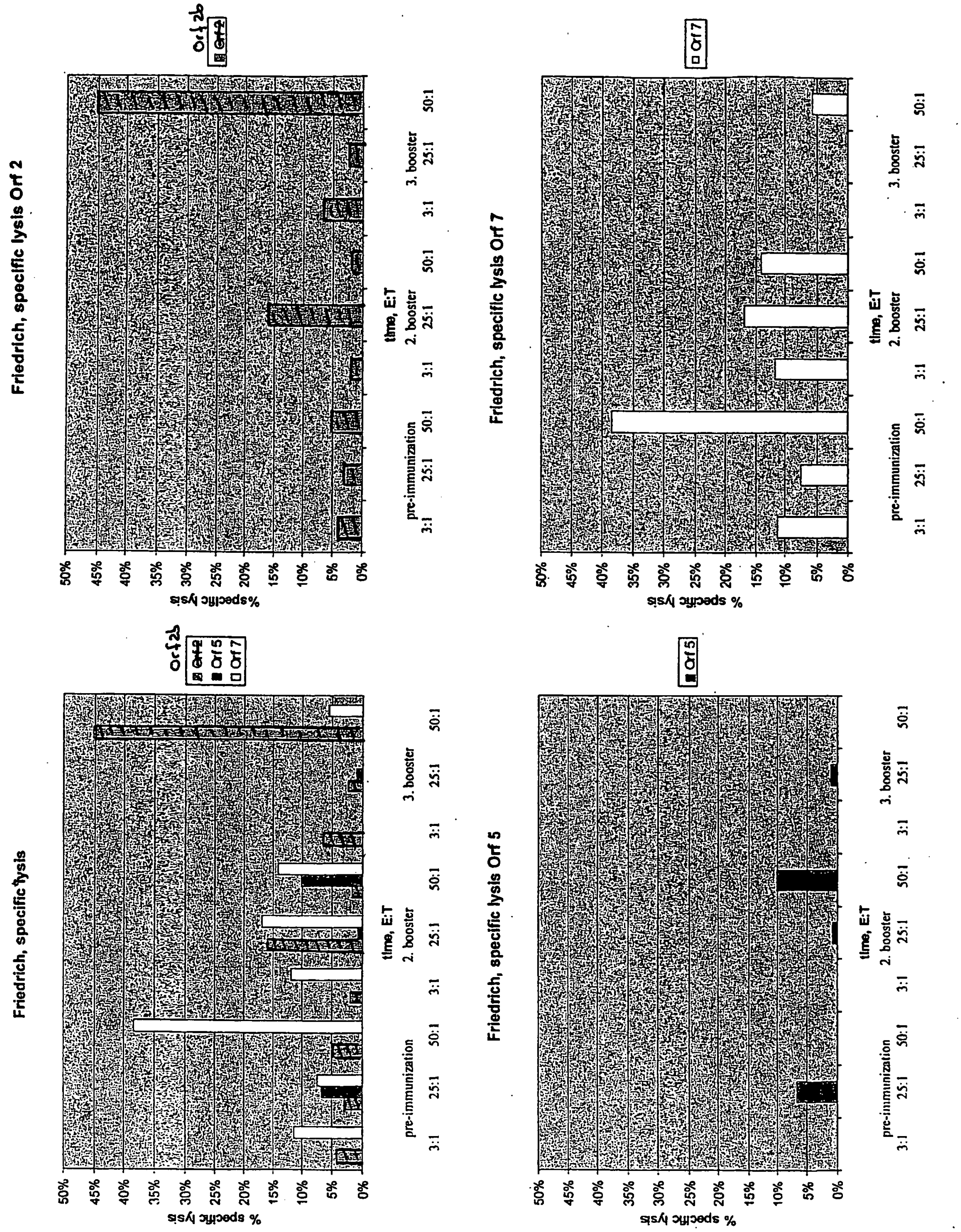
Friedrich, specific lysis
Friedrich, specific lysis Orf 2
Friedrich, specific lysis Orf 5
Friedrich, specific lysis Orf 7
% specific lysis
time, E:T
pre-immunization
2. booster
3. booster
3:1
25:1
50:1
Orf 2b
Orf 5
Orf 7
50%
45%
40%
35%
30%
25%
20%
15%
10%
5%
0%

Fig. 15 c)

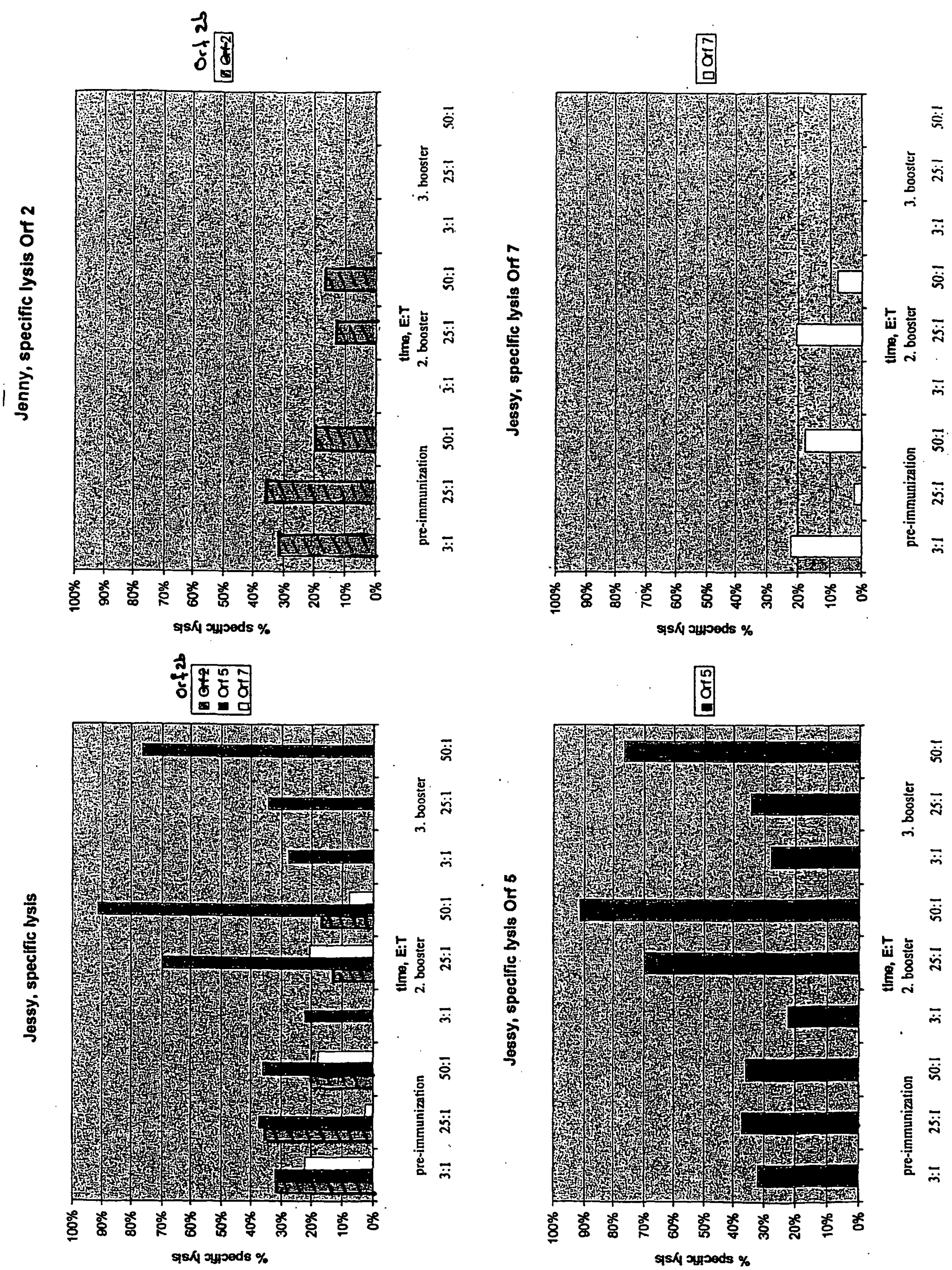
Jenny, specific lysis Orf 2
Orf 2b
% specific lysis
time, E:T
pre-immunization
2. booster
3. booster
3:1 25:1 50:1 3:1 25:1 50:1 3:1 25:1 50:1
Jessy, specific lysis
Orf 2b
Orf 5
Orf 7
Jessy, specific lysis Orf 7
Orf 7
Jessy, specific lysis Orf 5
Orf 5
100% 90% 80% 70% 60% 50% 40% 30% 20% 10% 0%

Fig. 15 d)

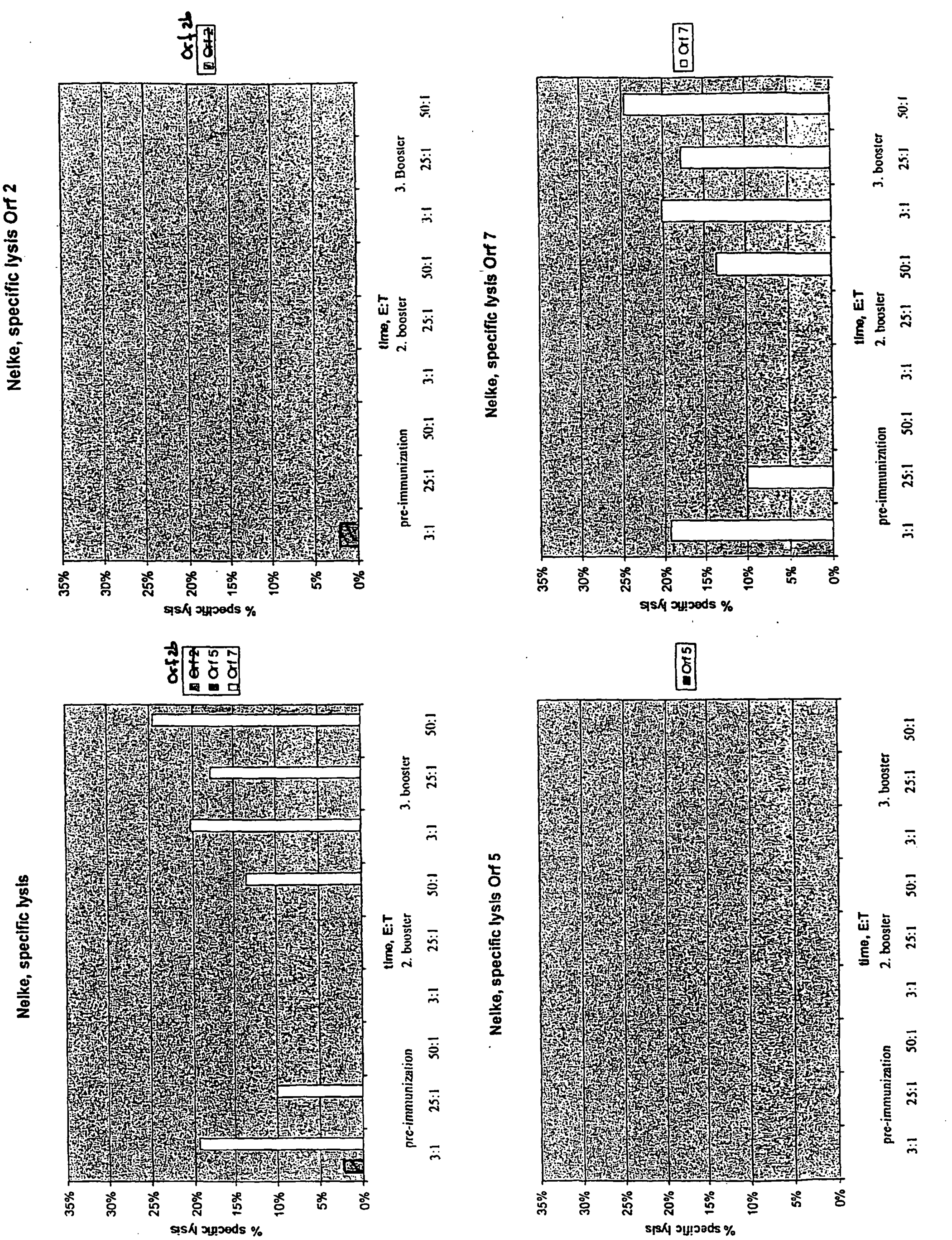
Nelke, specific lysis Orf 2
Orf 2b
Orf 2
Nelke, specific lysis
Orf 2b
Orf 5
Orf 7
Nelke, specific lysis Orf 7
Orf 7
Nelke, specific lysis Orf 5
Orf 5
35%
30%
25%
20%
15%
10%
5%
0%
% specific lysis
time, E:T
pre-immunization
2. booster
3. booster
3:1
25:1
50:1

Fig. 15 e)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Virus Genes,* March 2001, vol. 22 (2), 1787-199
- *Journal of Virology,* January 1997, vol. 71 (1), 169-178
- *Trends in Microbiology,* January 1998, vol. 6 (1), 23-27
- **SNIJDER E. et al.** *Journal of Virology,* August 1999, vol. 73 (8), 6335-6345
- **SAMBROOK et al.** Molecular Cloning. A Laboratory Manual. Cold Spring Harbor Laboratory Press, 1989
- **BERTRAM, S. ; GASSEN, H.G.** Gentechnische Methoden. G. Fischer Verlag, 1991
- Vaccine Design, The Subunit and Adjuvant Approach. Pharmaceutical Biotechnology. Plenum Press, vol. 6, 141-227
- **FRALEY R ; PAPAHADJOPOULOS D.** New generation liposomes - The engineering of an efficient vehicle for intracellular delivery of nucleic acids. *Trends Biochem Sci,* 1981, vol. 6, 77-80
- **MANNINO RJ ; GOULD-FOGERITE S.** Liposome mediated gene transfer. *BioTechniques,* 1988, vol. 6, 682-690
- **HAMMOND SA ; ISSEL CJ ; MONTELARO RC.** General method for the detection and in vitro expansion of equine cytolytic T lymphocytes. *J Immunol Method,* 1998, vol. 213, 73-85
- **HAMMOND SA ; ISSEL CJ ; MONTELARO RC.** *J. Immunol Methods,* 1998, vol. 213, 73-85
- **BALASURIYA, U. B. R. ; HEDGES, J. F. ; NADLER, S. A. ; MCCOLLUM, W.H. ; TIMONEY, P. J. ; MACLACHLAN, N. J.** Genetic stability of equine arteritis virus during horizontal and vertical transmission in an outbreak of equine viral arteritis. *J. Gen. Virol.,* 1999, vol. 80, 1949-1958
- **BALASURIYA, U. B. R. ; MACLACHLAN N. J. ; DEVRIES, A. A. F. ; ROSSITTO, P. V. ; ROTTIER, P. J. M.** Identification of a neutralization site in the major envelope glycoprotein (GL) of equine arteritis virus. *Virology,* 1995, vol. 207, 518-527
- **BALASURIYA, U.B. ; PATTON, J.F. ; ROSSITTO, P.V. ; TIMONEY, P.J. ; MCCOLLUM, W.H. ; MACLACHLAN, N.J.** Neutralization determinants of laboratory strains and field isolates of equine arteritis virus: identification of four neutralization sites in the amino-terminal ectodomain of the G(L) envelope glycoprotein. *Virology,* 1997, vol. 232, 114-128
- **BARRY M.A. ; JOHNSTON S.A.** Biological features of genetic immunization. *Vaccine,* 1997, vol. 15, 788-795
- **BRADFORD, M.** A rapid and sensitive method for the quantitation of microgram quantities of proteins utilizing the principle of protein dye binding. *Anal. Biochem.,* 1976, vol. 72, 248-254
- **DEN BOON, J.A. ; SNIJDER, E.J. ; CHIMSIDE, E.D. ; DE VRIES, A.A. ; HORZINEK, M.C. ; SPAAN, W.J.** Equine arteritis virus is not a togavirus but belongs to the coronaviruslike superfamily. *J Virol,* 1991, vol. 65, 2910-2920
- **CRKVENJAKOV, R. ; BYUS, C.** Ribonucleic acid isolated by cesium chloride centrifugation. *Biochemistry,* 1974, vol. 13, 2633-2637
- **HEDGES, J.F. ; BALASURIYA, U.B.R. ; TIMONEY, P.J. ; MCCOLLUM, W.H. ; MCLACHLAN, N.J.** Genetic divergence with emergence of novel phenotypic variants of equine arteritis virus during persistent infection of stallions. *J Virol,* 1999, vol. 73, 3672-3681
- **PASQUINI, S. ; XIANG, Z. ; WANG, Y. ; HE, Z. ; DENG, H. ; BLASZCZYK-THURIN, M. ; ERT1, H.C.** Cytokines and costimulatory molecules as genetic adjuvants. *Immunol. Cel.l Biol.,* 1997, vol. 75, 397-401
- **PATTON, J.F. ; BALASURIYA, U.B.R. ; HEDGES, J.F. ; SCHWEIDLER, T.M. ; HULLINGER, P.J. ; MACLACHLAN, N.J.** Phylogentic characterization of a highly attenuated strain of equine ateritis virus from the semen of a persistently infected standardbred stallion. *Arch Virol,* 1999, vol. 144, 817-827
- **PEDERSEN, K.W. ; VAN DER MEER ; Y. ROOS, N. ; SNIJDER, E.** Open reding frame 1a-encoded subunit of the arterivirus replicase induce endoplasmic reticulum-derived double-membrane vesicles which carry the viral replication complex. *J Virol,* 1999, vol. 73, 2016-2026
- **PIRZADEH, B. ; DEA, S.** Immune response in pigs vaccinated with plasmid DNA encoding ORF5 of porcine reproductive and respiratory syndrome virus. *J. Gen. Virol.,* 1998, vol. 79, 989-999
- **PYCOCK, J.F.** Equine viral arteritis risk from imported semen. *The Veterinary Record,* 1998, vol. 26, 699

- **ROSEN-WOLFF, A. ; BEN-HUR, T. ; BECKER, Y. ; DARAI, G.** Comparative analysis of the transcripts mapped in the BamHI DNA fragment B of avirulent HSV-1 HFEM, virulent HSV-1 F, and their intratypic recombinant viruses. *Virus Res.,* 1988, vol. 10, 315-324
- **ROSEN-WOLFF, A. ; SCHOLZ, J. ; DARAI, G.** Organotropism of latent herpes simplex virus type 1 is correlated to the presence of a 1.5 kb RNA transcript mapped within the BamHI DNA fragment B (0.738 to 0.809 map units). *Virus Res.,* 1989, vol. 12, 43-52
- **ROSEN-WOLFF, A. ; DARAI, G.** Identification and mapping of the UL56 gene transcript of herpes simplex virus type 1. *Virus Res.,* 1991, vol. 19, 115-126
- **STADEJEK, T. ; BJÖRKLUND, H. ; ROS BASCUNANA, C. ; CIABATTI, I.M. ; SCICLUNA, M.T. ; AMADDEO, D. ; MCCOLLUM, W.H. ; AUTORINO, G.L. ; TIMONEY, P.J. ; PATON, D. J.** Genetic diversity of equine ateritis virus. *J. Gen. Virol.,* 1999, vol. 80, 691-699
- **SNIJDER, E. ; VAN TOL, H. ; PEDERSEN, K.W. ; RAAMSMAN, M.J.B. ; DE VRIES, A.A.F.** Idenification of a novel structural protein of arteriviruses. *J Virol,* 1999, vol. 73, 6335-6345
- **THOMPSON, J. D. ; GIBSON, T. J. ; PLEWNIAK, F. ; JEANMOUGIN, F. ; HIGGINS, D. G.** The CLUSTAL_X windows interface: flexible strategies for multiple sequence alignment aided by quality analysis tools. *Nucleic Acids Res.,* 1997, vol. 25, 4876-4882
- **VAN DER MEER, Y. ; VAN TOL, H. LOCKER J.K. ; SNIJDER, E.** DORF1a-encoded replicase subunits are involved in the membrane association of the arterivirus replication complex. *Journal of Virology,* 1998, vol. 72, 6689-6698
- **VAN DINTEN, L.C. ; RENSEN, S. ; GORBALENYA, A. E. ; SNIJDER, E.J.** Proteolytic processing of the open reading frame 1b-encoded part of arterivirus replicase is mediated by nsp4 Serine protease and is essential for virus replication. *J Virol,* 1999, vol. 73, 2027-2037
- **WILL, H. ; CATTANEO, R. ; KOCH, H.G. ; DARAI, G. ; SCHALLER, H. ; SCHELLEKENS H. ; VAN EERD, P.M.C.A. ; DEINHARDT, F.** Cloned HBV DNA causes hepatitis in a chimpanzee. *Nature,* 1982, vol. 299, 740-742
- **WILL, H. ; CATTANEO, R. ; DARAI, G. ; DEINHARDT, F. ; SCHELLEKENS H. ; SCHALLER, H.** Infectious hepatitis B virus from cloned DNA of known nucleotide sequence. *Proc. Natn. Acad. Sci. U.S.A.,* 1985, vol. 82, 891-895